# EUROPEAN PATENT SPECIFICATION

(11) **EP 3 125 885 B1**
(45) Date of publication and mention of the grant of the patent: **30.06.2021**
(21) Application number: 15715805.6
(22) Date of filing: 02.04.2015
(51) Int. Cl.: A61K 31/4184, A61K 31/506, A61P 35/00, A61K 31/5377

(54) **COMBINATION OF EGFR INHIBITOR AND MEK INHIBITOR FOR USE IN THE TREATMENT OF NRAS MUTATED CANCER**
KOMBINATION VON EGFR-HEMMER UND MEK-HEMMER ZUR VERWENDUNG BEI DER BEHANDLUNG VON NRAS-MUTIERTEM KREBS
COMBINAISON D'UN INHIBITEUR DE EGFR ET UN INHIBITEUR DE MEK POUR L'UTILISATION DANS LE TRAITEMENT D'UN CANCER MUTÉ SUR NRAS

(30) Priority: 04.04.2014 US 201461975088 P; 18.06.2014 US 201462013573 P
(43) Date of publication of application: 08.02.2017
(73) Proprietor: Astrazeneca AB, 151 85 Södertälje (SE)
(72) Inventor: CROSS, Darren Anthony Edward, Cambridge Cambridgeshire CB4 0WG (GB); EBERLEIN, Catherine Anne, Macclesfield Cheshire SK10 4TG (GB)
(74) Representative: AstraZeneca
(86) International application number: PCT/GB2015/051042
(87) International publication number: WO 2015/150826

(56) References cited:
- US-A1- 2013 053 409
- MISALE SANDRA ET AL: "Blockade of EGFR and MEK intercepts heterogeneous mechanisms of acquired resistance to anti-EGFR therapies in colorectal cancer", SCIENCE TRANSLATIONAL MEDICINE 19 FEB 2014, vol. 6, no. 224, 19 February 2014 (2014-02-19), page 224ra26, XP002740843, ISSN: 1946-6242
- HUANG MING-HUNG ET AL: "MEK inhibitors reverse resistance in epidermal growth factor receptor mutation lung cancer cells with acquired resistance to gefitinib", MOLECULAR ONCOLOGY, ELSEVIER, AMSTERDAM, NL, vol. 7, no. 1, 1 February 2013 (2013-02-01), pages 112-120, XP008165510, ISSN: 1574-7891, DOI: 10.1016/J.MOLONC.2012.09.002 [retrieved on 2012-10-13]
- SONG JI-YOUNG ET AL: "Dual inhibition of MEK1/2 and EGFR synergistically induces caspase-3-dependent apoptosis in EGFR inhibitor-resistant lung cancer cells via BIM upregulation.", INVESTIGATIONAL NEW DRUGS DEC 2013, vol. 31, no. 6, December 2013 (2013-12), pages 1458-1465, XP002740844, ISSN: 1573-0646
- C. H. DIEP ET AL: "Synergistic Effect between Erlotinib and MEK Inhibitors in KRAS Wild-Type Human Pancreatic Cancer Cells", CLINICAL CANCER RESEARCH, vol. 17, no. 9, 8 March 2011 (2011-03-08), pages 2744-2756, XP055132319, ISSN: 1078-0432, DOI: 10.1158/1078-0432.CCR-10-2214
- FEI-YU NIU ET AL: "Novel agents and strategies for overcoming EGFR TKIs resistance", EXPERIMENTAL HEMATOLOGY & ONCOLOGY, BIOMED CENTRAL LTD, LONDON, UK, vol. 3, no. 1, 11 January 2014 (2014-01-11), pages 1-5, XP021175239, ISSN: 2162-3619, DOI: 10.1186/2162-3619-3-2
- SUN CHONG ET AL: "Intrinsic resistance to MEK inhibition in KRAS mutant lung and colon cancer through transcriptional induction of ERBB3", CELL REPORTS 10 APR 2014, vol. 7, no. 1, 27 March 2014 (2014-03-27), pages 86-93, XP002740911, ISSN: 2211-1247
- None

### SEQUENCE LISTING

<110> AstraZeneca AB and AstraZeneca UK Limited
<120> New Methods
<130> 200196-WO-PCT
<140> US 62/013573
   <141> 2014-06-18
<150> US 61/975088
   <151> 2014-04-04
<160> 17
<170> PatentIn version 3.5
<210> 1
   <211> 1210
   <212> PRT (1-protein) Homo sapiens
   <213> amino acid sequence: NP_005219.2
<400> 1
<210> 2
   <211> 5616
   <212> DNA Homo sapiens
   <213> cDNA sequence: NM_005228.3
<400> 2
   ccccggcgca gcgcggccgc agcagcctcc gccccccgca cggtgtgagc gcccgacgcg      60
   gccgaggcgg ccggagtccc gagctagccc cggcggccgc cgccgcccag accggacgac     120
   aggccacctc gtcggcgtcc gcccgagtcc ccgcctcgcc gccaacgcca caaccaccgc     180
   gcacggcccc ctgactccgt ccagtattga tcgggagagc cggagcgagc tcttcgggga     240
   gcagcgatgc gaccctccgg gacggccggg gcagcgctcc tggcgctgct ggctgcgctc     300
   tgcccggcga gtcgggctct ggaggaaaag aaagtttgcc aaggcacgag taacaagctc     360
   acgcagttgg gcacttttga agatcatttt ctcagcctcc agaggatgtt caataactgt     420
   gaggtggtcc ttgggaattt ggaaattacc tatgtgcaga ggaattatga tctttccttc     480
   ttaaagacca tccaggaggt ggctggttat gtcctcattg ccctcaacac agtggagcga     540
   attcctttgg aaaacctgca gatcatcaga ggaaatatgt actacgaaaa ttcctatgcc     600
   ttagcagtct tatctaacta tgatgcaaat aaaaccggac tgaaggagct gcccatgaga     660
   aatttacagg aaatcctgca tggcgccgtg cggttcagca acaaccctgc cctgtgcaac     720
   gtggagagca tccagtggcg ggacatagtc agcagtgact ttctcagcaa catgtcgatg     780
   gacttccaga accacctggg cagctgccaa aagtgtgatc caagctgtcc caatgggagc     840
   tgctggggtg caggagagga gaactgccag aaactgacca aaatcatctg tgcccagcag     900
   tgctccgggc gctgccgtgg caagtccccc agtgactgct gccacaacca gtgtgctgca     960
   ggctgcacag gcccccggga gagcgactgc ctggtctgcc gcaaattccg agacgaagcc    1020
   acgtgcaagg acacctgccc cccactcatg ctctacaacc ccaccacgta ccagatggat    1080
   gtgaaccccg agggcaaata cagctttggt gccacctgcg tgaagaagtg tccccgtaat    1140
   tatgtggtga cagatcacgg ctcgtgcgtc cgagcctgtg gggccgacag ctatgagatg 1200
   gaggaagacg gcgtccgcaa gtgtaagaag tgcgaagggc cttgccgcaa agtgtgtaac 1260
   ggaataggta ttggtgaatt taaagactca ctctccataa atgctacgaa tattaaacac 1320
   ttcaaaaact gcacctccat cagtggcgat ctccacatcc tgccggtggc atttaggggt 1380
   gactccttca cacatactcc tcctctggat ccacaggaac tggatattct gaaaaccgta 1440
   aaggaaatca cagggttttt gctgattcag gcttggcctg aaaacaggac ggacctccat 1500
   gcctttgaga acctagaaat catacgcggc aggaccaagc aacatggtca gttttctctt 1560
   gcagtcgtca gcctgaacat aacatccttg ggattacgct ccctcaagga gataagtgat 1620
   ggagatgtga taatttcagg aaacaaaaat ttgtgctatg caaatacaat aaactggaaa 1680
   aaactgtttg ggacctccgg tcagaaaacc aaaattataa gcaacagagg tgaaaacagc 1740
   tgcaaggcca caggccaggt ctgccatgcc ttgtgctccc ccgagggctg ctggggcccg 1800
   gagcccaggg actgcgtctc ttgccggaat gtcagccgag gcagggaatg cgtggacaag 1860
   tgcaaccttc tggagggtga gccaagggag tttgtggaga actctgagtg catacagtgc 1920
   cacccagagt gcctgcctca ggccatgaac atcacctgca caggacgggg accagacaac 1980
   tgtatccagt gtgcccacta cattgacggc ccccactgcg tcaagacctg cccggcagga 2040
   gtcatgggag aaaacaacac cctggtctgg aagtacgcag acgccggcca tgtgtgccac 2100
   ctgtgccatc caaactgcac ctacggatgc actgggccag gtcttgaagg ctgtccaacg 2160
   aatgggccta agatcccgtc catcgccact gggatggtgg gggccctcct cttgctgctg 2220
   gtggtggccc tggggatcgg cctcttcatg cgaaggcgcc acatcgttcg gaagcgcacg 2280
   ctgcggaggc tgctgcagga gagggagctt gtggagcctc ttacacccag tggagaagct 2340
   cccaaccaag ctctcttgag gatcttgaag gaaactgaat tcaaaaagat caaagtgctg 2400
   ggctccggtg cgttcggcac ggtgtataag ggactctgga tcccagaagg tgagaaagtt 2460
   aaaattcccg tcgctatcaa ggaattaaga gaagcaacat ctccgaaagc caacaaggaa 2520
   atcctcgatg aagcctacgt gatggccagc gtggacaacc cccacgtgtg ccgcctgctg 2580
   ggcatctgcc tcacctccac cgtgcagctc atcacgcagc tcatgccctt cggctgcctc 2640
   ctggactatg tccgggaaca caaagacaat attggctccc agtacctgct caactggtgt 2700
   gtgcagatcg caaagggcat gaactacttg gaggaccgtc gcttggtgca ccgcgacctg 2760
   gcagccagga acgtactggt gaaaacaccg cagcatgtca agatcacaga ttttgggctg 2820
   gccaaactgc tgggtgcgga agagaaagaa taccatgcag aaggaggcaa agtgcctatc 2880
   aagtggatgg cattggaatc aattttacac agaatctata cccaccagag tgatgtctgg 2940
   agctacgggg tgaccgtttg ggagttgatg acctttggat ccaagccata tgacggaatc 3000
   cctgccagcg agatctcctc catcctggag aaaggagaac gcctccctca gccacccata 3060
   tgtaccatcg atgtctacat gatcatggtc aagtgctgga tgatagacgc agatagtcgc 3120
   ccaaagttcc gtgagttgat catcgaattc tccaaaatgg cccgagaccc ccagcgctac 3180
   cttgtcattc agggggatga aagaatgcat ttgccaagtc ctacagactc caacttctac 3240
   cgtgccctga tggatgaaga agacatggac gacgtggtgg atgccgacga gtacctcatc 3300
   ccacagcagg gcttcttcag cagcccctcc acgtcacgga ctcccctcct gagctctctg 3360
   agtgcaacca gcaacaattc caccgtggct tgcattgata gaaatgggct gcaaagctgt 3420
   cccatcaagg aagacagctt cttgcagcga tacagctcag accccacagg cgccttgact 3480
   gaggacagca tagacgacac cttcctccca gtgcctgaat acataaacca gtccgttccc 3540
   aaaaggcccg ctggctctgt gcagaatcct gtctatcaca atcagcctct gaaccccgcg 3600
   cccagcagag acccacacta ccaggacccc cacagcactg cagtgggcaa ccccgagtat 3660
   ctcaacactg tccagcccac ctgtgtcaac agcacattcg acagccctgc ccactgggcc 3720
   cagaaaggca gccaccaaat tagcctggac aaccctgact accagcagga cttctttccc 3780
   aaggaagcca agccaaatgg catctttaag ggctccacag ctgaaaatgc agaataccta 3840
   agggtcgcgc cacaaagcag tgaatttatt ggagcatgac cacggaggat agtatgagcc 3900
   ctaaaaatcc agactctttc gatacccagg accaagccac agcaggtcct ccatcccaac 3960
   agccatgccc gcattagctc ttagacccac agactggttt tgcaacgttt acaccgacta 4020
   gccaggaagt acttccacct cgggcacatt ttgggaagtt gcattccttt gtcttcaaac 4080
   tgtgaagcat ttacagaaac gcatccagca agaatattgt ccctttgagc agaaatttat 4140
   ctttcaaaga ggtatatttg aaaaaaaaaa aaagtatatg tgaggatttt tattgattgg 4200
   ggatcttgga gtttttcatt gtcgctattg atttttactt caatgggctc ttccaacaag 4260
   gaagaagctt gctggtagca cttgctaccc tgagttcatc caggcccaac tgtgagcaag 4320
   gagcacaagc cacaagtctt ccagaggatg cttgattcca gtggttctgc ttcaaggctt 4380
   ccactgcaaa acactaaaga tccaagaagg ccttcatggc cccagcaggc cggatcggta 4440
   ctgtatcaag tcatggcagg tacagtagga taagccactc tgtcccttcc tgggcaaaga 4500
   agaaacggag gggatggaat tcttccttag acttactttt gtaaaaatgt ccccacggta 4560
   cttactcccc actgatggac cagtggtttc cagtcatgag cgttagactg acttgtttgt 4620
   cttccattcc attgttttga aactcagtat gctgcccctg tcttgctgtc atgaaatcag 4680
   caagagagga tgacacatca aataataact cggattccag cccacattgg attcatcagc 4740
   atttggacca atagcccaca gctgagaatg tggaatacct aaggatagca ccgcttttgt 4800
   tctcgcaaaa acgtatctcc taatttgagg ctcagatgaa atgcatcagg tcctttgggg 4860
   catagatcag aagactacaa aaatgaagct gctctgaaat ctcctttagc catcacccca 4920
   accccccaaa attagtttgt gttacttatg gaagatagtt ttctcctttt acttcacttc 4980
   aaaagctttt tactcaaaga gtatatgttc cctccaggtc agctgccccc aaaccccctc 5040
   cttacgcttt gtcacacaaa aagtgtctct gccttgagtc atctattcaa gcacttacag 5100
   ctctggccac aacagggcat tttacaggtg cgaatgacag tagcattatg agtagtgtgg 5160
   aattcaggta gtaaatatga aactagggtt tgaaattgat aatgctttca caacatttgc 5220
   agatgtttta gaaggaaaaa agttccttcc taaaataatt tctctacaat tggaagattg 5280
   gaagattcag ctagttagga gcccaccttt tttcctaatc tgtgtgtgcc ctgtaacctg 5340
   actggttaac agcagtcctt tgtaaacagt gttttaaact ctcctagtca atatccaccc 5400
   catccaattt atcaaggaag aaatggttca gaaaatattt tcagcctaca gttatgttca 5460
   gtcacacaca catacaaaat gttccttttg cttttaaagt aatttttgac tcccagatca 5520
   gtcagagccc ctacagcatt gttaagaaag tatttgattt ttgtctcaat gaaaataaaa 5580
   ctatattcat ttccactcta aaaaaaaaaa aaaaaa 5616
<210> 3
   <211> 189
   <212> PRT (1-protein)
   <213> Homo sapiens amino-acid sequence: NP_002515.1
<400> 3
   Met Thr Glu Tyr Lys Leu Val Val Val Gly Ala Gly Gly Val Gly Lys
   1               5                   10                  15
   Ser Ala Leu Thr Ile Gln Leu Ile Gln Asn His Phe Val Asp Glu Tyr
               20                  25                  30
   Asp Pro Thr Ile Glu Asp Ser Tyr Arg Lys Gln Val Val Ile Asp Gly
           35                  40                  45
   Glu Thr Cys Leu Leu Asp Ile Leu Asp Thr Ala Gly Gln Glu Glu Tyr
       50                  55                  60
   Ser Ala Met Arg Asp Gln Tyr Met Arg Thr Gly Glu Gly Phe Leu Cys
   65                  70                  75                  80
   Val Phe Ala Ile Asn Asn Ser Lys Ser Phe Ala Asp Ile Asn Leu Tyr
                   85                  90                  95
   Arg Glu Gln Ile Lys Arg Val Lys Asp Ser Asp Asp Val Pro Met Val
               100                 105                 110
   Leu Val Gly Asn Lys Cys Asp Leu Pro Thr Arg Thr Val Asp Thr Lys
               115                 120                 125
   Gln Ala His Glu Leu Ala Lys Ser Tyr Gly Ile Pro Phe Ile Glu Thr
       130                 135                 140
   Ser Ala Lys Thr Arg Gln Gly Val Glu Asp Ala Phe Tyr Thr Leu Val
   145                 150                 155                 160
   Arg Glu Ile Arg Gln Tyr Arg Met Lys Lys Leu Asn Ser Ser Asp Asp
                   165                 170                 175
   Gly Thr Gln Gly Cys Met Gly Leu Pro Cys Val Val Met
               180                 185
<210> 4
   <211> 4454
   <212> DNA
   <213> Homo sapiens cDNA sequence NM_002524.4
<400> 4
   gaaacgtccc gtgtgggagg ggcgggtctg ggtgcggcct gccgcatgac tcgtggttcg      60
   gaggcccacg tggccggggc ggggactcag gcgcctgggg cgccgactga ttacgtagcg     120
   ggcggggccg gaagtgccgc tccttggtgg gggctgttca tggcggttcc ggggtctcca     180
   acatttttcc cggctgtggt cctaaatctg tccaaagcag aggcagtgga gcttgaggtt     240
   cttgctggtg tgaaatgact gagtacaaac tggtggtggt tggagcaggt ggtgttggga     300
   aaagcgcact gacaatccag ctaatccaga accactttgt agatgaatat gatcccacca     360
   tagaggattc ttacagaaaa caagtggtta tagatggtga aacctgtttg ttggacatac     420
   tggatacagc tggacaagaa gagtacagtg ccatgagaga ccaatacatg aggacaggcg     480
   aaggcttcct ctgtgtattt gccatcaata atagcaagtc atttgcggat attaacctct     540
   acagggagca gattaagcga gtaaaagact cggatgatgt acctatggtg ctagtgggaa     600
   acaagtgtga tttgccaaca aggacagttg atacaaaaca agcccacgaa ctggccaaga     660
   gttacgggat tccattcatt gaaacctcag ccaagaccag acagggtgtt gaagatgctt     720
   tttacacact ggtaagagaa atacgccagt accgaatgaa aaaactcaac agcagtgatg     780
   atgggactca gggttgtatg ggattgccat gtgtggtgat gtaacaagat acttttaaag     840
   ttttgtcaga aaagagccac tttcaagctg cactgacacc ctggtcctga cttccctgga     900
   ggagaagtat tcctgttgct gtcttcagtc tcacagagaa gctcctgcta cttccccagc     960
   tctcagtagt ttagtacaat aatctctatt tgagaagttc tcagaataac tacctcctca    1020
   cttggctgtc tgaccagaga atgcacctct tgttactccc tgttattttt ctgccctggg    1080
   ttcttccaca gcacaaacac acctctgcca ccccaggttt ttcatctgaa aagcagttca    1140
   tgtctgaaac agagaaccaa accgcaaacg tgaaattcta ttgaaaacag tgtcttgagc 1200
   tctaaagtag caactgctgg tgattttttt tttcttttta ctgttgaact tagaactatg 1260
   ctaatttttg gagaaatgtc ataaattact gttttgccaa gaatatagtt attattgctg 1320
   tttggtttgt ttataatgtt atcggctcta ttctctaaac tggcatctgc tctagattca 1380
   taaatacaaa aatgaatact gaattttgag tctatcctag tcttcacaac tttgacgtaa 1440
   ttaaatccaa ctttcacagt gaagtgcctt tttcctagaa gtggtttgta gacttccttt 1500
   ataatatttc agtggaatag atgtctcaaa aatccttatg catgaaatga atgtctgaga 1560
   tacgtctgtg acttatctac cattgaagga aagctatatc tatttgagag cagatgccat 1620
   tttgtacatg tatgaaattg gttttccaga ggcctgtttt ggggctttcc caggagaaag 1680
   atgaaactga aagcacatga ataatttcac ttaataattt ttacctaatc tccacttttt 1740
   tcataggtta ctacctatac aatgtatgta atttgtttcc cctagcttac tgataaacct 1800
   aatattcaat gaacttccat ttgtattcaa atttgtgtca taccagaaag ctctacattt 1860
   gcagatgttc aaatattgta aaactttggt gcattgttat ttaatagctg tgatcagtga 1920
   ttttcaaacc tcaaatatag tatattaaca aattacattt tcactgtata tcatggtatc 1980
   ttaatgatgt atataattgc cttcaatccc cttctcaccc caccctctac agcttccccc 2040
   acagcaatag gggcttgatt atttcagttg agtaaagcat ggtgctaatg gaccagggtc 2100
   acagtttcaa aacttgaaca atccagttag catcacagag aaagaaattc ttctgcattt 2160
   gctcattgca ccagtaactc cagctagtaa ttttgctagg tagctgcagt tagccctgca 2220
   aggaaagaag aggtcagtta gcacaaaccc tttaccatga ctggaaaact cagtatcacg 2280
   tatttaaaca tttttttttc ttttagccat gtagaaactc taaattaagc caatattctc 2340
   atttgagaat gaggatgtct cagctgagaa acgttttaaa ttctctttat tcataatgtt 2400
   ctttgaaggg tttaaaacaa gatgttgata aatctaagct gatgagtttg ctcaaaacag 2460
   gaagttgaaa ttgttgagac aggaatggaa aatataatta attgatacct atgaggattt 2520
   ggaggcttgg cattttaatt tgcagataat accctggtaa ttctcatgaa aaatagactt 2580
   ggataacttt tgataaaaga ctaattccaa aatggccact ttgttcctgt ctttaatatc 2640
   taaatactta ctgaggtcct ccatcttcta tattatgaat tttcatttat taagcaaatg 2700
   tcatattacc ttgaaattca gaagagaaga aacatatact gtgtccagag tataatgaac 2760
   ctgcagagtt gtgcttctta ctgctaattc tgggagcttt cacagtactg tcatcatttg 2820
   taaatggaaa ttctgctttt ctgtttctgc tccttctgga gcagtgctac tctgtaattt 2880
   tcctgaggct tatcacctca gtcatttctt ttttaaatgt ctgtgactgg cagtgattct 2940
   ttttcttaaa aatctattaa atttgatgtc aaattaggga gaaagatagt tactcatctt 3000
   gggctcttgt gccaatagcc cttgtatgta tgtacttaga gttttccaag tatgttctaa 3060
   gcacagaagt ttctaaatgg ggccaaaatt cagacttgag tatgttcttt gaatacctta 3120
   agaagttaca attagccggg catggtggcc cgtgcctgta gtcccagcta cttgagaggc 3180
   tgaggcagga gaatcacttc aacccaggag gtggaggtta cagtgagcag agatcgtgcc 3240
   actgcactcc agcctgggtg acaagagaga cttgtctcca aaaaaaaagt tacacctagg 3300
   tgtgaatttt ggcacaaagg agtgacaaac ttatagttaa aagctgaata acttcagtgt 3360
   ggtataaaac gtggttttta ggctatgttt gtgattgctg aaaagaattc tagtttacct 3420
   caaaatcctt ctctttcccc aaattaagtg cctggccagc tgtcataaat tacatattcc 3480
   ttttggtttt tttaaaggtt acatgttcaa gagtgaaaat aagatgttct gtctgaaggc 3540
   taccatgccg gatctgtaaa tgaacctgtt aaatgctgta tttgctccaa cggcttacta 3600
   tagaatgtta cttaatacaa tatcatactt attacaattt ttactatagg agtgtaatag 3660
   gtaaaattaa tctctatttt agtgggccca tgtttagtct ttcaccatcc tttaaactgc 3720
   tgtgaatttt tttgtcatga cttgaaagca aggatagaga aacactttag agatatgtgg 3780
   ggttttttta ccattccaga gcttgtgagc ataatcatat ttgctttata tttatagtca 3840
   tgaactccta agttggcagc tacaaccaag aaccaaaaaa tggtgcgttc tgcttcttgt 3900
   aattcatctc tgctaataaa ttataagaag caaggaaaat tagggaaaat attttatttg 3960
   gatggtttct ataaacaagg gactataatt cttgtacatt atttttcatc tttgctgttt 4020
   ctttgagcag tctaatgtgc cacacaatta tctaaggtat ttgttttcta taagaattgt 4080
   tttaaaagta ttcttgttac cagagtagtt gtattatatt tcaaaacgta agatgatttt 4140
   taaaagcctg agtactgacc taagatggaa ttgtatgaac tctgctctgg agggagggga 4200
   ggatgtccgt ggaagttgta agacttttat ttttttgtgc catcaaatat aggtaaaaat 4260
   aattgtgcaa ttctgctgtt taaacaggaa ctattggcct ccttggccct aaatggaagg 4320
   gccgatattt taagttgatt attttattgt aaattaatcc aacctagttc tttttaattt 4380
   ggttgaatgt tttttcttgt taaatgatgt ttaaaaaata aaaactggaa gttcttggct 4440
   tagtcataat tctt 4454
<210> 5
   <211> 393
   <212> PRT(1-protein)
   <213> Homo sapiens amino acid sequence NP-002746.1
<400> 5
<210> 6
   <211> 2603
   <212> DNA
   <213> Homo sapiens cDNA sequence: NM_002755.3
<400> 6
   aggcgaggct tccccttccc cgcccctccc ccggcctcca gtccctccca gggccgcttc 60
   gcagagcggc taggagcacg gcggcggcgg cactttcccc ggcaggagct ggagctgggc 120
   tctggtgcgc gcgcggctgt gccgcccgag ccggagggac tggttggttg agagagagag 180
   aggaagggaa tcccgggctg ccgaaccgca cgttcagccc gctccgctcc tgcagggcag 240
   cctttcggct ctctgcgcgc gaagccgagt cccgggcggg tggggcgggg gtccactgag 300
   accgctaccg gcccctcggc gctgacggga ccgcgcgggg cgcacccgct gaaggcagcc 360
   ccggggcccg cggcccggac ttggtcctgc gcagcgggcg cggggcagcg cagcgggagg 420
   aagcgagagg tgctgccctc cccccggagt tggaagcgcg ttacccgggt ccaaaatgcc 480
   caagaagaag ccgacgccca tccagctgaa cccggccccc gacggctctg cagttaacgg 540
   gaccagctct gcggagacca acttggaggc cttgcagaag aagctggagg agctagagct 600
   tgatgagcag cagcgaaagc gccttgaggc ctttcttacc cagaagcaga aggtgggaga 660
   actgaaggat gacgactttg agaagatcag tgagctgggg gctggcaatg gcggtgtggt 720
   gttcaaggtc tcccacaagc cttctggcct ggtcatggcc agaaagctaa ttcatctgga 780
   gatcaaaccc gcaatccgga accagatcat aagggagctg caggttctgc atgagtgcaa 840
   ctctccgtac atcgtgggct tctatggtgc gttctacagc gatggcgaga tcagtatctg 900
   catggagcac atggatggag gttctctgga tcaagtcctg aagaaagctg gaagaattcc 960
   tgaacaaatt ttaggaaaag ttagcattgc tgtaataaaa ggcctgacat atctgaggga 1020
   gaagcacaag atcatgcaca gagatgtcaa gccctccaac atcctagtca actcccgtgg 1080
   ggagatcaag ctctgtgact ttggggtcag cgggcagctc atcgactcca tggccaactc 1140
   cttcgtgggc acaaggtcct acatgtcgcc agaaagactc caggggactc attactctgt 1200
   gcagtcagac atctggagca tgggactgtc tctggtagag atggcggttg ggaggtatcc 1260
   catccctcct ccagatgcca aggagctgga gctgatgttt gggtgccagg tggaaggaga 1320
   tgcggctgag accccaccca ggccaaggac ccccgggagg ccccttagct catacggaat 1380
   ggacagccga cctcccatgg caatttttga gttgttggat tacatagtca acgagcctcc 1440
   tccaaaactg cccagtggag tgttcagtct ggaatttcaa gattttgtga ataaatgctt 1500
   aataaaaaac cccgcagaga gagcagattt gaagcaactc atggttcatg cttttatcaa 1560
   gagatctgat gctgaggaag tggattttgc aggttggctc tgctccacca tcggccttaa 1620
   ccagcccagc acaccaaccc atgctgctgg cgtctaagtg tttgggaagc aacaaagagc 1680
   gagtcccctg cccggtggtt tgccatgtcg cttttgggcc tccttcccat gcctgtctct 1740
   gttcagatgt gcatttcacc tgtgacaaag gatgaagaac acagcatgtg ccaagattct 1800
   actcttgtca tttttaatat tactgtcttt attcttatta ctattattgt tcccctaagt 1860
   ggattggctt tgtgcttggg gctatttgtg tgtatgctga tgatcaaaac ctgtgccagg 1920
   ctgaattaca gtgaaatttt ggtgaatgtg ggtagtcatt cttacaattg cactgctgtt 1980
   cctgctccat gactggctgt ctgcctgtat tttcgggatt ctttgacatt tggtggtact 2040
   ttattcttgc tgggcatact ttctctctag gagggagcct tgtgagatcc ttcacaggca 2100
   gtgcatgtga agcatgcttt gctgctatga aaatgagcat cagagagtgt acatcatgtt 2160
   attttattat tattatttgc ttttcatgta gaactcagca gttgacatcc aaatctagcc 2220
   agagcccttc actgccatga tagctggggc ttcaccagtc tgtctactgt ggtgatctgt 2280
   agacttctgg ttgtatttct atatttattt tcagtatact gtgtgggata cttagtggta 2340
   tgtctcttta agttttgatt aatgtttctt aaatggaatt attttgaatg tcacaaattg 2400
   atcaagatat taaaatgtcg gatttatctt tccccatatc caagtaccaa tgctgttgta 2460
   aacaacgtgt atagtgccta aaattgtatg aaaatccttt taaccatttt aacctagatg 2520
   tttaacaaat ctaatctctt attctaataa atatactatg aaataaaaaa aaaaggatga 2580
   aagctaaaaa aaaaaaaaaa aaa 2603
<210> 7
   <211> 400
   <212> PRT (1-protein)
   <213> Homo sapiens amino acid sequence: NP_109587.1
<400> 7
<210> 8
   <211> 1759
   <212> DNA
   <213> Homo sapiens cDNA sequence: NM_030662.3
<400> 8
   cccctgcctc tcggactcgg gctgcggcgt cagccttctt cgggcctcgg cagcggtagc 60
   ggctcgctcg cctcagcccc agcgcccctc ggctaccctc ggcccaggcc cgcagcgccg 120
   cccgccctcg gccgccccga cgccggcctg ggccgcggcc gcagccccgg gctcgcgtag 180
   gcgccgaccg ctcccggccc gccccctatg ggccccggct agaggcgccg ccgccgccgg 240
   cccgcggagc cccgatgctg gcccggagga agccggtgct gccggcgctc accatcaacc      300
   ctaccatcgc cgagggccca tcccctacca gcgagggcgc ctccgaggca aacctggtgg      360
   acctgcagaa gaagctggag gagctggaac ttgacgagca gcagaagaag cggctggaag      420
   cctttctcac ccagaaagcc aaggtcggcg aactcaaaga cgatgacttc gaaaggatct      480
   cagagctggg cgcgggcaac ggcggggtgg tcaccaaagt ccagcacaga ccctcgggcc      540
   tcatcatggc caggaagctg atccaccttg agatcaagcc ggccatccgg aaccagatca      600
   tccgcgagct gcaggtcctg cacgaatgca actcgccgta catcgtgggc ttctacgggg      660
   ccttctacag tgacggggag atcagcattt gcatggaaca catggacggc ggctccctgg      720
   accaggtgct gaaagaggcc aagaggattc ccgaggagat cctggggaaa gtcagcatcg      780
   cggttctccg gggcttggcg tacctccgag agaagcacca gatcatgcac cgagatgtga      840
   agccctccaa catcctcgtg aactctagag gggagatcaa gctgtgtgac ttcggggtga      900
   gcggccagct catcgactcc atggccaact ccttcgtggg cacgcgctcc tacatggctc      960
   cggagcggtt gcagggcaca cattactcgg tgcagtcgga catctggagc atgggcctgt     1020
   ccctggtgga gctggccgtc ggaaggtacc ccatcccccc gcccgacgcc aaagagctgg     1080
   aggccatctt tggccggccc gtggtcgacg gggaagaagg agagcctcac agcatctcgc     1140
   ctcggccgag gccccccggg cgccccgtca gcggtcacgg gatggatagc cggcctgcca     1200
   tggccatctt tgaactcctg gactatattg tgaacgagcc acctcctaag ctgcccaacg     1260
   gtgtgttcac ccccgacttc caggagtttg tcaataaatg cctcatcaag aacccagcgg     1320
   agcgggcgga cctgaagatg ctcacaaacc acaccttcat caagcggtcc gaggtggaag     1380
   aagtggattt tgccggctgg ttgtgtaaaa ccctgcggct gaaccagccc ggcacaccca     1440
   cgcgcaccgc cgtgtgacag tggccgggct ccctgcgtcc cgctggtgac ctgcccaccg     1500
   tccctgtcca tgccccgccc ttccagctga ggacaggctg gcgcctccac ccaccctcct     1560
   gcctcacccc tgcggagagc accgtggcgg ggcgacagcg catgcaggaa cgggggtctc     1620
   ctctcctgcc cgtcctggcc ggggtgcctc tggggacggg cgacgctgct gtgtgtggtc     1680
   tcagaggctc tgcttcctta ggttacaaaa caaaacaggg agagaaaaag caaaaaaaaa     1740
   aaaaaaaaaa aaaaaaaaa                                                  1759
<210> 9
   <211> 19
   <212> RNA
   <213> Synthetic Nontargeting control (NTC)
<400> 9
   ugguuuacau gucgacuaa 19
<210> 10
   <211> 19
   <212> RNA
   <213> Synthetic Nontargeting control (NTC)
<400> 10
   ugguuuacau guuguguga 19
<210> 11
   <211> 19
   <212> RNA
   <213> Synthetic Nontargeting control (NTC)
<400> 11
   ugguuuacau guuuucuga 19
<210> 12
   <211> 19
   <212> RNA
   <213> Synthetic Nontargeting control (NTC)
<400> 12
   ugguuuacau guuuuccua 19
<210> 13
   <211> 19
   <212> RNA
   <213> Homo sapiens NRAS_1
<400> 13
   gagcagauua agcgaguaa 19
<210> 14
   <211> 19
   <212> RNA
   <213> Homo sapiens NRAS_2
<400> 14
   gaaauacgcc aguaccgaa 19
<210> 15
   <211> 19
   <212> RNA
   <213> Homo sapiens NRAS_3
<400> 15
   guggugaugu aacaagaua 19
<210> 16
   <211> 19
   <212> RNA
   <213> Homo sapiens KRAS_1
<400> 16
   gaaguuaugg aauuccuuu 19
<210> 17
   <211> 19
   <212> RNA
   <213> Homo sapiens KRAS_2
<400> 17
   ggagggcuuu cuuugugua 19

## Description

### Field of the invention

The invention relates to methods for predicting development of resistance to EGFR-inhibitor-based cancer therapy. The invention further relates to methods for selecting suitable cancer treatment regimens for patients and to methods for treating certain drug-resistant cancers, as well as products for use in such methods. In particular, the invention relates to methods and products for predicting development of resistance to EGFR-inhibitor mediated cancer therapy, and to methods and products for treating such drug-resistant cancer using a combination of an EGFR inhibitor and a MEK inhibitor.

### Background of the invention

The epidermal growth factor receptor (EGFR) has been identified as a target for treatment of a number of cancers, in particular, solid tumours, as it is involved in regulating cellular functions important in the proliferation and survival of cancer cells. Increased expression of EGFR has been observed in bladder, breast, glioblastoma, head and neck, lung and stomach cancer. Development of cancer may for example be associated with an activating mutation in EGFR, and high expression of EGFR is often related to poor prognosis. Activating cancerous mutations in EGFR are often somatic gain-of-function mutations in exons that encode the tyrosine kinase domain of the receptor. Examples of such mutations, identified in lung adenocarcinomas in non-small cell lung cancer (NSCLC) patients, include multi-nucleotide in-frame deletions in exon 19 (involving elimination of four amino acids, Leu-Arg-Glu-Ala), and a single nucleotide substitution at nucleotide 2573 (T→G) in exon 21, resulting in substitution of arginine for leucine at position 858 (L858R). These mutations have been found to increase sensitivity to EGFR tyrosine kinase inhibitors (TKIs). Consequently, first line therapies targeting EGFR are often based on inhibition of the tyrosine kinase activity of the mutant receptor.

Established first line therapies for patients with an activating mutation in EGFR include use of EGFR TKIs such as gefitinib (Iressa™), erlotinib (Tarceva™) and afatinib (Gilotrif™). However, despite initial responses to these EGFR TKIs, a significant proportion of patients ultimately show disease progression due to acquired resistance, which has been shown in many cases to be associated with an additional mutation in EGFR, known as T790M.

Acquired resistance has led to the development of further TKIs, such as AZD9291, CO-1686 and WZ4002, which inhibit EGFR receptors that possess the activating mutations in the tyrosine kinase domain, such as exon 19del and L858R mutations, as well as the T790M mutation in pre-clinical models. However, it is a concern that tumours may ultimately develop resistances to these drugs also, limiting their longer-term effectiveness in patients. Ji-Young Song et al. discloses that the dual inhibition of MEK1/2 and EGFR synergistically induces apoptosis in EGFR resistant lung cancer cells, but does not disclose the specific NRAS mutations.

In view of this, there is a need to determine the mechanisms underlying acquired resistance to EGFR inhibitors in cancer therapy, and try to provide new treatments which can overcome these further resistance mechanisms.

### Summary of the invention

This invention is limited to the subject-matter defined in the claims; the following description is subject to this limitation. Based on laboratory experiments with populations of cancer cells, the present inventors have found that resistance to EGFR-inhibitor mediated cancer therapy may be associated in some patients with genetic anomalies in the Neuroblastoma RAS viral oncogene homolog gene (*NRAS*) that encodes the Neuroblastoma RAS viral oncogene homolog (NRAS) protein, manifested by certain mutations resulting in codon substitution in the encoded protein or via a gain of copy number of the *NRAS* gene. According to one aspect of the present invention, resistance to EGFR-inhibitor mediated cancer therapy may be associated with an E63K NRAS mutation and/or a G12V NRAS mutation.

The NRAS protein activates the Ras, Raf, MAP protein kinase/extracellular signal-regulated kinase kinase (MEK), extracellular signal-regulated kinase (ERK) pathway (Ras/Raf/MEK/ERK pathway) in cells. This pathway is downstream of the EGFR receptor, and plays a central role in the regulation of a variety of cellular functions dependent upon cellular context, including cellular proliferation, differentiation, survival, immortalization, invasion and angiogenesis (reviewed in Peyssonnaux and Eychene, Biology of the Cell, 2001, 93:3-62). Indeed, the Ras-dependent Raf-MEK-MAPK cascade is one of the key signalling pathways responsible for conveying both mitogenic and invasive signals from the cell surface to the nucleus resulting in changes in gene expression and cell fate.

A number of *NRAS* mutations have previously been identified as occurring in some cancers. However, the E63K mutation has not previously been described and the E63K / G12V mutations of the present invention have not previously been associated with resistance to EGFR inhibition in lung cancer therapy.

Without wishing to be bound by theory, it is believed that, in cells addicted to the EGFR pathway, inhibition of this pathway also inhibits the (downstream) Ras/Raf/MEK/ERK pathway. However, cells chronically treated with EGFR inhibitor find an alternative mechanism to circumvent EGFR inhibition, (e.g. by an activating mutation in *NRAS*), which enables the cells to survive in the absence of EGFR signalling, and so allows disease progression in a patient.

By detecting one or more of the present *NRAS* mutations in a patient (or more specifically, detecting the relevant mutation(s) in a suitable tissue or blood sample taken from a patient), it may be possible to identify patients who have developed, or who are predicted to develop, a form of cancer which is resistant to EGFR-inhibitor mediated therapy.

The inventors have further found that cells which have developed resistance to an EGFR inhibitor based on an *NRAS* mutation described herein surprisingly show increased sensitivity to a MEK inhibitor (which inhibits the Ras/Raf/MEK/ERK pathway) compared to both a parental cell line (sensitive to EGFR inhibitor and *NRAS* wild type) and compared to EGFR inhibitor-resistant cells lines which are NRAS wild type. Surprisingly, it appears that this increased sensitivity is dependent upon maintaining both EGFR inhibition in combination with MEK inhibition. Without wishing to be bound by theory, it is believed that EGFR-mutant cells may return to EGFR signalling in the absence of continued inhibition by an EGFR inhibitor, such that the cells are no longer sensitive to MEK inhibition.

EGFR inhibitor-resistant cancer patients bearing an *NRAS* mutation in their cancer as described herein may therefore benefit from treatment using a combination of an EGFR inhibitor and a MEK inhibitor. In this way, a combination of EGFR inhibitor and MEK inhibitor provides an effective follow-on (e.g. second+ line) therapy in cancer patients who have already received or are receiving EGFR inhibition therapy.

A combination of EGFR inhibitor and MEK inhibitor may also provide an effective first line therapy against EGFR-associated cancer, even in patients who have not yet been treated with an EGFR inhibitor. In such patients, the combination treatment may act to delay or prevent development of resistance based on activation of NRAS (and the Ras/Raf/MEK/ERK pathway).

More specifically, the present inventors have determined that EGFR-inhibitor resistant cell populations containing the NRAS E63K, G12V and G12R mutations each show sensitivity to treatment with a MEK inhibitor in combination with an EGFR inhibitor. As understood by the skilled person and explained in more detail hereinafter, involvement of G12V and G12R suggests that any single mutation at positions 288 or 289 in NRAS gene may result in sensitivity to a MEK inhibitor in combination with an EGFR inhibitor. Overall, the skilled person will understand that single mutations detectable at positions 288 or 289 of *NRAS* gene (for example, from a cDNA) corresponds to the NRAS protein mutations G12A, G12D, G12S and G12C in addition to the G12V and G12R NRAS protein mutations that have been experimentally described hereinafter. Accordingly, disclosed herein is a MEK inhibitor in combination with an EGFR inhibitor, for use in the treatment of EGFR-inhibitor resistant cancer involving the presence/detection of any of the aforementioned mutations.

Therefore, in one aspect, the disclosure provides a method for selecting a cancer patient suitable for treatment with a combination of an EGFR inhibitor and a MEK inhibitor, the method comprising;
(a) testing a cancer patient to determine the presence of an NRAS mutation; and
(b) selecting a patient as suitable for treatment with the combination of EGFR inhibitor and MEK inhibitor if an NRAS mutation is present;
wherein the NRAS mutation is selected from the group consisting of: E63K, G12V, G12R, G12A, G12D, G12S and G12C.

In one embodiment the NRAS mutation is selected from E63K, G12V and G12R.

As disclosed herein, the NRAS mutation is selected from G12V, G12R, G12A, G12D, G12S and G12C.

As disclosed herein, the NRAS mutation is selected from G12R, G12A, G12D, G12S and G12C.

In one embodiment the NRAS mutation is selected from G12V and G12R.

In one embodiment the NRAS mutation is G12R.

In one embodiment the EGFR inhibitor is selected from AZD9291 and CO 1686; or a pharmaceutically acceptable salt of either.

In a further embodiment the EGFR inhibitor is AZD9291 or a pharmaceutically acceptable salt thereof. In a further embodiment the EGFR inhibitor is AZD9291 or CO1686; or a pharmaceutically acceptable salt thereof, and the MEK inhibitor is selumetinib or a pharmaceutically acceptable salt thereof.

In a further embodiment the EGFR inhibitor is AZD9291 or a pharmaceutically acceptable salt thereof, and the MEK inhibitor is selumetinib or a pharmaceutically acceptable salt thereof.

In one aspect, the disclosure provides a method for selecting a cancer patient suitable for treatment with a combination of an EGFR inhibitor and a MEK inhibitor, the method comprising;
(a) testing a cancer patient to determine the presence of an NRAS activating mutation; and
(b) selecting a patient as suitable for treatment with the combination of EGFR inhibitor and MEK inhibitor if an NRAS activating mutation is present;
wherein the NRAS activating mutation is selected from the group consisting of: an NRAS E63K mutation; and an NRAS G12V mutation.

In a further aspect, the disclosure provides a method for selecting a cancer patient suitable for treatment with a combination of an EGFR inhibitor and a MEK inhibitor, the method comprising;
(a) testing a cancer patient to determine the presence of a gain of *NRAS* copy number; and
(b) selecting a patient as suitable for treatment with the combination of EGFR inhibitor and MEK inhibitor if a gain *of NRAS* copy number is present.

In a further aspect, the disclosure provides a method for selecting a suitable cancer treatment regimen for a cancer patient, the method comprising:
(a) determining the presence of an NRAS mutation in the patient; and
(b) selecting a cancer treatment regimen for the patient comprising provision of a combination of an EGFR inhibitor and a MEK inhibitor, if an NRAS mutation is present;
wherein the NRAS mutation is selected from the group consisting of: E63K, G12V, G12R, G12A, G12D, G12S and G12C.

In one embodiment the NRAS mutation is selected from E63K, G12V and G12R.

As disclosed herein, the NRAS mutation is selected from G12R, G12A, G12D, G12S and G12C.

As disclosed herein, the NRAS mutation is selected from G12V, G12R, G12A, G12D, G12S and G12C.

In one embodiment the NRAS mutation is selected from G12V and G12R.

In one embodiment the NRAS mutation is G12R.

In a further embodiment the EGFR inhibitor is selected from AZD9291 and CO1686; or a pharmaceutically acceptable salt of either.

In a further embodiment the EGFR inhibitor is AZD9291 or a pharmaceutically acceptable salt thereof. In a further embodiment the EGFR inhibitor is AZD9291 or CO1686; or a pharmaceutically acceptable salt thereof, and the MEK inhibitor is selumetinib or a pharmaceutically acceptable salt thereof.

In a further embodiment the EGFR inhibitor is AZD9291 or a pharmaceutically acceptable salt thereof, and the MEK inhibitor is selumetinib or a pharmaceutically acceptable salt thereof.

In a further aspect, the disclosure provides a method for selecting a suitable cancer treatment regimen for a cancer patient, the method comprising:
(a) determining the presence of an NRAS mutation in the patient; and
(b) selecting a cancer treatment regimen for the patient comprising provision of a combination of an EGFR inhibitor and a MEK inhibitor, if an NRAS mutation is present;
wherein the NRAS activating mutation is selected from the group consisting of: E63K, G12V, G12R, G12A, G12D, G12S and G12C.

In one embodiment the NRAS mutation is selected from E63K, G12V and G12R.

As disclosed herein, the NRAS mutation is selected from G12V, G12R, G12A, G12D, G12S and G12C.

As disclosed herein, the NRAS mutation is selected from G12R, G12A, G12D, G12S and G12C.

In one embodiment the NRAS mutation is selected from G12V and G12R.

In one embodiment the NRAS mutation is G12R.

In a further embodiment the EGFR inhibitor is selected from AZD9291 and CO1686; or a pharmaceutically acceptable salt of either.

In a further embodiment the EGFR inhibitor is AZD9291 or a pharmaceutically acceptable salt thereof. In a further embodiment the EGFR inhibitor is AZD9291 or CO1686; or a pharmaceutically acceptable salt thereof, and the MEK inhibitor is selumetinib or a pharmaceutically acceptable salt thereof.

In a further embodiment the EGFR inhibitor is AZD9291 or a pharmaceutically acceptable salt thereof, and the MEK inhibitor is selumetinib or a pharmaceutically acceptable salt thereof.

In a further aspect, the disclosure provides a method for selecting a suitable cancer treatment regimen for a cancer patient, the method comprising:
(a) determining the presence of an NRAS activating mutation in the patient; and
(b) selecting a cancer treatment regimen for the patient comprising provision of a combination of an EGFR inhibitor and a MEK inhibitor, if an NRAS activating mutation is present;
wherein the NRAS activating mutation is selected from the group consisting of: an NRAS E63K mutation; and an NRAS G12V mutation.

In a further aspect, the disclosure provides a method for selecting a suitable cancer treatment regimen for a cancer patient, the method comprising:
(a) determining the presence of a gain of *NRAS* copy number in the patient; and
(b) selecting a cancer treatment regimen for the patient comprising provision of a combination of an EGFR inhibitor and a MEK inhibitor, if an gain *of NRAS* copy number is present;

In a further aspect, the disclosure provides a method for predicting development of acquired resistance to the therapeutic effect of an EGFR inhibitor in cancer patient, the method comprising:
(a) determining the presence of an NRAS activating mutation in the patient; and
(b) identifying the cancer as one that has become or will become resistant if an NRAS activating mutation is present;
wherein the NRAS activating mutation is selected from the group consisting of: an NRAS E63K mutation; and an NRAS G12V mutation.

In a further aspect, the disclosure provides a method for predicting development of acquired resistance to the therapeutic effect of an EGFR inhibitor in cancer patient, the method comprising:
(a) determining the presence of a gain *of NRAS* copy number in the patient; and
(b) identifying the cancer as one that has become or will become resistant if a gain of *NRAS* copy number is present.

In particular disclosures of any of the aspects of the invention, the patient has received or is receiving treatment with an EGFR inhibitor. In other disclosures, determination of the *NRAS* gene/NRAS protein anomaly in the patient is carried out on a suitable biological sample obtained from the patient. This sample may have been taken previous to or as part of the treatment method. This sample will typically contain tumour cells, tumour nucleic acid or nucleic acid derived therefrom.

In a further aspect, the invention provides a method for determining the likelihood of effectiveness of a combination treatment comprising an EGFR inhibitor and a MEK inhibitor to treat cancer in a patient affected with cancer comprising: determining whether the *NRAS* gene obtained from a biological sample obtained from said patient comprises at least one nucleic acid variance, selected from:
a) a mutation that results in the presence of lysine in place of glutamic acid at position 63 of NRAS;
b) a mutation that results in the presence of valine, arginine, alanine, aspartic acid, serine, or cysteine in place of glycine at position 12 of NRAS; or
c) an increase in copy number *of NRAS* gene;
wherein the biological sample is a sample of tissue or fluid isolated from the patient that comprises tumour cells or nucleic acid from tumour cells and wherein the presence of the at least one nucleic acid variance indicates that the combination treatment comprising an EGFR tyrosine kinase inhibitor and a MEK inhibitor is likely to be effective.

Accordingly, the invention provides a method for determining the likelihood of effectiveness of a combination treatment comprising an EGFR tyrosine kinase inhibitor and a MEK inhibitor to treat cancer in a patient affected with cancer comprising: determining whether the *NRAS* gene obtained from a biological sample obtained from said patient comprises at least one nucleic acid variance, selected from:
a) presence of adenine in place of guanine at a position corresponding to base 441 in *NRAS* cDNA;
b) presence of a nucleic acid other than guanine at a position corresponding to base 288 in *NRAS* cDNA;
c) presence of a nucleic acid other than guanine at a position corresponding to base 289 in *NRAS* cDNA; or
d) an increase in copy number *of NRAS* gene;
wherein the biological sample is a sample of tissue or fluid isolated from the patient that comprises tumour cells or nucleic acid from tumour cells and wherein the presence of the at least one nucleic acid variance indicates that the combination treatment comprising an EGFR tyrosine kinase inhibitor and a MEK inhibitor is likely to be effective.

In one embodiment the at least one nucleic acid variance is selected from:
a) presence of adenine in place of guanine at a position corresponding to base 441 in *NRAS* cDNA;
b) presence of thymine in place of guanine at a position corresponding to base 289 in *NRAS* cDNA; or
c) an increase in copy number of *NRAS* gene.

In one embodiment the at least one nucleic acid variance is selected from:
a) presence of adenine in place of guanine at a position corresponding to base 441 in *NRAS* cDNA;
b) presence of thymine in place of guanine at a position corresponding to base 289 in *NRAS* cDNA;
c) presence of cytosine in place of guanine at a position corresponding to base 288 in *NRAS* cDNA; or
d) an increase in copy number *of NRAS* gene.

In a further embodiment the EGFR inhibitor is selected from AZD9291 and CO1686; or a pharmaceutically acceptable salt of either.

In a further embodiment the EGFR inhibitor is AZD9291 or a pharmaceutically acceptable salt thereof. In a further embodiment the EGFR inhibitor is AZD9291 or CO1686; or a pharmaceutically acceptable salt thereof, and the MEK inhibitor is selumetinib or a pharmaceutically acceptable salt thereof.

In a further embodiment the EGFR inhibitor is AZD9291 or a pharmaceutically acceptable salt thereof, and the MEK inhibitor is selumetinib or a pharmaceutically acceptable salt thereof.

In a further aspect, the invention provides a method for determining the likelihood of effectiveness of a combination treatment comprising an EGFR tyrosine kinase inhibitor and a MEK inhibitor to treat cancer in a patient affected with cancer comprising: determining whether the *NRAS* gene obtained from a biological sample obtained from said patient comprises at least one nucleic acid variance, selected from:
a) a mutation that results in a substitution of lysine for glutamic acid at position 63 of NRAS or in a substitution of valine for glycine at position 12 of NRAS; or
b) an increase in copy number *of NRAS* gene;
wherein the biological sample is a sample of tissue or fluid isolated from the patient that comprises tumour cells or nucleic acid from tumour cells and wherein the presence of the at least one nucleic acid variance indicates that the combination treatment comprising an EGFR tyrosine kinase inhibitor and a MEK inhibitor is likely to be effective.

In a further aspect, the invention provides a method for determining the likelihood of effectiveness of a combination treatment comprising an EGFR tyrosine kinase inhibitor and a MEK inhibitor to treat cancer in a patient affected with cancer comprising: determining whether the *NRAS* gene or protein obtained from a biological sample obtained from said patient comprises at least one variance, selected from:
a) a mutation that results in a substitution of lysine for glutamic acid at position 63 of NRAS or in a substitution of valine for glycine at position 12 of NRAS; or
b) an increase in copy number *of NRAS* gene;
wherein the biological sample is a sample of tissue or fluid isolated from the patient that comprises tumour cells or nucleic acid from tumour cells and wherein the presence of the at least one nucleic acid variance indicates that the combination treatment comprising an EGFR tyrosine kinase inhibitor and a MEK inhibitor is likely to be effective.

As disclosed herein, prior to determining whether or not the sample from the patient comprises the at least one nucleic acid variance the patient has been treated with an EGFR inhibitor.

### Description of the Figures

**Figure 1****: Time to resistance** - A plot of gefitinib concentration vs time taken for PC9 cells, grown in the presence of gefitinib at that concentration, to reach about 80% confluency (gefitinib concentration was doubled each time this confluency was reached).
**Figure 2****: Example dose response curve** - An example dose response curve for PC9 NRAS (WT) cells treated with titrations of selumetinib.
**Figure 3****: Example dose response curve** - An example dose response curve for PC9 AZD9291 resistant NRAS (E63K) cells treated with titrations of selumetinib.
**Figure 4****: Example dose response curve** - An example dose response curve for PC9_gefitinib resistant NRAS E63K cells treated with titrations of selumetinib.
**Figure 5****: Example dose response curve** - An example dose response curve for PC9_afatinib resistant NRAS (WT) gain cells treated with titrations of selumetinib.
**Figure 6****: Example dose response curve** - An example dose response curve for PC9_AZD9291 resistant NRAS (G12V) cells treated with titrations of selumetinib.
**Figure 7****: Ras activation assay** - Western blot analysis showing active NRAS level,s in PC9 and PC9 AZD9291-resistant cell lines with NRAS E63K (PC9 AZDR_5 cells) and G12V (PC9 AZDR_2 cells) mutations, following treatment for 2 hours with or without 160nM AZD929.
**Figure 8****: Effect of siRNA knockdown of WT NRAS and E63K NRAS on downstream signalling** - The effects of 48 hours siRNA-mediated knockdown of NRAS or KRAS expression on: phosphorylation indicative of activity of proteins: phosphorylated EGFR (P EGFR); phosphorylated ERK (P ERK); NRAS; KRAS; and GAPDH (loading control); in PC9 cells, in PC9 AZD9291-resistant cells (AKA. PC9 AZDR_5) having an NRAS E63K mutation; and in PC9 gefitinib-resistant cells (AKA. PC9 GR_2) having an NRAS E63K mutation.
**Figure 9****: Effect of siRNA knockdown of WT NRAS and E63K NRAS on proliferation** - The effects of 96 hours siRNA-mediated knockdown of NRAS or KRAS expression on cell growth in PC9 cells, in PC9 AZD9291-resistant cells (AKA. PC9 AZDR_5) having an NRAS E63K mutation; and in PC9 gefitinib-resistant cells (AKA. PC9 GR_2) having an NRAS E63K mutation.
**Figure 10****: Effect of exogenous over expression of E63K NRAS on resistance to growth inhibition by gefitinib or AZD9291 in PC9 cells** - The effects of over expression of E63K mutant NRAS on growth of PC9 cells in the absence and presence of either 100nM AZD9291 or 300nM gefitinib.

### Description of the sequences

Various sequences are referred to in the present specification. In particular:
SEQ ID NO:1 provides the amino acid sequence of the EGFR;
SEQ ID NO:2 provides the sequence of cDNA encoding the EGFR;
SEQ ID NO:3 provides the amino acid sequence of NRAS;
SEQ ID NO:4 provides the sequence of cDNA encoding NRAS;
SEQ ID NO:5 provides the amino acid sequence of MEK1;
SEQ ID NO:6 provides the sequence of cDNA encoding MEK1;
SEQ ID NO:7 provides the amino acid sequence of MEK2; and
SEQ ID NO:8 provides the sequence of cDNA encoding MEK2.

### Detailed description of the invention

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. Accordingly, all embodiments, definitions, aspects and claims described herein may be combined with each other in any combination, (except where such combination would clearly be inappropriate, considering the context), in order to provide further embodiments, aspects or claims.

Throughout the description and claims of this specification, the words "comprise" and "contain" and variations of the words, for example "comprising" and "comprises", can mean "including but not limited to", and do not in general exclude other moieties, additives, components, integers or steps.

Unless otherwise noted, technical terms are used according to conventional usage. According to convention, it is usual to recite genes in italics and proteins in normal text. According to well-established convention in this technical field, in this document the names of genes are written in italicised text, and the names of proteins are written in normal text (i.e. not italicised). Therefore, "NRAS" is used to indicate the NRAS protein and "*NRAS*" is used to indicate the gene. Whilst efforts have been made in this document to indicate the *NRAS* gene or NRAS protein according to this convention, the person skilled in the art will understand, based on the scientific context, whether any of the references to "*NRAS*" or "NRAS" are incorrect.

Definitions of common terms in molecular biology may be found in Benjamin Lewin, Genes V, published by Oxford University Press, 1994 (ISBN 0-19- 854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

As described herein above, the present inventors have determined that resistance to EGFR-inhibitor mediated therapy in cancer patients may be associated with one or more NRAS mutations, selected from the group consisting of: an NRAS E63K mutation; and an NRAS G12V mutation. These mutations are believed to be NRAS activating mutations. The inventors have also found that resistance to EGFR-inhibitor mediated therapy in cancer patients may be associated with a gain *of NRAS* copy number. Moreover, the inventors have found that cancer patients who harbour one or more of these NRAS activating mutations or gains *of NRAS* copy number may be effectively treated with a combination of EGFR inhibitor and MEK inhibitor, even if the patient displays (or would display) resistance to EGFR-inhibitor-mediated therapy alone. Furthermore, the inventors have found that cancer patients who harbour the G12R NRAS mutation may be effectively treated with a combination of EGFR inhibitor and MEK inhibitor, even if the patient displays (or would display) resistance to EGFR-inhibitor-mediated therapy alone. The present invention allows more effective targeting of treatments to cancer patients, and in particular, more effective identification and treatment of cancers which have developed, or will develop, resistance to the effects of an EGFR inhibitor.

In one aspect therefore the methods of the disclosure can be used to predict development of acquired resistance to the therapeutic effect of an EGFR inhibitor in cancer patients. In such a method, a patient is tested to determine the presence of an NRAS mutation or a gain *of NRAS* copy number as described herein, and a cancer is identified as developing acquired resistance if a patient is shown to harbour an NRAS activating mutation or a gain *of NRAS* copy number according to the invention. Such a patient may be described as positive for an NRAS activating mutation or gain *of NRAS* copy number described herein. In a further aspect the methods of the disclosure can be used to select cancer patients suitable for treatment with a combination of EGFR inhibitor and MEK inhibitor. In such a method, a cancer patient is tested to determine the presence of an NRAS mutation or a gain *of NRAS* copy number as described herein, and is selected as suitable for treatment if one or more of the NRAS mutations describe herein, or a gain of *NRAS* copy number is present. As disclosed herein, when tested to determine the presence of an NRAS mutation or a gain of *NRAS* copy number, the cancer patient is one who has received or is receiving treatment with an EGFR inhibitor.

The methods of the disclosure can also be used to select a suitable cancer treatment regimen for a cancer patient. In this method, a cancer patient is tested to determine the presence of an NRAS mutation as described herein or a gain of *NRAS* copy number, and a treatment regimen comprising provision of a combination of an EGFR inhibitor and a MEK inhibitor is selected if an NRAS mutation or a gain of *NRAS* copy number is present. As disclosed herein, when tested to determine the presence of an NRAS mutation, the cancer patient is one who has received or is receiving treatment with an EGFR inhibitor.

### Cancer

A number of cancers are associated with aberrant or over-expression of EGFR and/or over-expression of its specific ligands, e.g. transforming growth factor receptor α (Gullick, Br Med Bull. 47:87-98, 1991; Modijtahedi and Dean, Int. J. Oncol. 4:277-296, 1994; Salomon et al., Crit Rev Oncol Hematol.19:183-232, 1995). EGFR over-expression has been associated with an adverse prognosis in a number of human cancers, including NSCLC. As disclosed herein, "cancer" refers to an EGFR-associated cancer.

Methods for identifying and diagnosing EGFR-associated cancers are well known to those skilled in the art. By way of example, details of various methods and the circumstances in which they may be used are provided in Ellison, et al. J Clin Pathol 2013;66:2 79-89. For example, an EGFR-associated cancer may be one which has been, or which is, responsive to treatment with an EGFR inhibitor, such as any of the EGFR inhibitors described herein.

An EGFR-associated cancer may be one which has become or which will become resistant to the therapeutic effect of one or more EGFR inhibitors as described herein. Such resistance may be associated with a mutation in EGFR, such as T790M.

An EGFR-associated cancer may be a cancer (or tumour) which is (or which was) dependent at least in part upon the EGFR signalling pathway.

An EGFR-associated cancer may be associated with one or more mutations in EGFR. Such an EGFR mutation may transform the cell to a cancerous state.

An EGFR mutation which is associated with cancer may be an EGFR-activating mutation. Such a mutation generally results in over-expression and/or over-activity of the EGFR protein. Any activity of EGFR may be affected. Mutation may for example, occur in a nucleotide sequence, e.g. in DNA encoding EGFR and/or in an amino acid sequence, e.g. in the amino acid sequence of EGFR protein. Mutation may occur in a nucleotide sequence encoding a different protein, or in the amino acid sequence of a different protein, wherein the effect of the mutation is to produce over-expression and/or over-activity of the EGFR protein. Typically a mutation is a somatic mutation.

A mutation may, for example, be a somatic gain of function mutation in the tyrosine kinase domain of EGFR. Some such mutations enhance sensitivity of the cancer to an EGFR tyrosine kinase inhibitor. These mutations generally reside within exons 18-21 of EGFR gene. See, e.g. WO2005/094397 and WO2005/118876. Examples of these mutations include multi-nucleotide in-frame deletions in exon 19, involving elimination of four amino acids, Leu-Arg-Glu-Ala, and a single nucleotide substitution at nucleotide 2573 (T→G) in exon 21, resulting in substitution of arginine for leucine at position 858 (L858R). These mutations are particularly associated with lung cancer, e.g. non-small cell lung cancer (NSCLC).

L858R is one of a number of EGFR mutations of particular interest in the context of the present invention, because it is frequently associated with cancers that respond to EGFR tyrosine kinase inhibitors such as erlotinib, gefitinib, afatinib and AZD9291 (further details included elsewhere herein). In one embodiment of the invention, the EGFR-associated cancer may be a cancer that is associated with an EGFR mutation selected from the group consisting of: L858R; and exon 19 deletion mutations.

Additionally or alternatively, an EGFR-associated cancer may be associated with an EGFR T790M mutation. This mutation is often seen as a second site mutation in EGFR, occurring in addition to a first primary activating mutation. Typically the secondary mutation arises during treatment with an EGFR inhibitor, and provides a mechanism of resistance to the inhibitor. The T790M mutation is particularly associated with lung cancer, e.g. NSCLC. AZD9291 may be used for treatment of EGFR-associated cancers associated with exon 19 deletion mutations, and/or L858R substitution mutations, and optionally T790M resistance mutation.

Examples of EGFR-associated cancers may include: non-solid tumours such as leukaemia, for example acute myeloid leukaemia, multiple myeloma, haematologic malignancies (e.g. myelodysplastic syndrome or myeloproliferative syndrome) or lymphoma; and solid tumours and their metastases such as melanoma, non-small cell lung cancer (NSCLC), glioma, hepatocellular (liver) carcinoma, glioblastoma, carcinoma of the thyroid, bile duct, bone, gastric, brain/CNS, head and neck, hepatic, stomach, prostate, breast, renal, testicular, ovarian, skin, cervical, lung, muscle, neuronal, oesophageal, bladder, lung, uterine, vulval, endometrial, kidney, colorectal, pancreatic, pleural/peritoneal membranes, salivary gland, and epidermoid tumours.

As disclosed herein, the EGFR-associated cancer is a solid tumour.

As disclosed herein, the EGFR-associated cancer is lung cancer.

In one embodiment the EGFR-associated cancer is NSCLC.

The use of the methods, medical uses, pharmaceutical compositions, and other aspects of the disclosure in the context of cancers selected from this group, or metastases of such cancers, is to be considered a suitable aspect of the disclosure.

### Cancer patient

The invention may be practised in any suitable organism. In one aspect, the patient is an animal or a human, for example, a mammal, such as a warm-blooded mammal. In one aspect, the patient is a human.

Typically a patient displays one or more clinical or pre-clinical symptoms of an EGFR-associated cancer described herein. A patient may have been clinically diagnosed as suffering from such a cancer, or as susceptible to such a cancer.

A patient may be receiving, or may have received, cancer therapy with an EGFR inhibitor, such as an EGFR TKI. A patient may be one who has already developed, or who is likely to develop, acquired resistance to therapy with an EGFR inhibitor, as described herein. Thus the present methods may be used to provide second or subsequent line therapy.

### Resistance and acquired resistance

As used herein, resistance to a treatment describes a clinical situation wherein a cancer (or a cancer patient) is not responsive to the treatment, or is less responsive than when the treatment was initially applied (often referred to as acquired resistance).

Acquired resistance refers to a situation in which a cancer (or a cancer patient) is initially responsive to treatment with an agent (e.g. an EGFR inhibitor), but where over time, the cancer (or patient) becomes less or non responsive to further treatment with the agent, and the cancer progresses. A cancer or cancer patient may exhibit degrees of resistance (and so degrees of responsiveness to treatment) over time.

By responsive is meant that the treatment produces at least one clinically detectable therapeutic effect as described herein. A patient is non-responsive if there is no clinically detectable therapeutic effect as described herein. A therapeutic effect may be for example an anti-cancer effect as described herein.

A cancer which is predicted as developing acquired resistance to an EGFR inhibitor in accordance with the invention is a cancer which has already become, or which will become, resistant to treatment with an EGFR inhibitor.

### Treatment, therapy and therapeutic effect

The terms treatment, therapy or therapeutic effect as used herein include the following and combinations thereof: (1) inhibiting, e.g. delaying initiation and/or progression of, cancer, for example arresting, reducing or delaying the development of cancer, or a relapse thereof in case of maintenance treatment or secondary prophylaxis, or of at least one clinical or subclinical symptom thereof; (2) preventing or delaying the appearance of clinical symptoms of cancer developing in a subject that may be afflicted with or predisposed to cancer but does not yet experience or display clinical or subclinical symptoms of cancer; and/or (3) relieving and/or curing cancer (*e.g.*, causing regression of cancer or at least one of its clinical or subclinical symptoms, curing a patient or putting a patient into remission). A treatment or therapy as used herein may include prophylaxis or prophylactic treatment. Thus, for example, therapeutic effect herein may refer to one or more of (1), (2) and (3) occurring in a cancer patient.

A therapeutic effect herein may be an anti-cancer effect. Anti-cancer effects include but are not limited to, inhibition of tumour growth, tumour growth delay, regression of tumour, shrinkage of tumour, increased time to regrowth of tumour on cessation of treatment, slowing of disease progression.

A therapeutic effect in a first line therapy referred to herein may refer to delaying or preventing development of acquired resistance to EGFR-inhibitor mediated therapy, which may, for example, be acquired by development of an NRAS mutation described herein. Typically a therapeutic effect provides a benefit that is clinically detectable. The benefit to a subject or patient to be treated may be either statistically significant or at least perceptible to the patient or to the physician or other skilled person. It will be understood that a medicament will not necessarily produce a clinical effect in each patient to whom it is administered; thus, in any individual patient or even in a particular patient population, a treatment may fail or be successful only in part, and the meanings of the terms "treatment", "prophylaxis" and "inhibitor" and of cognate terms are to be understood accordingly.

The term "prophylaxis" or "prophylactic treatment" includes reference to treatments or therapies for the purpose of preserving health or inhibiting or delaying the initiation and/or progression of cancer, for example for the purpose of reducing the chance of, or preventing, cancer occurring. The outcome of the prophylaxis may be, for example, preservation of health or delaying the initiation and/or progression of cancer. It will be recalled that, in any individual patient or even in a particular patient population, a treatment may fail, and this paragraph is to be understood accordingly.

Treatment of cancer according to the invention may be assessed by conventional means such as the extent of the anti-tumour effect, the response rate, the time to disease progression and/or the survival rate.

A first line therapy for EGFR-associated cancer herein refers to therapy administered to a patient who has not previously been treated for the cancer using an EGFR inhibitor.

A second line therapy for EGFR-associated cancer herein refers to therapy administered to a patient who has previously been treated for the cancer using an EGFR inhibitor, but where the EGFR-associated cancer had progressed (or otherwise become resistant) during treatment with the EGFR inhibitor.

A third line therapy for EGFR-associated cancer herein refers to therapy administered to a patient who has previously been treated for the cancer, in particular, who has previously been treated with two different therapies, for example different EGFR inhibitors.

The inventors believe that the methods of treatment and/or medical uses disclosed herein may, as necessary, be applicable in the context of first, second, third or further line therapies for EGFR-associated cancers.

### EGFR/EGFR

EGFR as used herein refers to epidermal growth factor receptor, in general a transmembrane glycoprotein that is a member of the protein kinase superfamily.

EGFR may be of any species corresponding to the species to be treated. In one aspect, EGFR is human EGFR.

Human EGFR (Gene ID: 1956) exists in different isoforms according to alternatively spliced transcripts. EGFR as used herein may refer to any of these isoforms.

A wild-type human EGFR gene sequence is described in Gene ID: 1956. The EGFR gene may have the mRNA expression product corresponding to the cDNA sequence in GenBank Accession No. X00588.1, and protein expression product having the EGFR protein amino acid sequence in UniProtKB/Swiss-Prot P00533.2 (this includes a 24 amino acid signal sequence at amino acids 1-24 which is cleaved in the mature protein). Reference herein to EGFR gene, mRNA, cDNA or EGFR protein may refer to this human gene, mRNA (or corresponding cDNA) or protein, with additional mutation as the context allows. An EGFR mRNA, cDNA or protein referred to herein may also refer to an isoform of the foregoing, e.g. a soluble isoform lacking a transmembrane domain and intracellular domain, with additional mutation as the context allows.

EGFR may in some instances refer to a variant of the above, for example, a naturally occurring wild type variant (e.g. from a non-tumourous cell).

The above applies mutatis mutandis for EGFR of a non-human species. For example, reference herein to EGFR gene, mRNA or EGFR protein may refer to a species homolog of the above human gene, mRNA or protein, with additional mutation as the context allows.

Amino acid sequence numbering for EGFR and EGFR mutations as used herein is generally with reference to the amino acid sequence and numbering of wild type human EGFR as presented in UniProtKB/Swiss-Prot P00533.2 (which as above includes a signal sequence at amino acids 1-24). Thus for example, reference to a T790M mutation refers to a change from T to M at amino acid 790 in the 1210 amino acid sequence in UniProtKB/Swiss-Prot P00533.2. Because of the reference sequence used, the T790M mutation was initially referred to as T766M (Blencke et al. J. Biol. Chem. 278:15435-15440, 2003).

Similarly, EGFR cDNA sequence numbering as used herein is with reference to the wild type EGFR cDNA sequence in GenBank X00588.1 (with protein coding sequence from bases 187-3819)..

Reference to EGFR mutations herein may also encompass mutations occurring at corresponding positions in EGFR of a non-human species having the same functional effect.

### Inhibitor

An inhibitor as referred to herein may be, for example, a polypeptide, nucleic acid, carbohydrate, lipid, small molecular weight compound, an oligonucleotide, an oligopeptide, siRNA, antisense, a recombinant protein, an antibody, a peptibody, or conjugates or fusion proteins thereof. For a review of siRNA see Milhavet O, Gary DS, Mattson MP. (Pharmacol Rev. 2003 Dec;55(4):629-48. For a review of antisense see Opalinska JB, Gewirtz AM. Sci STKE. 2003 Oct 28; 2003 (206): pe47.

A small molecular weight compound may for example refer to a compound with a molecular weight of less than 2000 Daltons, 1000 Daltons, 700 Daltons or 500 Daltons. Particular inhibitors for use in the present invention are small molecule EGFR TKIs.

### EGFR inhibitor

An EGFR inhibitor generally reduces or prevents expression and/or activity of EGFR. A suitable inhibitor may be identified by assaying for inhibitory effect on EGFR expression and/or activity.

Typically an inhibitor reduces expression or activity by at least a detectable amount in a suitable assay. An inhibitor may, for example, reduce expression or activity by at least 10, 20, 30, 40, 50, 60, 70, 80 or at least 90%.

Many suitable strategies by which expression of EGFR may be reduced are known to those skilled in the art. Furthermore, the skilled person will also be aware of many agents able to reduce EGFR activity, including, but not limited, to the examples provided elsewhere in this specification.

Any suitable EGFR activity may be affected, for example, tyrosine kinase activity. Thus in one aspect, an EGFR inhibitor may comprise a tyrosine kinase inhibitor (TKI). Inhibition of tyrosine kinase activity may be determined in any suitable assay, such as the assay described in Ward et.al. J. Med. Chem. 2013, 56:7025-7048.

Examples of EGFR tyrosine kinase inhibitors include: gefitinib, erlotinib, afatinib , AZD9291, CO-1686, WZ4002, PD153035, and PF 00299804. Inhibitors may include pharmaceutically acceptable salts of these compounds, as described elsewhere herein. Such inhibitors are well known to those of skill in the art.

Established EGFR TKIs such as gefitinib, erlotinib and afatinib may be used as a first line therapy against cancer in patients. More recently developed EGFR TKIs, including AZD9291, CO-1686, WZ4002, can sometimes be used against EGFR-associated cancer where resistance has developed to one of the more established EGFR TKI drugs (for example, against cancer associated with both a primary EGFR activating mutation and an additional EGFR mutation, such as T790M).

Of the EGFR TKIs set out above, the group consisting of: gefitinib; erlotinib; afatinib; AZD9291; and CO-1686 represent examples of particular interest in the context of the present invention, (where it is understood by the skilled person that such EGFR TKIs may each optionally be used in the form of a pharmaceutically acceptable salt). The use of an EGFR TKI selected from this group represents a suitable embodiment of the present invention. Therefore, in any embodiment, aspect or claim where an EGFR inhibitor is mentioned, further embodiments, aspects and claims may be formed where the EGFR inhibitor is selected from gefitinib, erlotinib, afatinib, AZD9291 and CO1686, or a pharmaceutically acceptable salt of any thereof. Further embodiments, aspects and claims may be formed where the EGFR inhibitor is selected from gefitinib, AZD9291 and C01686.

### Gefitinib

An EGFR inhibitor may comprise gefitinib or a pharmaceutically acceptable salt thereof. Gefitinib is an EGFR TKI drug that is approved for use in advanced NSCLC in a number of territories.

The gefitinib structure is shown below:

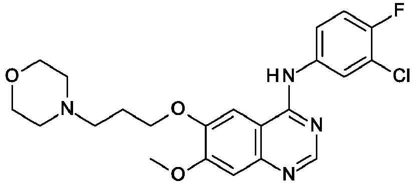

Gefitinib may also be known by the chemical name *N*-(3-chloro-4-fluorophenyl)-7-methoxy-6-(3-morpholin-4-ylpropoxy)quinazolin-4-amine.

### Erlotinib

An EGFR inhibitor may comprise erlotinib or a pharmaceutically acceptable salt thereof. Erlotinib is an EGFR TKI drug used against pancreatic, lung and other cancers as described herein.

The erlotinib structure is shown below:

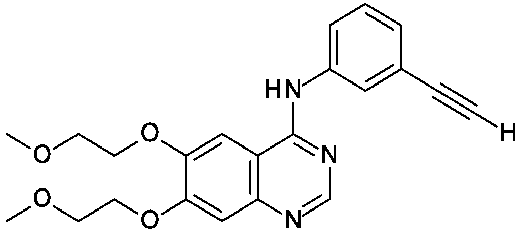

Erlotinib may also be known by the chemical name *N*-(3-ethynylphenyl)-6,7-bis(2-methoxyethoxy)
quinazolin-4-amine.

### Afatinib

An EGFR inhibitor may comprise afatinib or a pharmaceutically acceptable salt thereof. Afatinib is an EGFR TKI drug used against NSCLC in particular, as described herein. The drug can sometimes be used to treat cancers which are, or have become, resistant to treatment with gefitinib or erlotinib.

The afatinib structure is shown below:

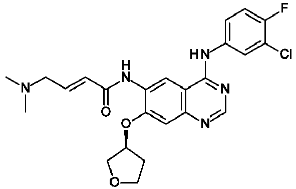

Afatinib may also be known by the chemical name *N*-[4-[(3-chloro-4-fluorophenyl)amino]-7-[[(3*S*)-tetrahydro-3-furanyl]oxy]-6-quinazolinyl]-4(dimethylamino)-2-butenamide.

### AZD9291

An EGFR inhibitor may comprise AZD9291 or a pharmaceutically acceptable salt thereof. AZD9291 may sometimes be used to treat cancers which are, or have become, resistant to treatment with EGFR inhibitors such as gefitinib, erlotinib, and/or afatinib.

AZD9291 has the structure: 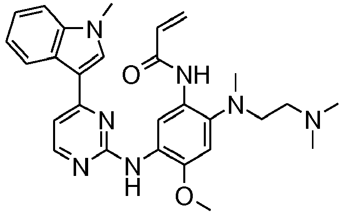 and may be known by the chemical name: '*N*-(2-{2-dimethylaminoethyl-methylamino}-4-methoxy-5-{[4-(1-methylindol-3-yl)pyrimidin-2-yl]amino}phenyl)prop-2-enamide'. AZD9291 and pharmaceutically acceptable salts thereof are disclosed in international patent application number PCT/GB2012/051783 (publication number WO2013/014448). A pharmaceutically acceptable salt of AZD9291 may comprise any of the salts described herein, for example a mesylate salt.

### CO-1686 - also known as "rociletinib"

An EGFR inhibitor may comprise CO-1686 or a pharmaceutically acceptable salt thereof. The chemical structure of CO-1686 is:

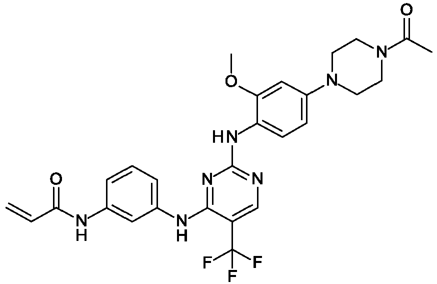

CO-1686 may be known by the chemical name: N-(3-{[2-{[4-(4-acetylpiperazin-1-yl)-2-methoxyphenyl] amino } -5 -(trifluoromethyl)pyrimidin-4-yl] amino } phenyl)prop-2-enamide. A pharmaceutically acceptable salt of CO-1686 may, for example, comprise a HBr salt.

### WZ4002

Is a mutant-selective EGFR tyrosine kinase inhibitor effective against EGFR T790M. This compound is described in a publication by Zhou et al. (Nature 462, 1070-1074; 2009)

### NRAS/NRAS

NRAS as used herein refers to Neuroblastoma RAS viral oncogene homolog, and is encoded by the *NRAS* oncogene.

NRAS is a member of the Ras gene family. The mammalian Ras gene family includes the Harvey and Kirsten ras genes (HRAS and KRAS), an inactive pseudogene of each (c-Hras2 and c-Kras1) and the *NRAS* gene. The RAS proteins have GTP-GDP binding and GTPase activity.

NRAS may be of any species corresponding to the species to be treated. In one aspect, NRAS is human NRAS.

A (wild type) human *NRAS* (*NRAS*) gene is described in Gene ID: 4893, and consists of 7 exons (-I, 1, II, II, IV, V, VI). The wild type human *NRAS* gene typically has an mRNA expression product corresponding to the cDNA sequence in GenBank Accession No. NM_002524, and protein expression product having the NRAS protein amino acid sequence in UniProtKB/Swiss-Prot P01111.1.

Reference herein to *NRAS* gene, mRNA, cDNA or NRAS protein may refer to this human gene, mRNA or corresponding cDNA sequence, or protein, with additional mutation as the context allows.

NRAS may in some instances refer to a variant of any of the above, for example, a naturally occurring wild-type variant.

The above applies mutatis mutandis for NRAS of a non-human species. For example, reference herein to *NRAS* gene, mRNA, cDNA or NRAS protein may refer to a species homolog of the above human gene, mRNA or protein, with additional mutation as the context allows.

NRAS amino acid numbering and mutations are described herein with reference to the amino acid sequence in UniProtKB/Swiss-Prot P01111.1. Thus, for example, reference herein to NRAS mutation E63K refers to a change from glutamic acid (E) to lysine (K) at amino acid number 63 in the 189 amino acid sequence in UniProtKB/Swiss-Prot P01111.1. In the *NRAS* cDNA sequence in GenBank Accession No. NM_002524, the protein coding sequence is shown at nucleotides 255-824. The 570 nucleotide consenus coding sequence is also presented in the CCDS database under Accession No. CCDS877.1. *NRAS* cDNA sequence numbering and mutations described herein are with reference to the cDNA sequence in GenBank Accession No. NM_002524.

Reference to NRAS mutations herein may also encompass mutations occurring at corresponding positions in NRAS of a non-human species having the same functional effect.

### NRAS activating mutation

An NRAS activating mutation as used herein generally results in over expression and/or over activity of NRAS protein. A mutation may for example occur in a nucleotide sequence (e.g. of DNA encoding NRAS) and/or in an amino acid sequence, (e.g. in the NRAS protein sequence). An NRAS mutation described herein is typically a somatic mutation.

Any activity of NRAS may be affected, for example, by GTPase activity.

NRAS activating mutations described herein include: an NRAS E63K mutation; an NRAS G12V mutation. An NRAS activating mutation described herein may arise spontaneously with cancer development or it may be acquired in a cancer (or a cancer patient) in response to treatment of the cancer (or patient), such as with an EGFR inhibitor. A further NRAS mutation described herein is a G12R NRAS mutation.

### NRAS E63K mutation

An E63K NRAS mutation refers to a change in amino acid from glutamic acid (E) to lysine (K) at amino acid position corresponding to E63 in NRAS protein amino acid sequence (SEQ ID NO:3).

The change in amino acid is typically encoded by a change in the DNA sequence in the *NRAS* gene. Typically this is a change in codon 63 of the coding sequence (nucleotides 441-443 of the *NRAS* cDNA sequence). In particular, the change may result from a change from G to A at a position corresponding to base 441 in the *NRAS* cDNA sequence, meaning a change from GAG to AAG at a position corresponding to bases 441 to 443. (It may also arise from a double nucleobase substitution, such as from GAG to AAA at a position corresponding to bases 441 to 443 of the *NRAS* cDNA sequence).

### NRAS G12V mutation

A G12V NRAS mutation refers to a change in amino acid sequence from glycine (G) to valine (V) at amino acid position corresponding to G12 in NRAS protein amino acid sequence (SEQ ID NO:3).

The change in amino acid is typically encoded by a change in the DNA sequence in the *NRAS* gene. Typically this is a change in codon 12 of the coding sequence (nucleotides 288-290 of the *NRAS* cDNA sequence). In particular, the change may result from a change from G to T at a position corresponding to base 289 in the *NRAS* cDNA sequence, meaning a change from GGT to GTT for bases 288 to 290.

### NRAS G12R mutation

A G12R NRAS mutation refers to a change in amino acid sequence from glycine (G) to arginine (R) at amino acid position corresponding to G12 in NRAS protein amino acid sequence (SEQ ID NO:3). The change in amino acid is typically encoded by a change in the DNA sequence in the *NRAS* gene. Typically this is a change in codon 12 of the coding sequence (nucleotides 288-290 of the *NRAS* cDNA sequence). In particular, the change may result from a change from G to C at the position corresponding to base 288 in the *NRAS* cDNA sequence, meaning a change from GGT to CGT for bases 288 to 290.

### NRAS gain of copy number

An *NRAS* (N-ras) gain of copy number refers to a change in the DNA which results in the presence of an increased number of copies of the *NRAS* gene as compared to control cells. A control cell could be a normal non-cancerous cell or a matched normal control cell, i.e. a non-cancerous cell/cell population taken from the same patient. The increased number of copies of the *NRAS* gene may also be gauged relative to a reference number of copies indicative of normal situation.

For example, an amplifying mutation may result in an increase in *NRAS* gene copy number of at least 1 compared to control cells, such as at least 2, 3, 4, 5, 6, 7 or 8 compared to cells lacking the gain of copy number. Gene copy number may show a fold increase of greater than 1, greater than 2, greater than 3 or greater than 4.

### Detecting mutations and gain of copy number

According to one aspect of the invention, patients are tested for the presence of an NRAS activating mutation selected from any one or more of: an NRAS E63K mutation; and an NRAS G12V mutation.

According to a further aspect of the invention, patients are tested for the presence of a gain *of NRAS* copy number.

Suitable techniques by which mutations or gain of copy number may be detected include those selected from the group consisting of: next generation sequencing (NGS); exome sequencing; allele-specific amplification, and array comparative genomic hybridisation (aCGH).

More particularly, in a suitable embodiment, the presence of an NRAS activating mutation selected from an NRAS E63K mutation, an NRAS G12V mutation and an NRAS G12R mutation may be detected by means of NGS, allele-specific amplification or exome sequencing. In a suitable embodiment a gain *of NRAS* copy number may be determined by aCGH.

It will be understood that a patient may be tested for the presence of such a mutation by assaying for any suitable indicator in the patient which is representative of the mutation. For example, an indicator may be a change in: the sequence or amount of genomic DNA (e.g. a particular gene or expression control element); the sequence or amount of a particular mRNA (or corresponding cDNA); the sequence or amount of a protein; or protein activity, which is representative of the mutation. Where appropriate, any of the above may be detected by assaying a suitable fragment of genomic DNA, mRNA (or corresponding cDNA) or protein.

An expression control element for a gene refers to a sequence of DNA which is operably linked to a gene and which at least partially controls gene expression, e.g. a promoter or enhancer sequence.

A suitable indicator for determining one or more of the above activating NRAS mutations may be a change in: the sequence of the *NRAS* gene or expression control element for the gene; the amount (an increase) or sequence of *NRAS* mRNA expressed from the *NRAS* gene (or cDNA corresponding to it); the amount (an increase) or sequence of NRAS protein; or the activity of NRAS protein (an increase). A suitable indicator for determining an increase in *NRAS* copy number will be an increase in the number of copies of the *NRAS* gene present. Where appropriate, any of the above may be detected by assaying a suitable fragment of *NRAS* genomic DNA, mRNA (or corresponding cDNA) or NRAS protein. Without wishing to be bound by theory, it is believed that the NRAS mutations occur in the DNA of the tumour cells in the patient, typically (one of) the tumours which is being treated in the patient. Therefore the DNA, mRNA or protein which is assayed may be DNA, mRNA or protein present or expressed in such a tumour cell, typically *NRAS* genomic DNA, *NRAS* mRNA, or NRAS protein.

A mutation may be detected by any suitable means. Typically, the method comprises assaying a sample obtained from a patient for the presence of the mutation (typically by assaying a suitable indicator as described). Thus, the present methods may be carried out in vitro. In some instances, the method may comprise obtaining a suitable sample from a patient.

Any suitable sample, representative of the mutation, may be used. For example, a sample may comprise cells or tissue that are, or are suspected of being, cancerous, or comprise cancer/tumour nucleic acid. Thus the methods may comprise testing cancerous tissue or cells to determine the present of a mutation. Typically the cancer referred to is the cancer for which the patient is being treated. Suitable samples may be obtained by biopsy or other surgical procedure. Thus a sample may be a tumour biopsy. Cancer cells, or their DNA, may in some instances be present in the circulation and may be isolated from biological fluids such as blood, plasma, serum or pleural effusion. The methods may thus comprise testing of biological fluids that either contains cancer cells that have sloughed off from the tumour mass or circulating free DNA from such cancer cells to determine the present of a mutation.

Methods which involve assaying for a change in an amount of *NRAS* mRNA or NRAS protein (or fragment thereof), or for a change in the amount of NRAS activity, or for a change in copy number of *NRAS* gene, typically comprise:
- determining the level of *NRAS* mRNA or NRAS protein (or fragment), or the level of NRAS activity, or the *NRAS* gene copy number, in a suitable sample;
- comparing the determined level or copy number with a reference value; and
- determining the presence of a gain of *NRAS* copy number if the determined level or copy number is greater than the reference value.

A suitable reference value for the level of NRAS mRNA/protein/NRAS activity may be obtained with reference to the median level in a control population matched to the subject and sample type. The skilled person will be able to select an appropriate control to provide the requisite reference value.

A suitable reference value for *NRAS* gene copy number is 2 (i.e. two copies per cell).

### Determining the presence of a gain of NRAS copy number

Any increase in *NRAS* copy number may be indicative of increased resistance to EGFR-mediated cancer therapy, and such increased resistance may be indicated by determining an increase in *NRAS* gene copy number. *NRAS* gene copy number may be assayed directly, or *NRAS* gene copy number may be determined indirectly by assaying another indicator which is representative of *NRAS* gene copy number.

As referred to above, the presence of a gain of *NRAS* copy number may be detected by aCGH assays. Alternatively, a gain of *NRAS* copy number may be detected by sequencing of the cancer patient's DNA.

A gain of *NRAS* copy number may also be detected by determining an increase in the amount of *NRAS* mRNA, or NRAS protein, or an increase in the amount of NRAS activity. Amount of *NRAS* mRNA or NRAS protein may be assayed directly in a suitable sample. Alternatively, a suitable sample may be assayed for another indicator which is representative of amount of *NRAS* mRNA or NRAS protein.

Levels of mRNA or protein may be determined by any suitable means known to those skilled in the art.

Merely by way of example, the level of protein (e.g. NRAS protein) in the subject may be determined by means of an assay in which protein is detected, and the level present determined, by binding of the protein to a specific binding partner. The binding partner may be directly or indirectly labelled.

Antibodies or antibody fragments that bind to the protein represent particularly suitable examples of such binding partners, and these by be used in immunoassays. Suitable examples of immunoassays of this sort that may be used in the methods of the invention include those selected from the group consisting of: enzyme-linked immunosorbent assay (ELISA); radioimmunoassay (RIA); and multiplex immunoassays (such as Luminex™ assays produced by Luminex Corporation).

An antibody for specific binding to NRAS may be one obtained from a hybridoma. Procedures for the production of hybridomas are well known to those skilled in the art. Once the desired hybridoma has been selected and cloned, the resultant antibody is produced by in vitro culturing of the desired hybridoma in a suitable medium. As an alternative method, the desired hybridoma can be injected directly into mice to yield concentrated amounts of antibody.

Patents relating to monoclonal antibodies against human tumours produced by hybridoma technology include U.S. patent numbers 4,182,124 and 4,196,265. Representative of the art concerning monoclonal antibodies that have specificity for antigens on carcinoma cells are U.S. patent number 4,350,683.

Furthermore, the skilled person will appreciate that a wealth of commercially available antibodies specific for NRAS, and that these may be used without the need to produce novel antibodies.

Assays that may be used to determine the amount *of NRAS* mRNA in a sample include real time quantitative PCR. Merely by way of example, the inventors have found that this technique may be practised using commercially available Taqman primers, and probes from ABI Lifetechnologies.

Any suitable activity of NRAS may be assayed to determine an increase. In one aspect, NRAS GTPase activity is assayed in order to assess whether or not an increase is exhibited.

### Determining the presence of an NRAS E63K mutation

### In nucleic acid

The presence of an NRAS E63K mutation in a patient may be determined by detecting the corresponding mutation in the *NRAS* gene coding sequence. This may involve, for example, detecting a mutation in the genomic DNA sequence itself, or in the corresponding expressed mRNA (or corresponding cDNA), or in a suitable fragment of any of these polynucleotides, wherein the fragment spans the nucleic acid mutation encoding the E63K mutation. The mutation may also be detected in a nucleic acid that has been replicated based on the test sequence using amplification, such as polymerase chain reaction. For example, primers either side of the *NRAS* sequence encoding NRAS codon 63 are used to amplify up the target sequence and the presence or absence of the mutation can be discerned from this amplification reaction or its amplification product. The mutation site of a nucleic acid mutation encoding the E63K mutation is typically at codon 63 of the NRAS coding sequence. This may be understood as a change in any of the bases corresponding to bases 441-443 in *NRAS* encoding cDNA sequence which results in a lysine being coded for. In particular, a mutation may occur at a position corresponding to base 441 in the *NRAS* encoding cDNA.

The mutation may be a change from guanine (G) to adenine (A) (G to A, corresponding to uridine in mRNA) at codon 63 in the NRAS coding sequence. In particular the change may be a change from G to A at a position corresponding to base 441 in the *NRAS* encoding cDNA sequence, for example, a change from GAG to AAG at a position corresponding to nucleotides 441-443. The change may be a double change from G to A at a position corresponding to base 441 and 443 in the *NRAS* encoding cDNA sequence, for example, a change from GAG to AAA at a position corresponding to nucleotides 441-443. A nucleic acid mutation according to the invention may be detected by any suitable means. Typically a suitable method comprises amplifying the nucleic acid region of interest, i.e. the nucleic acid sequence containing the polymorphic locus. Any suitable amplification method may be used, for example, PCR amplification, allele-specific amplification, nucleic acid sequence-based amplification (NASBA), ligation activated amplification (LAT), the QB replicase system ligase chain reaction (LCR), repair chain reaction (RCR) nucleic acid amplification, or nucleic acid amplification by strand displacement activation (SDA). Preferably PCR amplification is used.

PCR primers for amplification of the nucleic acid sequence spanning the mutation site described herein above may be derived by those skilled in the art using for example, the *NRAS* gene sequence or the *NRAS* cDNA sequence. Such a pair of PCR primers hybridise under suitable PCR conditions to a polynucleotide encoding NRAS polypeptide or a fragment thereof, such that the primers bracket the mutation site.

A suitable PCR primer generally hybridises under suitable PCR conditions to a sense strand or antisense strand of a polynucleotide sequence 5' in each respective strand to the mutation site. A PCR primer may bind within about 200 nucleotides of the mutation. Any nucleic acid specimen, in purified or non-purified form, can be utilized as the starting nucleic acid(s), providing it contains, or is suspected of containing, the specific nucleic acid sequence containing the polymorphic locus. Thus, the process may amplify, for example, DNA or RNA, including messenger RNA, wherein DNA or RNA may be single stranded or double stranded. In the event that RNA is to be used as a template, enzymes, and/or conditions optimal for reverse transcribing the template to cDNA would be utilized. In addition, a DNA-RNA hybrid which contains one strand of each may be utilized. A method may for example, involve extracting nucleic acids from a suitable patient sample for assay. For example, mRNA may be extracted and corresponding cDNA (to all or a part of the mRNA) prepared by reverse transcription. Methods for mRNA extraction and cDNA preparation are known in the art and described herein.

Detecting a nucleic acid mutation described herein typically comprises contacting nucleic acids with a means for selectively detecting the mutation. Typically the means distinguishes between the mutant sequence at the mutation site, and the wild type sequence at that site. The means may be detectably labelled.

Thus, a method for detection of a nucleic acid mutation encoding E63K may comprise contacting a suitable sample with a means for selectively detecting a nucleotide sequence containing a mutant G at the position corresponding to base 441 *of NRAS* encoding cDNA and identifying that the base at the position is A. Typically the means distinguishes between the mutant T and the wild type C at this position.

The means may be, for example, a PCR primer, an oligonucleotide probe, or a sequence-specific cleaving means, such as any of those described herein.

In some instances, the amplification method itself can be used to distinguish between the mutant and wild type nucleic acid sequences, for example, amplification may be selective for nucleic acid containing the mutation, using for example allele-specific amplification or other suitable techniques that are known in the art.

For example, PCR amplification may be carried out using a PCR primer which binds selectively to a mutant nucleic acid, but not to the wild type nucleic acid under suitable PCR conditions. Typically such a primer hybridises under suitable PCR conditions to the sense strand sequence or the antisense strand sequence of an *NRAS*-encoding polynucleotide or a fragment thereof containing a mutation at the mutation site, but hybridises weakly or not at all to a second *NRAS* polynucleotide which is wild type at this position, under the PCR conditions.

For example, a selective PCR primer may hybridise under suitable PCR conditions to the sense strand sequence or the antisense strand sequence of an *NRAS-*encoding polynucleotide or a fragment thereof that includes a mutant A at the position corresponding to base 441 *of NRAS* encoding cDNA but hybridise weakly or not at all to a second *NRAS* encoding polynucleotide containing a wild type G at this position, under the PCR conditions.

PCR amplification can be carried out using this primer and a suitable second primer to provide a PCR amplicon containing a mutation (e.g. mutant T) at the mutation site. Detection of the amplicon therefore indicates the presence of the mutation.

According to one aspect of the disclosure there is provided an oligonucleotide capable of selectively hybridising to a nucleic acid sequence that encodes for a lysine at the position corresponding to position 63 of NRAS protein As disclosed herein, the oligonucleotide is capable of hybridising under suitable conditions to the sense strand sequence or the antisense strand sequence of an *NRAS*-encoding polynucleotide or a fragment thereof that includes a mutant A at the position corresponding to base 441 *of NRAS* encoding cDNA but hybridises weakly or not at all to a second *NRAS* encoding polynucleotide containing a wild type G at this position, The oligonucleotide may contain or be associated with a detectable label. As disclosed herein, the oligonucleotide is used or is suitable for use as a primer in a PCR reaction. As disclosed herein, the oligonucleotide is used or is suitable for use as a hybridisation probe.

According to another aspect of the disclosure there is provided an oligonucleotide capable of selectively hybridising to a nucleic acid sequence that encodes for a lysine at the position corresponding to position 63 of NRAS protein for use in a method of determining whether or not a patient has nucleic acid that encodes for the E63K substitution in NRAS protein.

According to another aspect of the disclosure there is provided an oligonucleotide capable of selectively hybridising to a nucleic acid sequence that encodes for a lysine at the position corresponding to position 63 of NRAS protein for use in a method of selecting a patient for combination treatment with an EGFR inhibitor and a MEK inhibitor as described herein.

The PCR reaction may incorporate a detectable label into the PCR amplicon, and the method may comprise detecting the label.

In other instances, amplified sequences can be further analysed, either in solution or after binding to a solid support, by any method usually applied to the detection of a specific DNA sequence such as PCR, oligomer restriction (Saiki, et. al., Bio/Technology, 3:1008-1012, (1985)), allele-specific oligonucleotide (ASO) probe analysis (Conner, et. al., Proc. Natl. Acad. Sci. U.S.A., 80:278, (1983)), oligonucleotide ligation assays (OLAs) (Landgren, et. al., Science, 241:1007, (1988)), and the like. Molecular techniques for DNA analysis have been reviewed (Landgren, et. al., Science, 242:229-237, (1988)).

In one example, target nucleic acid may be subjected to PCR amplification using at least one allele-specific (mutation-selective) PCR primer, as described above.

In another example, a mutation-selective oligonucleotide probe (sometimes referred to as an allele-specific probe) may be used to detect mutated nucleic acid. In such a method, amplified nucleic acid is contacted with an oligonucleotide probe which hybridises selectively to nucleic acid containing a mutation at the mutation site, but hybridises weakly or not at all nucleic acid which is wild type at the mutation site, under suitable hybridisation conditions. Detection of binding of probe to nucleic acid indicates presence of the mutation.

Either the probe means or the nucleic acids may be immobilised before the contacting step. In such a method, the component that binds to the immobilised partner is typically labelled, and determining the presence of the mutation includes detecting the label.

A suitable oligonucleotide probe for use in the method may for example hybridise to a polynucleotide encoding *NRAS* or a fragment thereof, wherein the sequence of the probe includes a base complementary to a mutant A at a position corresponding to base 441 of *NRAS* cDNA, and wherein the probe hybridises weakly or not at all to a polynucleotide containing a wild type C at this position under suitable hybridisation conditions.

Methods for designing allele-specific oligonucleotide probes are known in the art. Generally these are short, single stranded polynucleotides engineered to hybridize exactly to a target sequence under a given set of conditions. A primer or probe for use herein is generally a short, single stranded polynucleotide that hybridises specifically to a target sequence under a given set of conditions. A primer or probe typically has a length of at least 8 nucleotides, for example, at least 10, 12, 14, 16, 18, 20, 30 nucleotides, up to about 50 nucleotides. A probe or primer may be for example, no more than 15, 20, 25 or 20 nucleotides in length, such as 8-15, 10-20 or 10-30 nucleotides in length. An allele-specific oligonucleotide probe may be for example, 14-17 base pairs, such as for example, about 17 bases.

Methods of detecting a nucleic acid mutation herein may also include large-scale chip array sequence-based techniques. A review of the differences in the application and development of chip arrays is covered by Southern, E. M., Trends In Genetics, 12:110-115 (March 1996) and Cheng et al., Molecular Diagnosis, 1:183-200 (September 1996). A further methodology for large scale analysis on 'DNA chips' is described in detail in Hacia et al., Nature Genetics, 14:441-447 (1996).

An NRAS E63K mutation described herein may be detected by assaying for a change from glutamic acid (E) to lysine (K) at an amino acid position corresponding to amino acid E63 in the amino acid sequence of NRAS protein or a suitable fragment thereof encompassing amino acid 63. This may be done by any suitable method.

For example, the presence of NRAS protein (or a fragment thereof) having the E63K mutation may be determined by an assay in which the mutated NRAS protein or fragment thereof is detected by binding of the mutated NRAS protein or fragment to a specific binding partner. The specific binding partner binds to the mutant NRAS protein or fragment bearing the E63K mutation and not to NRAS protein or fragment without the mutation. The binding partner may be directly or indirectly labelled.

Antibodies or antibody fragments that bind to the mutant protein represent particularly suitable examples of such binding partners, and these by be used in immunoassays. Suitable examples of immunoassays of this sort that may be used in the methods of the invention include those selected from the group consisting of: enzyme-linked immunosorbent assay (ELISA); radioimmunoassay (RIA); and multiplex immunoassays (such as Luminex™ assays produced by Luminex Corporation). Western blotting may be used.

An antibody for detecting the mutant E63K NRAS specifically may be an antibody obtained from a hybridoma. Methods for obtaining antibodies from hybridomas are described elsewhere herein.

According to one aspect of the disclosure there is provided an antibody capable of selectively binding to an EGFR polypeptide that contains a lysine at the position corresponding to position 63 of NRAS protein, wherein the antibody preferentially binds to a polypeptide having lysine at the position corresponding to position 63 of NRAS protein over one having glutamic acid at said position.

According to another aspect of the disclosure there is provided an antibody capable of selectively binding to an EGFR polypeptide that contains a lysine at the position corresponding to position 63 of NRAS protein, for use in a method of determining whether or not a patient has an NRAS protein with a lysine corresponding to position 63 of NRAS protein, wherein the antibody preferentially binds to a polypeptide having lysine at the position corresponding to position 63 of NRAS protein over one having glutamic acid at said position. In a particular aspect of the disclosure such method is used to select the patient for combination treatment with an EGFR inhibitor and a MEK inhibitor as described herein.

An assay may comprise contacting the specific binding partner with a suitable sample such as any of those described herein. In some examples, proteins in general or NRAS protein specifically may first be extracted from a patient sample, and the specific binding partner contacted with the protein extract. Methods for protein extraction are known in the art. NRAS protein may be extracted from a patient sample by any suitable means. For example, antibodies binding specifically to NRAS protein may be used. Antibodies binding NRAS can be obtained using hybridomas using methods similar to those described above.

Extracted protein or proteins may be regarded as isolated. The term "isolated" as used herein generally means a biological component (such as a nucleic acid molecule or protein) that has been substantially separated or purified away from other biological components in the cell of the organism in which the component naturally occurs, i.e., other chromosomal and extrachromosomal DNA and RNA, and proteins. In one aspect, extracted proteins or nucleic acids have been separated to an extent that they can interact with reagents in a desired reaction, e.g. with a primer or probe, or with an antibody. Nucleic acids and proteins that have been "isolated" include nucleic acids and proteins purified by standard purification methods. The term also embraces nucleic acids and proteins prepared by recombinant expression in a host cell as well as chemically synthesized nucleic acids, proteins and peptides.

In another example, the presence of NRAS protein (or a fragment thereof) having the E63K mutation may be determined by an assay in which the amino acid sequence of NRAS protein or fragment at amino acid 63 is determined, for example by amino acid sequencing or using sequence specific cleaving means.

In such an assay, NRAS protein or a fragment thereof spanning amino acid 63 is typically isolated from a patient sample using the methods described herein above. Isolated protein or fragment may then be subjected to sequencing at least insofar as to determine the amino acid at position 63. Suitable sequencing methods are known in the art.

### Determining the presence of an NRAS G12V mutation

### In nucleic acid

The presence of an NRAS G12V mutation in a patient may be determined by detecting the corresponding mutation in the *NRAS* gene coding sequence.

The methods described above in relation to the E63K mutation apply mutatis mutandis to the G12V mutation, except for the location of the mutation site.

The mutation site of a nucleic acid mutation encoding the G12V mutation is typically at codon 12 of the NRAS coding sequence. This may be understood as a change in any of the bases corresponding to bases 288-290 in the *NRAS* cDNA sequence which results in a valine being coded for. In particular, a mutation may occur at a position corresponding to base 289 in the *NRAS* cDNA.

The mutation may be a change from G to T at codon 12 in the NRAS coding sequence. In particular the change may be a change from G to T at a position corresponding to base 289 in the *NRAS* cDNA sequence, for example, a change from GGT to GTT at a position corresponding to nucleotides 288-290.

According to one aspect of the disclosure there is provided an oligonucleotide capable of selectively hybridising to a nucleic acid sequence that encodes for a valine at the position corresponding to position 12 of NRAS protein. As disclosed herein, the oligonucleotide is capable of hybridising under suitable conditions to the sense strand sequence or the antisense strand sequence of an NRAS-encoding polynucleotide or a fragment thereof that includes a mutant T at the position corresponding to base 289 of *NRAS* encoding cDNA but hybridises weakly or not at all to a second NRAS encoding polynucleotide containing a wild type G at this position, The oligonucleotide may contain or be associated with a detectable label. As disclosed herein, the oligonucleotide is used or is suitable for use as a primer in a PCR reaction. As disclosed herein, the oligonucleotide is used or is suitable for use as a hybridisation probe.

According to another aspect of the disclosure there is provided an oligonucleotide capable of selectively hybridising to a nucleic acid sequence that encodes for a valine at the position corresponding to position 12 of NRAS protein for use in a method of determining whether or not a patient has nucleic acid that encodes for the G12V substitution in NRAS protein.

According to another aspect of the disclosure there is provided an oligonucleotide capable of selectively hybridising to a nucleic acid sequence that encodes for a valine at the position corresponding to position 12 of NRAS protein for use in a method of selecting a patient for combination treatment with an EGFR inhibitor and a MEK inhibitor as described herein.

### In protein

An NRAS G12V mutation described herein may be detected by assaying for a change from glycine (G) to valine (V) at an amino acid position corresponding to G12 in the amino acid sequence of NRAS protein or a suitable fragment thereof encompassing amino acid 12. This may be done by any suitable method.

The methods described above in relation to the E63K mutation apply mutatis mutandis to the G12V mutation, except for the location of the mutation site.

Determining the presence of a G12V mutation may also include determining an increase in the amount of NRAS protein.

According to one aspect of the disclosure there is provided an antibody capable of selectively binding to an EGFR polypeptide that contains a valine at the position corresponding to position 12 of NRAS protein, wherein the antibody preferentially binds to a polypeptide having valine at the position corresponding to position 12 of NRAS protein over one having glycine at said position.

According to another aspect of the disclosure there is provided an antibody capable of selectively binding to an EGFR polypeptide that contains a valine at the position corresponding to position 12 of NRAS protein, for use in a method of determining whether or not a patient has an NRAS protein with a valine at the position corresponding to position 12 of NRAS protein, wherein the antibody preferentially binds to a polypeptide having valine at the position corresponding to position 12 of NRAS protein over one having glycine at said position. In a particular aspect of the disclosure such method is used to select the patient for combination treatment with an EGFR inhibitor and a MEK inhibitor as described herein.

### Determining the presence of an NRAS G12R mutation

### In nucleic acid

The presence of an NRAS G12R mutation in a patient may be determined by detecting the corresponding mutation in the *NRAS* gene coding sequence.

The methods described above in relation to the E63K mutation apply mutatis mutandis to the G12V mutation, except for the location of the mutation site.

The mutation site of a nucleic acid mutation encoding the G12R mutation is typically at codon 12 of the NRAS coding sequence. This may be understood as a change in any of the bases corresponding to bases 288-290 in the *NRAS* cDNA sequence that results in an arginine being coded for. In particular, a mutation may occur at a position corresponding to base 288 in the *NRAS* cDNA.

The mutation may be a change from G to C at codon 12 in the NRAS coding sequence. In particular the change may be a change from G to C at a position corresponding to base 288 in the *NRAS* cDNA sequence, for example, a change from GGT to CGT at a position corresponding to nucleotides 288-290.

### In protein

An NRAS G12R mutation described herein may be detected by assaying for a change from glycine (G) to arginine (R) at an amino acid position corresponding to G12 in the amino acid sequence of NRAS protein or a suitable fragment thereof encompassing amino acid 12. This may be done by any suitable method.

The methods described above in relation to the E63K mutation apply mutatis mutandis to the G12R mutation, except for the location of the mutation site.

Determining the presence of a G12R mutation may also include determining an increase in the amount of NRAS protein.

### Treatments according to the invention

According to the disclosure, EGFR-associated cancer patients who harbour an NRAS activating mutation or gain of copy number of *NRAS* gene according to the invention can be effectively treated with a combination of EGFR inhibitor and MEK inhibitor. Typically the cancer patient with the NRAS mutation or gain of copy number of *NRAS* gene has already received, or is receiving, therapy with an EGFR inhibitor. The patient may show symptoms of acquired resistance to treatment with an EGFR inhibitor alone. In this situation, the EGFR inhibitor in the combination treatment may be the same inhibitor that was used in the patient's treatment (and which to which the patient has acquired resistance). However, treatment is also envisaged wherein the EGFR inhibitor in the combination treatment is a different inhibitor to the one used in treatment. In that case, the EGFR inhibitor in the combination treatment is generally of the same generation or of a higher generation than the EGFR inhibitor used in the patient's treatment and to which resistance has been acquired.

As disclosed herein, after determining the presence of an NRAS E63K, G12V, G12R, G12A, G12D, G12S or G12C mutation or a gain of copy number of *NRAS* gene in a patient, the disclosure can also provide a first-line therapy for EGFR-associated cancer in a patient who has not yet received treatment with an EGFR inhibitor. In this first-line therapy, a combination of EGFR inhibitor and MEK inhibitor is administered to the patient in order to delay or prevent development of acquired resistance to the EGFR inhibitor (e.g. by occurrence of an NRAS mutation as described herein).

In one embodiment the EGFR inhibitor is selected from AZD9291 and CO1686; or a pharmaceutically acceptable salt of either.

In a further embodiment the EGFR inhibitor is AZD9291 or a pharmaceutically acceptable salt thereof. In a further embodiment the EGFR inhibitor is AZD9291 or CO1686; or a pharmaceutically acceptable salt of either, and the MEK inhibitor is selumetinib or a pharmaceutically acceptable salt thereof.

In a further embodiment the EGFR inhibitor is AZD9291 or a pharmaceutically acceptable salt thereof, and the MEK inhibitor is selumetinib or a pharmaceutically acceptable salt thereof.

In another embodiment, after determining the presence of E63K or G12V NRAS activating mutation or a gain of copy number of *NRAS* gene in a patient, the invention can also provide a first-line therapy for EGFR-associated cancer in a patient who has not yet received treatment with an EGFR inhibitor. In this first-line therapy, a combination of EGFR inhibitor and MEK inhibitor is administered to the patient in order to delay or prevent development of acquired resistance to the EGFR inhibitor (e.g. by occurrence of an activating NRAS mutation as described herein).

In the present methods, a combination of EGFR inhibitor and MEK inhibitor is administered to a patient in a therapeutically effective amount.

It will be understood that reference herein to a combination treatment comprising an EGFR inhibitor and a MEK inhibitor encompasses a combination comprising one or more EGFR inhibitors and one or more MEK inhibitors, and that a combination of, or a combination comprising, an EGFR inhibitor and a MEK inhibitor encompasses a combination of one or more EGFR inhibitors and/or one or more MEK inhibitors. Specific combinations of EGFR inhibitors and MEK inhibitors are considered elsewhere in the specification.

### MEK inhibitors

A MEK inhibitor as used herein generally has activity which inhibits the Ras/Raf/MEK/ERK pathway. An inhibitor may act on a component at any stage of the pathway, acting to inhibit for example, the expression and/or activity of a component of the pathway.

A MEK inhibitor may be, for example, a small molecular weight compound.

A MEK inhibitor may inhibit gene expression, for example by interfering with mRNA stability or translation. A MEK inhibitor may be selected from an antisense oligonucleotide, small interfering RNA (siRNA), which is sometimes known as short interfering RNA or silencing RNA, or short hairpin RNA (shRNA), which is sometimes known as small hairpin RNA. Such a MEK inhibitor may inhibit expression of the mek1 and/or mek2 gene, for example by interfering with mRNA stability or translation. Typically an inhibitor reduces expression or activity by at least a detectable amount in a suitable assay. An inhibitor may, for example, reduce expression or activity by at least 10, 20, 30, 40, 50, 60, 70, 80 or at least 90%.

Suitable examples of MEK inhibitors are considered in the following pages of this disclosure. Without limitation, MEK inhibitors suitable for use in the context of the present invention include those selected from the group consisting of: selumetinib; trametinib; MEK-162; and cobimetinib; as well as pharmaceutically acceptable salts of these inhibitors.

### Selumetinib

The chemical structure of selumetinib is:

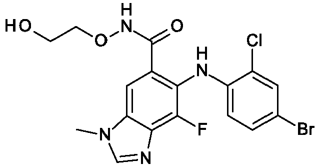

It may also be known by the chemical name: '(6-(4-bromo-2-chloro-phenylamino)-7-fluoro-3-methyl-3*H*-benzoimidazole-5-carboxylic acid (2-hydroxy-ethoxy)-amide)'. Equally, it may also be known by the chemical name: '5-[(4-bromo-2-chlorophenyl)amino]-4-fluoro-*N*-(2-hydroxyethoxy)-1-methyl-1*H*-benzimidazole-6-carboxamide'.

Selumetinib may also be provided in the form of a pharmaceutically acceptable salt, such as a hydrogensulphate salt; i.e. selumetinib hydrogensulphate. (i.e. 1:1 drug:H₂SO₄). In a suitable embodiment, a MEK inhibitor may comprise selumetinib, or its pharmaceutically acceptable salts such as the hydrogensulphate salt.

### Trametinib

The chemical structure of trametinib is:

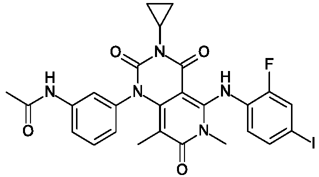

It may also be known by the chemical name: N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl)acetamide.

Trametinib may also be provided in the form of a pharmaceutically acceptable salt, and, unless the context requires otherwise, references to trametinib in the present disclosure should also be taken to encompass such pharmaceutically acceptable salts.

### MEK-162

The chemical structure of MEK-162 is:

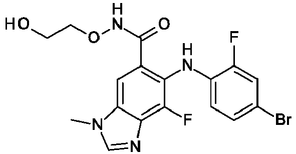

It may also be known by the chemical name: 5-[(4-bromo-2-fluorophenyl)amino]-4-fluoro-N-(2-hydroxyethoxy)-1-methyl-1H-benzimidazole-6-carboxamide. MEK-162 may also be provided in the form of a pharmaceutically acceptable salt, and, unless the context requires otherwise, references to MEK-162 in the present disclosure should also be taken to encompass such pharmaceutically acceptable salts.

### Cobimetinib

The chemical structure of cobimetinib is:

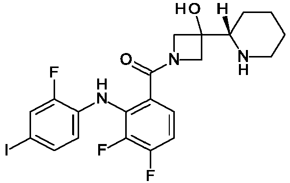

Cobimetinib may also be known by the chemical name: {3,4-difluoro-2-[(2-fluoro-4-iodophenyl)amino]phenyl} {3-hydroxy-3-[(2S)-piperidin-2-yl]azetidin-1-yl}methanone. Cobimetinib may also be provided in the form of a pharmaceutically acceptable salt, such as the fumarate salt of cobimetinib (2:1 drug: fumaric acid). Unless the context requires otherwise, references to cobimetinib in the present disclosure should also be taken to encompass pharmaceutically acceptable salts such as the fumarate salt.

In a suitable embodiment, a MEK inhibitor may inhibit the activity of both MEK1 and MEK2 (a so-called "MEK1/2 inhibitor"). In one aspect, a MEK inhibitor may inhibit the expression and/or activity of a MEK1 or MEK2 protein.

### MEK1

MEK1 as used herein refers to mitogen activated protein kinase kinase 1.

MEK1 may be of any species corresponding to the species to be treated. In one aspect,

MEK1 is human MEK1.

A (wild type) human MEK1 gene is described in Gene ID: 5604. The wild type human mek1 gene typically has an mRNA expression product with a sequence corresponding to the *MEK1* cDNA sequence in GenBank Accession No. NM_002755.3, and protein expression product having the MEK1 protein amino acid sequence in UniProtKB/Swiss-Prot Q02750. In the MEK1 cDNA sequence set out in GenBank Accession No. NM_002755.3, the protein coding sequence is shown at nucleotides 476-1657. The consenus coding sequence is also presented in the CCDS database under Accession No.

### CCDS10216.1

Reference herein to mek1 gene (*MEK1* gene), mRNA, cDNA or MEK1 protein may refer to this human gene, mRNA or corresponding cDNA sequence, or protein.

MEK1 may in some instances also refer to a variant of any of the above, such as a naturally occurring variant.

The above applies mutatis mutandis for MEK1 of a non-human species. For example, reference herein to mek1 gene, mRNA, cDNA or MEK1 protein may refer to a species homolog of the above human gene, mRNA or protein.

### MEK2

MEK2 as used herein refers to mitogen activated protein kinase kinase 2.

MEK2 may be of any species corresponding to the species to be treated. In one aspect,

MEK2 is human MEK2.

A (wild type) human MEK2 gene is described in Gene ID: 5605. The wild type human mek2 gene typically has an mRNA expression product with sequence corresponding to the MEK2 cDNA sequence in GenBank Accession No. NM_030662.3, and protein expression product having the MEK2 protein amino acid sequence in UniProtKB/Swiss-Prot P36507. In the *MEK2* cDNA sequence in GenBank Accession No. NM_030662.3, the protein coding sequence is shown at nucleotides 255-1457. The consensus coding sequence is also presented in the CCDS database under Accession No. CCDS12120.1.

Reference herein to mek2 gene (*MEK2* gene), mRNA, cDNA or MEK2 protein may refer to this human gene, mRNA or corresponding cDNA sequence, or protein.

MEK2 may in some instances also refer to a variant of any of the above, such as a naturally occurring variant.

The above applies mutatis mutandis for MEK2 of a non-human species. For example, reference herein to mek2 gene, mRNA, cDNA or MEK2 protein may refer to a species homolog of the above human gene, mRNA or protein.

### Inhibitors of MEK1 and MEK2

A MEK inhibitor may inhibit any suitable activity of MEK1 or MEK2, for example, protein kinase activity. Inhibition of protein kinase activity may be determined in a suitable assay, for example as described in Yeh et al. Clin Cancer Res March 1, 2007 13; 1576.

A MEK inhibitor may be, for example, selected from any one of an ATP-competitive MEK inhibitor, a non-ATP competitive MEK inhibitor, or an ATP-uncompetitive MEK inhibitor.

Suitable MEK inhibitors, which inhibit activity of MEK1 and/or MEK2 include selumetinib.

Specific combinations of EGFR inhibitors and MEK inhibitors for use in patients whose tumours have been found to contain an NRAS E63K, G12V, G12R, G12A, G12D, G12S or G12C mutation or a gain of copy number of *NRAS* gene.

In a suitable embodiment, a combination treatment may comprise the EGFR TKI gefitinib (or a pharmaceutically acceptable salt thereof) and the MEK inhibitor selumetinib (or a pharmaceutically acceptable salt thereof). In a suitable embodiment, a combination treatment may comprise the EGFR TKI gefitinib (or a pharmaceutically acceptable salt thereof) and the MEK inhibitor trametinib (or a pharmaceutically acceptable salt thereof). In a suitable embodiment, a combination treatment may comprise the EGFR TKI gefitinib (or a pharmaceutically acceptable salt thereof) and the MEK inhibitor MEK-162 (or a pharmaceutically acceptable salt thereof). In a suitable embodiment, a combination treatment may comprise the EGFR TKI gefitinib (or a pharmaceutically acceptable salt thereof) and the MEK inhibitor cobimetinib (or a pharmaceutically acceptable salt thereof). In a suitable embodiment, a combination treatment may comprise the EGFR TKI erlotinib (or a pharmaceutically acceptable salt thereof) and the MEK inhibitor selumetinib (or a pharmaceutically acceptable salt thereof). In a suitable embodiment, a combination treatment may comprise the EGFR TKI erlotinib (or a pharmaceutically acceptable salt thereof) and the MEK inhibitor trametinib (or a pharmaceutically acceptable salt thereof). In a suitable embodiment, a combination treatment may comprise the EGFR TKI erlotinib (or a pharmaceutically acceptable salt thereof) and the MEK inhibitor MEK-162 (or a pharmaceutically acceptable salt thereof). In a suitable embodiment, a combination treatment may comprise the EGFR TKI erlotinib (or a pharmaceutically acceptable salt thereof) and the MEK inhibitor cobimetinib (or a pharmaceutically acceptable salt thereof). In a suitable embodiment, a combination treatment may comprise the EGFR TKI afatinib (or a pharmaceutically acceptable salt thereof) and the MEK inhibitor selumetinib (or a pharmaceutically acceptable salt thereof). In a suitable embodiment, a combination treatment may comprise the EGFR TKI afatinib (or a pharmaceutically acceptable salt thereof) and the MEK inhibitor trametinib (or a pharmaceutically acceptable salt thereof). In a suitable embodiment, a combination treatment may comprise the EGFR TKI afatinib

(or a pharmaceutically acceptable salt thereof) and the MEK inhibitor MEK-162 (or a pharmaceutically acceptable salt thereof). In a suitable embodiment, a combination treatment may comprise the EGFR TKI afatinib (or a pharmaceutically acceptable salt thereof) and the MEK inhibitor cobimetinib (or a pharmaceutically acceptable salt thereof). In a suitable embodiment, a combination treatment may comprise the EGFR TKI AZD9291 (or a pharmaceutically acceptable salt thereof) and the MEK inhibitor selumetinib (or a pharmaceutically acceptable salt thereof). In a suitable embodiment, a combination treatment may comprise the EGFR TKI AZD9291 (or a pharmaceutically acceptable salt thereof) and the MEK inhibitor trametinib (or a pharmaceutically acceptable salt thereof). In a suitable embodiment, a combination treatment may comprise the EGFR TKI AZD9291 (or a pharmaceutically acceptable salt thereof) and the MEK inhibitor MEK-162 (or a pharmaceutically acceptable salt thereof). In a suitable embodiment, a combination treatment may comprise the EGFR TKI AZD9291 (or a pharmaceutically acceptable salt thereof) and the MEK inhibitor cobimetinib (or a pharmaceutically acceptable salt thereof).

In a suitable embodiment, a combination treatment may comprise the EGFR TKI CO-1686 (or a pharmaceutically acceptable salt thereof) and the MEK inhibitor selumetinib (or a pharmaceutically acceptable salt thereof). In a suitable embodiment, a combination treatment may comprise the EGFR TKI CO-1686 (or a pharmaceutically acceptable salt thereof) and the MEK inhibitor trametinib (or a pharmaceutically acceptable salt thereof). In a suitable embodiment, a combination treatment may comprise the EGFR TKI CO-1686 (or a pharmaceutically acceptable salt thereof) and the MEK inhibitor MEK-162 (or a pharmaceutically acceptable salt thereof). In a suitable embodiment, a combination treatment may comprise the EGFR TKI CO-1686 (or a pharmaceutically acceptable salt thereof) and the MEK inhibitor cobimetinib (or a pharmaceutically acceptable salt thereof).

### Inhibitors of NRAS

A MEK inhibitor may inhibit activity associated with NRAS signalling, since MEK1 and/or MEK2 are downstream components of the NRAS signalling pathway.

For example, a MEK inhibitor may inhibit expression of the *NRAS* gene, for example by interfering with mRNA stability or translation. Examples of siRNAs targeting expression of the *NRAS* gene are provided in the Examples.

### Other agents

A combination of EGFR inhibitor and MEK inhibitor according to the invention may be administered to a cancer patient as a sole therapy. Alternatively, EGFR inhibitor and MEK inhibitor may be administered in combination with additional surgery or radiotherapy or an additional chemotherapeutic agent or a therapeutic antibody.

### Combined administration

According to the invention, components of a combination treatment may be administered in combination or conjunction with each other. A combination treatment may be in the form of a combined preparation, for example a combined preparation of EGFR inhibitor and MEK inhibitor. A combination may comprise separate formulations of one or more of the components, for example, separate formulations of EGFR inhibitor and MEK inhibitor. Components in a combination (e.g. separate formulations) may be administered sequentially, separately and/or simultaneously as described herein in relation to the combination treatment methods. Thus, for example, an EGFR inhibitor may be administered separately, sequentially or simultaneously with a MEK inhibitor.

In one embodiment the components are administered simultaneously (optionally repeatedly). In one embodiment the components are administered sequentially (optionally repeatedly). In one embodiment the components are administered separately (optionally repeatedly).

The skilled person will understand that where separate formulations of the agents in a combination are administered sequentially or serially, that this could be in administration of the agents in any order. Thus, where an EGFR inhibitor and a MEK inhibitor are administered sequentially or serially, this could be administration of an EGFR inhibitor followed by a MEK inhibitor, or administration of a MEK inhibitor followed by an EGFR inhibitor.

In one embodiment, separate formulations of agents may be administered in alternative dosing patterns.

Where the administration of separate formulations is sequential or separate, the delay in administering the second (or subsequent) formulation should not be such as to lose the beneficial therapeutic effect of the combination treatment.

### Combination products

The components of a combination treatment described herein may be provided as a combination product.

A combination product typically comprises:
(a) an EGFR inhibitor; and
(b) a MEK inhibitor; as described herein.

The combination product is useful for treating EGFR-associated cancer, by a method described herein.

A combination product may comprise
(a) an EGFR inhibitor; and
(b) a MEK inhibitor;
in association with a pharmaceutically acceptable adjuvant, diluent or carrier.

A combination product, as defined herein, may be in the form of a combined preparation of the components. Thus a combination produce may be in the form of a combined preparation of an EGFR inhibitor, and an MEK inhibitor.

A combination product, as defined herein, may comprise a kit of parts comprising separate formulations of each of the agents in the product. Thus, the kit of parts may comprise separate formulations of an EGFR inhibitor, and an MEK inhibitor as described herein.

A combination product may comprise a kit of parts comprising
(a) an EGFR inhibitor or a pharmaceutically acceptable salt thereof, in association with a pharmaceutically acceptable adjuvant, diluent or carrier;
   and:
(b) an MEK inhibitor or a pharmaceutically acceptable salt thereof, in association with a pharmaceutically acceptable adjuvant, diluent or carrier;
wherein the components are provided in a form which is suitable for sequential, separate and/or simultaneous administration to a cancer patient whose tumour cells comprise a E63K, G12V, G12R, G12A, G12D, G12S or G12C NRAS mutation.

In further embodiments, the tumour cells comprise an NRAS mutation selected from E63K, G12V and G12R.

As disclosed herein,, the tumour cells comprise an NRAS mutation selected from G12R, G12A, G12D, G12S and G12C.

In a further embodiment, the EGFR inhibitor is selected from AZD9291 and CO1686; or a pharmaceutically acceptable salt thereof.

In a further embodiment the EGFR inhibitor is AZD9291 or a pharmaceutically acceptable salt thereof. In a further embodiment the EGFR inhibitor is AZD9291 or CO1686; or a pharmaceutically acceptable salt thereof, and the MEK inhibitor is selumetinib or a pharmaceutically acceptable salt thereof.

In a further embodiment the EGFR inhibitor is AZD9291 or a pharmaceutically acceptable salt thereof, and the MEK inhibitor is selumetinib or a pharmaceutically acceptable salt thereof.

In further embodiments the tumour cells comprise an E63K, G12V or G12R NRAS mutation.

In one embodiment the tumour cells comprise a G12R NRAS mutation.

A combination product may comprise a kit of parts comprising
(a) an EGFR inhibitor, in association with a pharmaceutically acceptable adjuvant, diluent or carrier;
   and:
(b) an MEK inhibitor, in association with a pharmaceutically acceptable adjuvant, diluent or carrier;
wherein the components are provided in a form which is suitable for sequential, separate and/or simultaneous administration to a cancer patient whose tumour cells comprise a gain of copy number of *NRAS* gene or NRAS protein with E63K and/or G12V NRAS activating mutation.

The kit of parts may comprise:
a first container comprising the first of the components in the combination product, in association with a pharmaceutically acceptable adjuvant, diluent or carrier; and
a second or subsequent container comprising the second or any subsequent of the components in the combination product respectively, each component in association with a pharmaceutically acceptable adjuvant, diluent or carrier, and
a container means for containing said first and second and any subsequent containers.

A combination product, as defined herein, may comprise a pharmaceutical composition which comprises:
(a) an EGFR inhibitor; and
(b) an MEK inhibitor.

A pharmaceutical composition generally comprises a pharmaceutically acceptable adjuvant, diluent or carrier.

A combination product may comprise a pharmaceutical composition which comprises:
(a) an EGFR inhibitor, in association with a pharmaceutically acceptable adjuvant, diluent or carrier; and
(b) an MEK inhibitor, in association with a pharmaceutically acceptable adjuvant, diluent or carrier.

A combination product of the invention may comprise more than one EGFR inhibitor, and/or more than one MEK inhibitor. A combination product may comprise one or more other active agent, selected from those described herein.

A kit of parts as described herein may further comprise instructions to administer the components sequentially, separately and/or simultaneously for treatment of cancer.

In one embodiment a combination product as described herein further comprises instructions indicating that the product, as defined herein, can be used for treating EGFR-associated cancer by a method described herein. A product may comprise instructions indicating that the product is for use in treating a cancer as described herein in a cancer patient as described herein. For example, a product may include instructions indicating that the product can be used for treating cancer in a patient who has received or is receiving EGFR inhibitor therapy, and who tests positive for an NRAS mutation or a gain of copy number of *NRAS* gene according to the invention. In another example, a product may include instructions indicating that the product can be used for treating EGFR-associated cancer in a patient whose tumour cells comprise a gain of copy number of *NRAS* gene or NRAS protein with E63K and/or G12V NRAS activating mutation, as a first line therapy. In another example, a product may include instructions indicating that the product can be used for treating EGFR-associated cancer in a patient whose tumour cells comprise a G12R NRAS mutation. In one embodiment a product includes instructions indicating that the product can be used for treating EGFR-associated cancer in a patient whose tumour cells comprise a E63K, G12V, G12R, G12A, G12D, G12S or G12C NRAS mutation.

### Medical methods and medical uses

In one aspect, the invention relates to a combination product, as defined herein, comprising
(a) an EGFR inhibitor; and
(b) a MEK inhibitor;
for use sequentially, separately and/or simultaneously in any of the methods described herein.

Further disclosed is a combination product comprising an EGFR inhibitor and a MEK inhibitor as described herein, for use in treating EGFR-associated cancer in a patient, wherein the patient has an NRAS mutation selected from E63K, G12V, G12R, G12A, G12D, G12S and G12C; or a gain of copy number of *NRAS* gene; wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration.

Further provided is a combination product comprising an EGFR inhibitor and a MEK inhibitor as described herein, for use in treating EGFR-associated cancer in a patient, wherein the patient has an NRAS mutation selected from E63K, G12V and G12R; or a gain of copy number of *NRAS* gene; wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration.

As disclosed herein, the NRAS mutation is selected from G12R, G12A, G12D, G12S and G12C.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

Further provided is a combination product comprising an EGFR inhibitor and a MEK inhibitor as described herein, for use in treating EGFR-associated cancer in a patient, wherein the patient has an NRAS activating mutation selected from the group consisting of: an NRAS E63K mutation and an NRAS G12V mutation; or a gain of copy number of *NRAS* gene; wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration.

In a further aspect the disclosure provides a pharmaceutical composition comprising EGFR inhibitor and MEK inhibitor (each optionally in the form of a pharmaceutically acceptable salt) in association with a pharmaceutically acceptable adjuvant carrier or diluent as described herein, for use in treating EGFR-associated cancer in a patient, wherein the patient has an NRAS mutation selected from E63K, G12V, G12R, G12A, G12D, G12S and G12C; or a gain of *NRAS* copy number.

In a further aspect the invention provides a pharmaceutical composition comprising EGFR inhibitor and MEK inhibitor (each optionally in the form of a pharmaceutically acceptable salt) in association with a pharmaceutically acceptable adjuvant carrier or diluent as described herein, for use in treating EGFR-associated cancer in a patient, wherein the patient has an NRAS mutation selected from E63K, G12V and G12R, corresponding to the position in NRAS protein; or a gain of *NRAS* copy number.

As disclosed herein, the NRAS mutation is selected from G12R, G12A, G12D, G12S and G12C.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

In a further aspect the invention provides a pharmaceutical composition comprising EGFR inhibitor and MEK inhibitor in association with a pharmaceutically acceptable adjuvant carrier or diluent as described herein, for use in treating EGFR-associated cancer in a patient, wherein the patient has an NRAS activating mutation selected from the group consisting of: E63K and G12V corresponding to the position in NRAS protein; or a gain of *NRAS* copy number.

The disclosure also relates to use of a combination product or a pharmaceutical composition as described herein for the manufacture of a medicament for treating EGFR-associated cancer in a patient, wherein the patient has an NRAS mutation selected from E63K, G12V, G12R, G12A, G12D, G12S and G12C, or a gain of *NRAS* copy number. Accordingly, the disclosure relates to the use of a combination product or a pharmaceutical composition as described herein for the manufacture of a medicament for the treatment of NRAS mutated cancer, where the NRAS mutation is selected from E63K, G12V, G12R, G12A, G12D, G12S and G12C, or a gain of *NRAS* copy number.

The disclosure also relates to use of a combination product or a pharmaceutical composition as described herein for the manufacture of a medicament for treating EGFR-associated cancer in a patient, wherein the patient has an NRAS mutation selected from E63K, G12V, G12R, G12A, G12D, G12S and G12C.

Accordingly, the disclosure relates to the use of a combination product or a pharmaceutical composition as described herein for the manufacture of a medicament for the treatment of NRAS mutated cancer, where the NRAS mutation is selected from E63K, G12V, G12R, G12A, G12D, G12S and G12C. As disclosed herein, the NRAS mutation is selected from G12R, G12R, G12A, G12D, G12S and G12C. In a further embodiment the NRAS mutated cancer is G12R NRAS mutated cancer. In a further embodiment the NRAS mutated cancer has a gain of *NRAS* copy number.

The invention also relates to use of a combination product or a pharmaceutical composition as described herein for the manufacture of a medicament for treating EGFR-associated cancer in a patient, wherein the patient has an NRAS mutation selected from E63K, G12V and G12R.

Accordingly, the invention relates to the use of a combination product or pharmaceutical composition as described herein for the manufacture of a medicament for the treatment of NRAS mutated cancer where the NRAS mutation is selected from E63K, G12V and G12R. As disclosed herein, the NRAS mutation is selected from G12R, G12A, G12D, G12S and G12C. As disclosed herein, the NRAS mutated cancer has a gain of *NRAS* copy number. The invention also relates to use of a combination product or a pharmaceutical composition as described herein for the manufacture of a medicament for treating EGFR-associated cancer in a patient, wherein the patient has an NRAS activating mutation selected from the group consisting of: an NRAS E63K mutation and an NRAS G12V mutation; or a gain of *NRAS* copy number.

The disclosure also relates to an EGFR inhibitor and a MEK inhibitor for combined use in treating EGFR-associated cancer in a patient, wherein the patient has an NRAS mutation selected from E63K, G12V, G12R, G12A, G12D, G12S and G12C; or a gain of *NRAS* copy number.

Accordingly, the disclosure relates to an EGFR inhibitor and a MEK inhibitor for combined use in treating NRAS mutated cancer wherein the NRAS mutation is selected from E63K, G12V, G12R, G12A, G12D, G12S and G12C; or a gain of *NRAS* copy number.

The disclosure also relates to an EGFR inhibitor and a MEK inhibitor for combined use in treating EGFR-associated cancer in a patient, wherein the patient has an NRAS mutation selected from E63K, G12V, G12R, G12A, G12D, G12S and G12C.Accordingly, the disclosure relates to an EGFR inhibitor and a MEK inhibitor for combined use in treating NRAS mutated cancer, wherein the NRAS mutation is selected from E63K, G12V, G12R, G12A, G12D, G12S and G12C.

The invention also relates to an EGFR inhibitor and a MEK inhibitor for combined use in treating EGFR-associated cancer in a patient, wherein the patient has an NRAS mutation selected from E63K, G12V and G12R; or a gain of *NRAS* copy number.

Accordingly the invention relates to an EGFR inhibitor and a MEK inhibitor for combined use in treating NRAS mutated cancer wherein the NRAS mutation is selected from E63K, G12V and G12R; or a gain of *NRAS* copy number.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also relates to an EGFR inhibitor and a MEK inhibitor for combined use in treating EGFR-associated cancer in a patient, wherein the patient has an NRAS activating mutation selected from the group consisting of: an NRAS E63K mutation and an NRAS G12V mutation; or a gain of *NRAS* copy number.

Also disclosed is an EGFR inhibitor for use in treating EGFR-associated cancer in a patient, wherein the patient has an NRAS mutation selected from E63K, G12V, G12R, G12A, G12D, G12S and G12C; or a gain of *NRAS* copy number, and wherein the EGFR inhibitor is administered in combination with a MEK inhibitor.

Accordingly, there is disclosed an EGFR inhibitor for use in the treatment of NRAS mutated cancer wherein the NRAS mutation is selected from E63K, G12V, G12R, G12A, G12D, G12S and G12C; or a gain of *NRAS* copy number, and wherein the EGFR inhibitor is administered in combination with a MEK inhibitor.

Also disclosed is an EGFR inhibitor for use in treating EGFR-associated cancer in a patient, wherein the patient has an NRAS mutation selected from E63K, G12V, G12R, G12A, G12D, G12S and G12C; and wherein the EGFR inhibitor is administered in combination with a MEK inhibitor.

Accordingly, there is disclosed an EGFR inhibitor for use in the treatment of NRAS mutated cancer wherein the NRAS mutation is selected from E63K, G12V, G12R, G12A, G12D, G12S and G12C; and wherein the EGFR inhibitor is administered in combination with a MEK inhibitor.

Also provided is an EGFR inhibitor for use in treating EGFR-associated cancer in a patient, wherein the patient has an NRAS mutation selected from E63K, G12V and G12R; or a gain of *NRAS* copy number, and wherein the EGFR inhibitor is administered in combination with a MEK inhibitor.

Accordingly, there is provided an EGFR inhibitor for use in the treatment of NRAS mutated cancer wherein the NRAS mutation is selected from E63K, G12V and G12R, or a gain of *NRAS* copy number; and wherein the EGFR inhibitor is administered in combination with a MEK inhibitor.

Also provided is an EGFR inhibitor for use in treating EGFR-associated cancer in a patient, wherein the patient has an NRAS mutation selected from E63K, G12V and G12R; and wherein the EGFR inhibitor is administered in combination with a MEK inhibitor. Accordingly, there is provided an EGFR inhibitor for use in the treatment of NRAS mutated cancer wherein the NRAS mutation is selected from E63K, G12V and G12R; and wherein the EGFR inhibitor is administered in combination with a MEK inhibitor.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof. In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof. In further embodiments the EGFR inhibitor is AZD9291 or a pharmaceutically acceptable salt thereof, and the MEK inhibitor is selumetinib or a pharmaceutically acceptable salt thereof. Also provided is an EGFR inhibitor for use in treating EGFR-associated cancer in a patient, wherein the patient has an NRAS activating mutation selected from the group consisting of: an NRAS E63K mutation and an NRAS G12V mutation; or a gain of *NRAS* copy number, and wherein the EGFR inhibitor is administered in combination with a MEK inhibitor.

Also disclosed is a MEK inhibitor for use in treating EGFR-associated cancer in a patient, wherein the patient has an NRAS mutation selected from E63K, G12V, G12R, G12A, G12D, G12S and G12C; or a gain of *NRAS* copy number, and wherein the MEK inhibitor is administered in combination with an EGFR inhibitor.

Accordingly, there is disclosed a MEK inhibitor for use in the treatment of NRAS mutated cancer wherein the NRAS mutation is selected from E63K, G12V, G12R, G12A, G12D, G12S and G12C; or a gain of *NRAS* copy number, and wherein the MEK inhibitor is administered in combination with an EGFR inhibitor.

Also disclosed is a MEK inhibitor for use in treating EGFR-associated cancer in a patient, wherein the patient has an NRAS mutation selected from E63K, G12V, G12R, G12A, G12D, G12S and G12C, and wherein the MEK inhibitor is administered in combination with an EGFR inhibitor.

Accordingly, there is disclosed a MEK inhibitor for use in the treatment of NRAS mutated cancer wherein the NRAS mutation is selected from E63K, G12V, G12R, G12A, G12D, G12S and G12C; and wherein the MEK inhibitor is administered in combination with an EGFR inhibitor.

Also provided is a MEK inhibitor for use in treating EGFR-associated cancer in a patient, wherein the patient has an NRAS mutation selected from E63K, G12V and G12R; or a gain of *NRAS* copy number, and wherein the MEK inhibitor is administered in combination with an EGFR inhibitor.

Accordingly, there is provided a MEK inhibitor for use in the treatment of NRAS mutated cancer wherein the NRAS mutation is selected from E63K, G12V and G12R; or a gain of *NRAS* copy number, and wherein the MEK inhibitor is administered in combination with an EGFR inhibitor.

Also provided is a MEK inhibitor for use in treating EGFR-associated cancer in a patient, wherein the patient has an NRAS mutation selected from E63K, G12V and G12R, and wherein the MEK inhibitor is administered in combination with an EGFR inhibitor. Accordingly there is provided a MEK inhibitor for use in the treatment of NRAS mutated cancer wherein the NRAS mutation is selected from E63K, G12V and G12R, and wherein the MEK inhibitor is administered in combination with an EGFR inhibitor.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

Also provided is a MEK inhibitor for use in treating EGFR-associated cancer in a patient, wherein the patient has an NRAS activating mutation selected from the group consisting of: an NRAS E63K mutation and an NRAS G12V mutation; or a gain of *NRAS* copy number, and wherein the MEK inhibitor is administered in combination with an EGFR inhibitor.

The disclosure also relates to use of a MEK inhibitor for the manufacture of a medicament for treating EGFR-associated cancer in a patient, wherein the patient has an NRAS mutation selected from E63K, G12V, G12R, G12A, G12D, G12S and G12C; or a gain of *NRAS* copy number, and wherein the treatment comprises administering the MEK inhibitor in combination with an EGFR inhibitor.

Accordingly, the disclosure relates to the use of a MEK inhibitor for the manufacture of a medicament for the treatment of NRAS mutated cancer wherein the NRAS mutation is selected from E63K, G12V, G12R, G12A, G12D, G12S and G12C; or a gain of *NRAS* copy number, and wherein the treatment comprises administering the MEK inhibitor in combination with an EGFR inhibitor.

The disclosure also relates to use of a MEK inhibitor for the manufacture of a medicament for treating EGFR-associated cancer in a patient, wherein the patient has an NRAS mutation selected from E63K, G12V, G12R, G12A, G12D, G12S and G12C, and wherein the treatment comprises administering the MEK inhibitor in combination with an EGFR inhibitor.

Accordingly, the disclosure relates to the use of a MEK inhibitor for the manufacture of a medicament for the treatment of NRAS mutated cancer wherein the NRAS mutation is selected from E63K, G12V, G12R, G12A, G12D, G12S and G12C; and wherein the treatment comprises administering the MEK inhibitor in combination with an EGFR inhibitor.

The invention also relates to use of a MEK inhibitor for the manufacture of a medicament for treating EGFR-associated cancer in a patient, wherein the patient has an NRAS mutation selected from E63K, G12V and G12R; or a gain of *NRAS* copy number, and wherein the treatment comprises administering the MEK inhibitor in combination with an EGFR inhibitor.

Accordingly, the invention relates to the use of a MEK inhibitor for the manufacture of a medicament for the treatment of NRAS mutated cancer wherein the NRAS mutation is selected from E63K, G12V and G12R; or a gain of *NRAS* copy number, and wherein the treatment comprises administering the MEK inhibitor in combination with an EGFR inhibitor.

The invention also relates to use of a MEK inhibitor for the manufacture of a medicament for treating EGFR-associated cancer in a patient, wherein the patient has an NRAS mutation selected from E63K, G12V and G12R, and wherein the treatment comprises administering the MEK inhibitor in combination with an EGFR inhibitor.

Accordingly, the invention relates to the use of a MEK inhibitor for the manufacture of a medicament for the treatment of NRAS mutated cancer wherein the NRAS mutation is selected from E63K, G12V, G12R, G12A, G12D, G12S and G12C; and wherein the treatment comprises administering the MEK inhibitor in combination with an EGFR inhibitor.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also relates to use of a MEK inhibitor for the manufacture of a medicament for treating EGFR-associated cancer in a patient, wherein the patient has an NRAS activating mutation selected from the group consisting of: an NRAS E63K mutation and an NRAS G12V mutation; or a gain of *NRAS* copy number, and wherein the treatment comprises administering the MEK inhibitor in combination with an EGFR inhibitor.

The medicament (e.g. a combination product as described herein) may comprise the MEK inhibitor and the EGFR inhibitor.

The disclosure also relates to use of an EGFR inhibitor for the manufacture of a medicament for treating EGFR-associated cancer in a patient, wherein the patient has an NRAS mutation selected from E63K, G12V, G12R, G12A, G12D, G12S and G12C; or a gain of *NRAS* copy number, and wherein the treatment comprises administering the EGFR inhibitor in combination with a MEK inhibitor.

Accordingly, the disclosure relates to the use of an EGFR inhibitor for the manufacture of a medicament for the treatment of NRAS mutated cancer wherein the NRAS mutation is selected from E63K, G12V, G12R, G12A, G12D, G12S and G12C; or a gain of *NRAS* copy number and wherein the treatment comprises administering the EGFR inhibitor in combination with a MEK inhibitor.

The disclosure also relates to use of an EGFR inhibitor for the manufacture of a medicament for treating EGFR-associated cancer in a patient, wherein the patient has an NRAS mutation selected from E63K, G12V, G12R, G12A, G12D, G12S and G12C, and wherein the treatment comprises administering the EGFR inhibitor in combination with a MEK inhibitor.

Accordingly, the disclosure relates to the use of an EGFR inhibitor for the manufacture of a medicament for the treatment of NRAS mutated cancer wherein the NRAS mutation is selected from E63K, G12V, G12R, G12A, G12D, G12S and G12C; and wherein the treatment comprises administering the EGFR inhibitor in combination with a MEK inhibitor.

The invention also relates to use of an EGFR inhibitor for the manufacture of a medicament for treating EGFR-associated cancer in a patient, wherein the patient has an NRAS mutation selected from E63K, G12V and G12R; or a gain of *NRAS* copy number, and wherein the treatment comprises administering the EGFR inhibitor in combination with a MEK inhibitor.

Accordingly, the invention relates to the use of an EGFR inhibitor for the manufacture of a medicament for the treatment of NRAS mutated cancer wherein the NRAS mutation is selected from E63K, G12V and G12R; or a gain of *NRAS* copy number and wherein the treatment comprises administering the EGFR inhibitor in combination with a MEK inhibitor.

The invention also relates to use of an EGFR inhibitor for the manufacture of a medicament for treating EGFR-associated cancer in a patient, wherein the patient has an NRAS mutation selected from E63K, G12V and G12R, and wherein the treatment comprises administering the EGFR inhibitor in combination with a MEK inhibitor. Accordingly, the invention relates to the use of an EGFR inhibitor for the manufacture of a medicament for the treatment of NRAS mutated cancer wherein the NRAS mutation is selected from E63K, G12V and G12R, and wherein the treatment comprises administering the EGFR inhibitor in combination with a MEK inhibitor.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also relates to use of an EGFR inhibitor for the manufacture of a medicament for treating EGFR-associated cancer in a patient, wherein the patient has an NRAS activating mutation selected from the group consisting of: an NRAS E63K mutation and an NRAS G12V mutation; or a gain of *NRAS* copy number, and wherein the treatment comprises administering the EGFR inhibitor in combination with a MEK inhibitor.

The disclosure also relates to use of an EGFR inhibitor and a MEK inhibitor for the manufacture of a medicament for treating EGFR-associated cancer in a patient, wherein the patient has an NRAS mutation selected from E63K, G12V, G12R, G12A, G12D, G12S and G12C; or a gain of *NRAS* copy number, and wherein the treatment comprises administering the EGFR inhibitor in combination with the MEK inhibitor.

Accordingly, the disclosure relates to the use of an EGFR inhibitor and a MEK inhibitor for the manufacture of a medicament for the treatment of NRAS mutated cancer, wherein the NRAS mutation is selected from E63K, G12V, G12R, G12A, G12D, G12S and G12C; or a gain of *NRAS* copy number, and wherein the treatment comprises administering the EGFR inhibitor in combination with the MEK inhibitor.

The disclosure also relates to use of an EGFR inhibitor and a MEK inhibitor for the manufacture of a medicament for treating EGFR-associated cancer in a patient, wherein the patient has an NRAS mutation selected from E63K, G12V, G12R, G12A, G12D, G12S and G12C, and wherein the treatment comprises administering the EGFR inhibitor in combination with the MEK inhibitor.

Accordingly, the disclosure relates to the use of an EGFR inhibitor and a MEK inhibitor for the manufacture of a medicament for the treatment of NRAS mutated cancer, wherein the NRAS mutation is selected from E63K, G12V, G12R, G12A, G12D, G12S and G12C; and wherein the treatment comprises administering the EGFR inhibitor in combination with the MEK inhibitor.

The invention also relates to use of an EGFR inhibitor and a MEK inhibitor for the manufacture of a medicament for treating EGFR-associated cancer in a patient, wherein the patient has an NRAS mutation selected from E63K, G12V and G12R; or a gain of *NRAS* copy number, and wherein the treatment comprises administering the EGFR inhibitor in combination with the MEK inhibitor.

Accordingly, the invention relates to the use of an EGFR inhibitor and a MEK inhibitor for the manufacture of a medicament for the treatment of NRAS mutated cancer, wherein the NRAS mutation is selected from E63K, G12V and G12R; or a gain of *NRAS* copy number, and wherein the treatment comprises administering the EGFR inhibitor in combination with the MEK inhibitor.

The invention also relates to use of an EGFR inhibitor and a MEK inhibitor for the manufacture of a medicament for treating EGFR-associated cancer in a patient, wherein the patient has an NRAS mutation selected from E63K, G12V and G12R, and wherein the treatment comprises administering the EGFR inhibitor in combination with the MEK inhibitor.

Accordingly, the invention relates to the use of an EGFR inhibitor and a MEK inhibitor for the manufacture of a medicament for the treatment of NRAS mutated cancer, wherein the NRAS mutation is selected from E63K, G12V and G12R; and wherein the treatment comprises administering the EGFR inhibitor in combination with the MEK inhibitor.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also relates to use of an EGFR inhibitor and a MEK inhibitor for the manufacture of a medicament for treating EGFR-associated cancer in a patient, wherein the patient has an NRAS activating mutation selected from the group consisting of: an NRAS E63K mutation and an NRAS G12V mutation; or a gain of *NRAS* copy number, and wherein the treatment comprises administering the EGFR inhibitor in combination with the MEK inhibitor.

The medicament may be a combination product as described herein.

The disclosure also provides a combination product comprising an EGFR inhibitor and a MEK inhibitor as described herein, for use in treating EGFR-associated cancer in a patient, wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration to a patient whose tumour cells comprise a gain of copy number *of NRAS* gene, or NRAS protein mutation selected from E63K, G12V, G12R, G12A, G12D, G12S and G12C.

Accordingly, the disclosure provides a combination product comprising an EGFR inhibitor and a MEK inhibitor as described herein, for use in the treatment of NRAS mutated cancer wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration and wherein the NRAS mutation is selected from E63K, G12V, G12R, G12A, G12D, G12S and G12C, or a gain of copy number of the *NRAS* gene.

The disclosure also provides a combination product comprising an EGFR inhibitor and a MEK inhibitor as described herein, for use in treating EGFR-associated cancer in a patient, wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration to a patient whose tumour cells comprise an NRAS protein mutation selected from E63K, G12V, G12R, G12A, G12D, G12S and G12C. Accordingly, the disclosure provides a combination product comprising an EGFR inhibitor and a MEK inhibitor as described herein, for use in the treatment of NRAS mutated cancer wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration and wherein the NRAS mutation is selected from E63K, G12V, G12R, G12A, G12D, G12S and G12C.

The invention also provides a combination product comprising an EGFR inhibitor and a MEK inhibitor as described herein, for use in treating EGFR-associated cancer in a patient, wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration to a patient whose tumour cells comprise a gain of copy number of *NRAS* gene, or NRAS protein mutation selected from E63K, G12V and G12R. Accordingly, the invention provides a combination product comprising an EGFR inhibitor and a MEK inhibitor as described herein, for use in the treatment of NRAS mutated cancer wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration and wherein the NRAS mutation is selected from E63K, G12V and G12R, or a gain of copy number of the *NRAS* gene.

The invention also provides a combination product comprising an EGFR inhibitor and a MEK inhibitor as described herein, for use in treating EGFR-associated cancer in a patient, wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration to a patient whose tumour cells comprise an NRAS protein mutation selected from E63K, G12V and G12R.

Accordingly, the invention provides a combination product comprising an EGFR inhibitor and a MEK inhibitor as described herein, for use in the treatment of NRAS mutated cancer wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration and wherein the NRAS mutation is selected from E63K, G12V and G12R.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also provides a combination product comprising an EGFR inhibitor and a MEK inhibitor as described herein, for use in treating EGFR-associated cancer in a patient, wherein the EGFR inhibitor and MEK inhibitor are each in association with a pharmaceutically acceptable adjuvant carrier or diluent, and wherein the EGFR inhibitor and MEK inhibitor are provided in a form which is suitable for separate, simultaneous and/or sequential administration to a patient whose tumour cells comprise a gain of copy number of *NRAS* gene or NRAS protein with E63K and/or G12V NRAS activating mutation.

The disclosure also relates to a pharmaceutical composition comprising an EGFR inhibitor and a MEK inhibitor in association with a pharmaceutically acceptable adjuvant carrier or diluents as described herein, for use in treating EGFR-associated cancer in a patient whose tumour cells comprise a gain of copy number of *NRAS* gene, or an NRAS protein mutation selected from E63K, G12V, G12R, G12A, G12D, G12S and G12C. Accordingly, the disclosure provides a pharmaceutical composition comprising an EGFR inhibitor and a MEK inhibitor in association with a pharmaceutically acceptable adjuvant carrier or diluents as described herein, for use in the treatment of NRAS mutated cancer where the NRAS mutation is selected from E63K, G12V, G12R, G12A, G12D, G12S and G12C, or a gain of copy number of the *NRAS* gene.

The disclosure also relates to a pharmaceutical composition comprising EGFR inhibitor and MEK inhibitor in association with a pharmaceutically acceptable adjuvant carrier or diluents as described herein, for use in treating EGFR-associated cancer in a patient whose tumour cells comprise an NRAS protein mutation selected from E63K, G12V, G12R, G12A, G12D, G12S and G12C.

Accordingly, the disclosure provides a pharmaceutical composition comprising an EGFR inhibitor and a MEK inhibitor in association with a pharmaceutically acceptable adjuvant carrier or diluents as described herein, for use in the treatment of NRAS mutated cancer where the NRAS mutation is selected from E63K, G12V, G12R, G12A, G12D, G12S and G12C.

The invention also relates to a pharmaceutical composition comprising EGFR inhibitor and MEK inhibitor in association with a pharmaceutically acceptable adjuvant carrier or diluents as described herein, for use in treating EGFR-associated cancer in a patient whose tumour cells comprise a gain of copy number of *NRAS* gene, or an NRAS protein mutation selected from E63K, G12V and G12R.

Accordingly, the invention provides a pharmaceutical composition comprising an EGFR inhibitor and a MEK inhibitor in association with a pharmaceutically acceptable adjuvant carrier or diluents as described herein, for use in the treatment of NRAS mutated cancer where the NRAS mutation is selected from E63K, G12V and G12R, or a gain of copy number of the *NRAS* gene.

The invention also relates to a pharmaceutical composition comprising EGFR inhibitor and MEK inhibitor in association with a pharmaceutically acceptable adjuvant carrier or diluents as described herein, for use in treating EGFR-associated cancer in a patient whose tumour cells comprise an NRAS protein mutation selected from E63K, G12V and G12R. Accordingly, the invention provides a pharmaceutical composition comprising an EGFR inhibitor and a MEK inhibitor in association with a pharmaceutically acceptable adjuvant carrier or diluents as described herein, for use in the treatment of NRAS mutated cancer where the NRAS mutation is selected from E63K, G12V and G12R.

As disclosed herein, the NRAS mutation is selected from G12R, G12A, G12D, G12S and G12C.

In further embodiments the NRAS mutated cancer is G12R NRAS mutated cancer,

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also relates to a pharmaceutical composition comprising EGFR inhibitor and MEK inhibitor in association with a pharmaceutically acceptable adjuvant carrier or diluents as described herein, for use in treating EGFR-associated cancer in a patient whose tumour cells comprise a gain of copy number of *NRAS* gene or NRAS protein with E63K and/or G12V NRAS activating mutation.

Still further the disclosure relates to use of a combination product or a pharmaceutical composition as described herein for the manufacture of a medicament for treating EGFR-associated cancer in a patient whose tumour cells comprise a gain of copy number of *NRAS* gene, or an NRAS protein mutation selected from E63K, G12V, G12R, G12A, G12D, G12S and G12C.

Accordingly, there is disclosed the use of a combination product or a pharmaceutical composition as described herein for the manufacture of a medicament for the treatment of NRAS mutated cancer wherein the NRAS mutation is selected from E63K, G12V, G12R, G12A, G12D, G12S and G12C, or a gain of copy number of the *NRAS* gene.

Still further the disclosure relates to use of a combination product or a pharmaceutical composition as described herein for the manufacture of a medicament for treating EGFR-associated cancer in a patient whose tumour cells comprise an NRAS protein mutation selected from E63K, G12V, G12R, G12A, G12D, G12S and G12C.

Accordingly, there is disclosed the use of a combination product or a pharmaceutical composition as described herein for the manufacture of a medicament for the treatment of NRAS mutated cancer wherein the NRAS mutation is selected from E63K, G12V, G12R, G12A, G12D, G12S and G12C.

Still further the invention relates to use of a combination product or a pharmaceutical composition as described herein for the manufacture of a medicament for treating EGFR-associated cancer in a patient whose tumour cells comprise a gain of copy number of *NRAS* gene, or an NRAS protein mutation selected from E63K, G12V and G12R.

Accordingly, there is provided the use of a combination product or a pharmaceutical composition as described herein for the manufacture of a medicament for the treatment of

NRAS mutated cancer wherein the NRAS mutation is selected from E63K, G12V and G12R, or a gain of copy number of the *NRAS* gene.

Still further the invention relates to use of a combination product or a pharmaceutical composition as described herein for the manufacture of a medicament for treating EGFR-associated cancer in a patient whose tumour cells comprise an NRAS protein mutation selected from E63K, G12V and G12R.

Accordingly, there is provided the use of a combination product or a pharmaceutical composition as described herein for the manufacture of a medicament for the treatment of NRAS mutated cancer wherein the NRAS mutation is selected from E63K, G12V and G12R.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

Still further the invention relates to use of a combination product or a pharmaceutical composition as described herein for the manufacture of a medicament for treating EGFR-associated cancer in a patient whose tumour cells comprise a gain of copy number of *NRAS* gene or NRAS protein with E63K and/or G12V NRAS activating mutation.

The disclosure also relates to an EGFR inhibitor and a MEK inhibitor for combined use in treating EGFR-associated cancer in a patient whose tumour cells comprise a gain of copy number of *NRAS* gene, or an NRAS protein mutation selected from E63K, G12V, G12R, G12A, G12D, G12S and G12C.

Accordingly, the disclosure relates to an EGFR inhibitor and a MEK inhibitor for combined use in the treatment of NRAS mutated cancer wherein the NRAS mutation is selected from E63K, G12V, G12R, G12A, G12D, G12S and G12C, or a gain of copy number of the *NRAS* gene.

The disclosure also relates to an EGFR inhibitor and a MEK inhibitor for combined use in treating EGFR-associated cancer in a patient whose tumour cells comprise an NRAS protein mutation selected from E63K, G12V, G12R, G12A, G12D, G12S and G12C. Accordingly, the disclosure relates to an EGFR inhibitor and a MEK inhibitor for combined use in the treatment of NRAS mutated cancer wherein the NRAS mutation is selected from E63K, G12V, G12R, G12A, G12D, G12S and G12C.

The invention also relates to an EGFR inhibitor and a MEK inhibitor for combined use in treating EGFR-associated cancer in a patient whose tumour cells comprise a gain of copy number of *NRAS* gene, or an NRAS protein mutation selected from E63K, G12V and G12R.

Accordingly, the invention relates to an EGFR inhibitor and a MEK inhibitor for combined use in the treatment of NRAS mutated cancer wherein the NRAS mutation is selected from E63K, G12V and G12R, or a gain of copy number of the *NRAS* gene.

The invention also relates to an EGFR inhibitor and a MEK inhibitor for combined use in treating EGFR-associated cancer in a patient whose tumour cells comprise an NRAS protein mutation selected from E63K, G12V and G12R.

Accordingly, the invention relates to an EGFR inhibitor and a MEK inhibitor for combined use in the treatment of NRAS mutated cancer wherein the NRAS mutation is selected from E63K, G12V and G12R.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

The invention also relates to an EGFR inhibitor and a MEK inhibitor for combined use in treating EGFR-associated cancer in a patient whose tumour cells comprise a gain of copy number of *NRAS* gene or NRAS protein with E63K and/or G12V NRAS activating mutation.

Also disclosed is an EGFR inhibitor for use in treating EGFR-associated cancer in a patient wherein the EGFR inhibitor is administered in combination with a MEK inhibitor to a patient whose tumour cells comprise a gain of copy number of *NRAS* gene or an NRAS protein mutation selected from E63K, G12V, G12R, G12A, G12D, G12S and G12C. Accordingly there is disclosed an EGFR inhibitor for use in the treatment of NRAS mutated cancer wherein the EGFR inhibitor is administered in combination with a MEK inhibitor and the NRAS mutation is selected from E63K, G12V, G12R, G12A, G12D, G12S and G12C, or a gain of copy number of the *NRAS* gene.

Also disclosed is an EGFR inhibitor for use in treating EGFR-associated cancer in a patient wherein the EGFR inhibitor is administered in combination with a MEK inhibitor to a patient whose tumour cells comprise an NRAS protein mutation selected from E63K, G12V, G12R, G12A, G12D, G12S and G12C.

Accordingly there is disclosed an EGFR inhibitor for use in the treatment of NRAS mutated cancer wherein the EGFR inhibitor is administered in combination with a MEK inhibitor and the NRAS mutation is selected from E63K, G12V, G12R, G12A, G12D, G12S and G12C.

Also provided is an EGFR inhibitor for use in treating EGFR-associated cancer in a patient wherein the EGFR inhibitor is administered in combination with a MEK inhibitor to a patient whose tumour cells comprise a gain of copy number of *NRAS* gene or an NRAS protein mutation selected from E63K, G12V and G12R.

Accordingly there is provided an EGFR inhibitor for use in the treatment of NRAS mutated cancer wherein the EGFR inhibitor is administered in combination with a MEK inhibitor and the NRAS mutation is selected from E63K, G12V and G12R, or a gain of copy number of the *NRAS* gene.

Also provided is an EGFR inhibitor for use in treating EGFR-associated cancer in a patient wherein the EGFR inhibitor is administered in combination with a MEK inhibitor to a patient whose tumour cells comprise an NRAS protein mutation selected from E63K, G12V and G12R.

Accordingly there is provided an EGFR inhibitor for use in the treatment of NRAS mutated cancer wherein the EGFR inhibitor is administered in combination with a MEK inhibitor and the NRAS mutation is selected from E63K, G12V and G12R.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

Also provided is an EGFR inhibitor for use in treating EGFR-associated cancer in a patient wherein the EGFR inhibitor is administered in combination with a MEK inhibitor to a patient whose tumour cells comprise a gain of copy number of *NRAS* gene or NRAS protein with E63K and/or G12V NRAS activating mutation.

Also disclosed is a MEK inhibitor for use in treating EGFR-associated cancer in a patient whose tumour cells comprise a gain of copy number of *NRAS* gene or an NRAS protein mutation selected from E63K, G12V, G12R, G12A, G12D, G12S and G12C, wherein the MEK inhibitor is administered in combination with an EGFR inhibitor.

Accordingly, there is disclosed a MEK inhibitor for use in the treatment of NRAS mutated cancer wherein the NRAS mutation is selected from E63K, G12V, G12R, G12A, G12D, G12S and G12C, or a gain of copy number of the *NRAS* gene, and wherein the MEK inhibitor is administered in combination with an EGFR inhibitor.

Also disclosed is a MEK inhibitor for use in treating EGFR-associated cancer in a patient whose tumour cells comprise an NRAS protein mutation selected from E63K, G12V, G12R, G12A, G12D, G12S and G12C, wherein the MEK inhibitor is administered in combination with an EGFR inhibitor.

Accordingly, there is disclosed a MEK inhibitor for use in the treatment of NRAS mutated cancer wherein the NRAS mutation is selected from E63K, G12V, G12R, G12A, G12D, G12S and G12C, and wherein the MEK inhibitor is administered in combination with an EGFR inhibitor.

Also provided is a MEK inhibitor for use in treating EGFR-associated cancer in a patient whose tumour cells comprise a gain of copy number of *NRAS* gene or an NRAS protein mutation selected from E63K, G12V and G12R, wherein the MEK inhibitor is administered in combination with an EGFR inhibitor.

Accordingly, there is provided a MEK inhibitor for use in the treatment of NRAS mutated cancer wherein the NRAS mutation is selected from E63K, G12V and G12R, or a gain of copy number of the *NRAS* gene, and wherein the MEK inhibitor is administered in combination with an EGFR inhibitor.

Also provided is a MEK inhibitor for use in treating EGFR-associated cancer in a patient whose tumour cells comprise an NRAS protein mutation selected from E63K, G12V and

G12R, wherein the MEK inhibitor is administered in combination with an EGFR inhibitor. Accordingly, there is provided a MEK inhibitor for use in the treatment of NRAS mutated cancer wherein the NRAS mutation is selected from E63K, G12V and G12R, and wherein the MEK inhibitor is administered in combination with an EGFR inhibitor.

As disclosed herein, the NRAS mutation is selected from G12R, G12A, G12D, G12S and G12C.

In further embodiments the NRAS mutated cancer is G12R NRAS mutated cancer.

In further embodiments the EGFR inhibitor is selected from AZD9291 and CO1686; or a pharmaceutically acceptable salt thereof.

In further embodiments the EGFR inhibitor is AZD9291 or a pharmaceutically acceptable salt thereof. In further embodiments the EGFR inhibitor is AZD9291 or CO1686; or a pharmaceutically acceptable salt thereof, and the MEK inhibitor is selumetinib or a pharmaceutically acceptable salt thereof.

In further embodiments the EGFR inhibitor is AZD9291 or a pharmaceutically acceptable salt thereof, and the MEK inhibitor is selumetinib or a pharmaceutically acceptable salt thereof.

Also provided is a MEK inhibitor for use in treating EGFR-associated cancer in a patient whose tumour cells comprise a gain of copy number of *NRAS* gene or NRAS protein with E63K and/or G12V NRAS activating mutation, wherein the MEK inhibitor is administered in combination with an EGFR inhibitor.

The disclosure also relates to use of a MEK inhibitor for the manufacture of a medicament for treating EGFR-associated cancer in a patient whose tumour cells comprise a gain of copy number of *NRAS* gene, or an NRAS protein mutation selected from E63K, G12V, G12R, G12A, G12D, G12S and G12C, wherein the treatment comprises administering the MEK inhibitor in combination with an EGFR inhibitor.

The disclosure also relates to use of a MEK inhibitor for the manufacture of a medicament for treating EGFR-associated cancer in a patient whose tumour cells comprise an NRAS protein mutation selected from E63K, G12V, G12R, G12A, G12D, G12S and G12C, wherein the treatment comprises administering the MEK inhibitor in combination with an EGFR inhibitor.

The disclosure also relates to use of a MEK inhibitor for the manufacture of a medicament for treating EGFR-associated cancer in a patient whose tumour cells comprise a gain of copy number of *NRAS* gene, or an NRAS protein mutation selected from E63K, G12V and G12R, wherein the treatment comprises administering the MEK inhibitor in combination with an EGFR inhibitor.

The disclosure also relates to use of a MEK inhibitor for the manufacture of a medicament for treating EGFR-associated cancer in a patient whose tumour cells comprise an NRAS protein mutation selected from E63K, G12V and G12R, wherein the treatment comprises administering the MEK inhibitor in combination with an EGFR inhibitor.

As disclosed herein, the NRAS mutation is selected from G12R, G12A, G12D, G12S and G12C.

As disclosed herein, the NRAS mutation is G12R.

As disclosed herein, the EGFR inhibitor is selected from AZD9291 and CO1686; or a pharmaceutically acceptable salt thereof.

As disclosed herein, the EGFR inhibitor is AZD9291 or a pharmaceutically acceptable salt thereof. As disclosed herein, the EGFR inhibitor is AZD9291 or CO1686; or a pharmaceutically acceptable salt thereof, and the MEK inhibitor is selumetinib or a pharmaceutically acceptable salt thereof.

As disclosed herein, the EGFR inhibitor is AZD9291 or a pharmaceutically acceptable salt thereof, and the MEK inhibitor is selumetinib or a pharmaceutically acceptable salt thereof. The disclosure also relates to use of a MEK inhibitor for the manufacture of a medicament for treating EGFR-associated cancer in a patient whose tumour cells comprise a gain of copy number of *NRAS* gene or NRAS protein with E63K and/or G12V NRAS activating mutation, wherein the treatment comprises administering the MEK inhibitor in combination with an EGFR inhibitor.

The disclosure also relates to use of an EGFR inhibitor for the manufacture of a medicament for treating EGFR-associated cancer in a patient whose tumour cells comprise a gain of copy number of *NRAS* gene, or an NRAS protein mutation selected from E63K, G12V, G12R, G12A, G12D, G12S and G12C, wherein the treatment comprises administering the EGFR inhibitor in combination with a MEK inhibitor.

The disclosure also relates to use of an EGFR inhibitor for the manufacture of a medicament for treating EGFR-associated cancer in a patient whose tumour cells comprise an NRAS protein mutation selected from E63K, G12V, G12R, G12A, G12D, G12S and G12C, wherein the treatment comprises administering the EGFR inhibitor in combination with a MEK inhibitor.

The disclosure also relates to use of an EGFR inhibitor for the manufacture of a medicament for treating EGFR-associated cancer in a patient whose tumour cells comprise a gain of copy number of *NRAS* gene, or an NRAS protein mutation selected from E63K, G12V and G12R, wherein the treatment comprises administering the EGFR inhibitor in combination with a MEK inhibitor.

The disclosure also relates to use of an EGFR inhibitor for the manufacture of a medicament for treating EGFR-associated cancer in a patient whose tumour cells comprise an NRAS protein mutation selected from E63K, G12V and G12R, wherein the treatment comprises administering the EGFR inhibitor in combination with a MEK inhibitor.

As disclosed herein, the NRAS mutation is selected from G12R, G12A, G12D, G12S and G12C.

As disclosed herein, the NRAS mutation is G12R.

As disclosed herein, the EGFR inhibitor is selected from AZD9291 and CO1686; or a pharmaceutically acceptable salt thereof.

As disclosed herein, the EGFR inhibitor is AZD9291 or a pharmaceutically acceptable salt thereof. As disclosed herein, the EGFR inhibitor is AZD9291 or CO1686; or a pharmaceutically acceptable salt thereof, and the MEK inhibitor is selumetinib or a pharmaceutically acceptable salt thereof.

As disclosed herein, the EGFR inhibitor is AZD9291 or a pharmaceutically acceptable salt thereof, and the MEK inhibitor is selumetinib or a pharmaceutically acceptable salt thereof. The disclosure also relates to use of an EGFR inhibitor for the manufacture of a medicament for treating EGFR-associated cancer in a patient whose tumour cells comprise a gain of copy number of *NRAS* gene or NRAS protein with E63K and/or G12V NRAS activating mutation, wherein the treatment comprises administering the EGFR inhibitor in combination with a MEK inhibitor.

The disclosure also relates to use of an EGFR inhibitor and a MEK inhibitor for the manufacture of a medicament for treating EGFR-associated cancer in a patient whose tumour cells comprise a gain of copy number of *NRAS* gene, or an NRAS protein mutation selected from E63K, G12V, G12R, G12A, G12D, G12S and G12C, wherein the treatment comprises administering the EGFR inhibitor in combination with the MEK inhibitor.

The disclosure also relates to use of an EGFR inhibitor and a MEK inhibitor for the manufacture of a medicament for treating EGFR-associated cancer in a patient whose tumour cells comprise, or an NRAS protein mutation selected from E63K, G12V, G12R, G12A, G12D, G12S and G12C, wherein the treatment comprises administering the EGFR inhibitor in combination with the MEK inhibitor.

The disclosure also relates to use of an EGFR inhibitor and a MEK inhibitor for the manufacture of a medicament for treating EGFR-associated cancer in a patient whose tumour cells comprise a gain of copy number of *NRAS* gene, or an NRAS protein mutation selected from E63K, G12V and G12R, wherein the treatment comprises administering the EGFR inhibitor in combination with the MEK inhibitor.

The disclosure also relates to use of an EGFR inhibitor and a MEK inhibitor for the manufacture of a medicament for treating EGFR-associated cancer in a patient whose tumour cells comprise, or an NRAS protein mutation selected from E63K, G12V and G12R, wherein the treatment comprises administering the EGFR inhibitor in combination with the MEK inhibitor.

As disclosed herein, the NRAS mutation is selected from G12R, G12A, G12D, G12S and G12C.

As disclosed herein, the NRAS mutation is G12R.

As disclosed herein, the EGFR inhibitor is selected from AZD9291 and CO1686; or a pharmaceutically acceptable salt thereof.

As disclosed herein, the EGFR inhibitor is AZD9291 or a pharmaceutically acceptable salt thereof. As disclosed herein, the EGFR inhibitor is AZD9291 or CO1686; or a pharmaceutically acceptable salt thereof, and the MEK inhibitor is selumetinib or a pharmaceutically acceptable salt thereof.

As disclosed herein, the EGFR inhibitor is AZD9291 or a pharmaceutically acceptable salt thereof, and the MEK inhibitor is selumetinib or a pharmaceutically acceptable salt thereof.

The disclosure also relates to use of an EGFR inhibitor and a MEK inhibitor for the manufacture of a medicament for treating EGFR-associated cancer in a patient whose tumour cells comprise a gain of copy number of *NRAS* gene or NRAS protein with E63K and/or G12V NRAS activating mutation, wherein the treatment comprises administering the EGFR inhibitor in combination with the MEK inhibitor.

The medicament may be a combination product as described herein.

### Further embodiments

Accordingly, there is provided an EGFR inhibitor in combination with a MEK inhibitor for use in the treatment of E63K NRAS mutated cancer.

Accordingly, there is provided an EGFR inhibitor in combination with a MEK inhibitor for use in the treatment of G12V NRAS mutated cancer.

In a further embodiment there is provided EGFR inhibitor in combination with a MEK inhibitor for use in the treatment of G12R NRAS mutated cancer.

In any embodiments described herein, the EGFR inhibitor may be selected from gefitinib, erlotinib, afatinib, dacomitinib, AZD9291 and CO-1686 (where all of the aforementioned may be in the form of a pharmaceutically acceptable salt, provided there is at least a functional group permitting such salts to be formed).

In any embodiments described herein, the EGFR inhibitor may be selected from gefitinib, erlotinib, afatinib, AZD9291 and CO-1686 (where all of the aforementioned may be in the form of a pharmaceutically acceptable salt, provided there is at least a functional group permitting such salts to be formed).

Accordingly, there is disclosed an EGFR inhibitor in combination with a MEK inhibitor for use in the treatment of NRAS mutated cancer where the NRAS mutation is selected from E63K, G12V, G12R, G12A, G12D, G12S and G12C, wherein the EGFR inhibitor is selected from gefitinib, erlotinib, afatinib, AZD9291 and CO-1686; or a pharmaceutically acceptable salt thereof.

As disclosed herein, the NRAS mutation is selected from G12R, G12A, G12D, G12S and G12C.

In further embodiments the NRAS mutation is G12R.

Accordingly, there is provided an EGFR inhibitor in combination with a MEK inhibitor for use in the treatment of NRAS mutated cancer where the NRAS mutation is selected from E63K, G12V and G12R, wherein the EGFR inhibitor is selected from gefitinib, erlotinib, afatinib, AZD9291 and CO-1686; or a pharmaceutically acceptable salt thereof. Accordingly, there is disclosed an EGFR inhibitor in combination with a MEK inhibitor for use in the treatment of NRAS mutated cancer where the NRAS mutation is selected from G12R, G12A, G12D, G12S and G12C, wherein the EGFR inhibitor is selected from gefitinib, erlotinib, afatinib, AZD9291 and CO-1686; or a pharmaceutically acceptable salt thereof.

Accordingly, there is provided an EGFR inhibitor in combination with a MEK inhibitor for use in the treatment of G12R NRAS mutated cancer, wherein the EGFR inhibitor is selected from gefitinib, erlotinib, afatinib, AZD9291 and CO-1686; or a pharmaceutically acceptable salt thereof.

Accordingly, there is provided an EGFR inhibitor in combination with a MEK inhibitor for use in the treatment of cancer in a patient whose tumour cells comprise a gain of copy number of *NRAS* gene, wherein the EGFR inhibitor is selected from gefitinib, erlotinib, afatinib, AZD9291 and CO-1686; or a pharmaceutically acceptable salt thereof. Accordingly, there is provided an EGFR inhibitor in combination with a MEK inhibitor for use in the treatment of E63K NRAS mutated cancer, wherein the EGFR inhibitor is selected from gefitinib, erlotinib, afatinib, AZD9291 and CO-1686; or a pharmaceutically acceptable salt thereof.

Accordingly, there is provided an EGFR inhibitor in combination with a MEK inhibitor for use in the treatment of G12V NRAS mutated cancer, wherein the EGFR inhibitor is selected from gefitinib, erlotinib, afatinib, AZD9291 and CO-1686; or a pharmaceutically acceptable salt thereof.

Accordingly, there is provided an EGFR inhibitor in combination with a MEK inhibitor for use in the treatment of G12R NRAS mutated cancer, wherein the EGFR inhibitor is selected from gefitinib, erlotinib, afatinib, AZD9291 and CO-1686; or a pharmaceutically acceptable salt thereof.

In any embodiments described herein the MEK inhibitor may be selected from selumetinib, trametinib, MEK-162 and cobimetinib (where all of the aforementioned may be in the form of a pharmaceutically acceptable salt, provided there is at least functional group permitting such salts to be formed).

Accordingly, there is disclosed an EGFR inhibitor in combination with a MEK inhibitor for use in the treatment of NRAS mutated cancer, wherein the NRAS mutation is selected from E63K, G12V, G12R, G12A, G12D, G12S and G12C, wherein the MEK inhibitor is selected from selumetinib, trametinib, MEK-162 and cobimetinib; or a pharmaceutically acceptable salt thereof.

Accordingly, there is disclosed an EGFR inhibitor in combination with a MEK inhibitor for use in the treatment of NRAS mutated cancer, wherein the NRAS mutation is selected from G12R, G12A, G12D, G12S and G12C, wherein the MEK inhibitor is selected from selumetinib, trametinib, MEK-162 and cobimetinib; or a pharmaceutically acceptable salt thereof.

Accordingly, there is provided an EGFR inhibitor in combination with a MEK inhibitor for use in the treatment of NRAS mutated cancer, wherein the NRAS mutation is selected from E63K, G12V and G12R, wherein the MEK inhibitor is selected from selumetinib, trametinib, MEK-162 and cobimetinib; or a pharmaceutically acceptable salt thereof.

Accordingly, there is provided an EGFR inhibitor in combination with a MEK inhibitor for use in the treatment of G12R NRAS mutated cancer, wherein the MEK inhibitor is selected from selumetinib, trametinib, MEK-162 and cobimetinib; or a pharmaceutically acceptable salt thereof.

Accordingly, there is provided an EGFR inhibitor in combination with a MEK inhibitor for use in the treatment of E63K NRAS mutated cancer, wherein the MEK inhibitor is selected from selumetinib, trametinib, MEK-162 and cobimetinib; or a pharmaceutically acceptable salt thereof.

Accordingly, there is provided an EGFR inhibitor in combination with a MEK inhibitor for use in the treatment of G12V NRAS mutated cancer, wherein the MEK inhibitor is selected from selumetinib, trametinib, MEK-162 and cobimetinib; or a pharmaceutically acceptable salt thereof.

Accordingly there is provided an EGFR inhibitor in combination with a MEK inhibitor for use in the treatment of G12R NRAS mutated cancer, wherein the MEK inhibitor is selected from selumetinib, trametinib, MEK-162 and cobimetinib; or a pharmaceutically acceptable salt thereof.

As disclosed herein, there is provided the an EGFR inhibitor in combination with a MEK inhibitor for use in the treatment of NRAS mutated cancer wherein the NRAS mutation is selected from E63K, G12V, G12R, G12A, G12D, G12S and G12C, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

As disclosed herein, there is provided an EGFR inhibitor in combination with a MEK inhibitor for use in the treatment of NRAS mutated cancer wherein the NRAS mutation is selected from G12R, G12A, G12D, G12S and G12C, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

In one embodiment there is provided an EGFR inhibitor in combination with a MEK inhibitor for use in the treatment of NRAS mutated cancer wherein the NRAS mutation is selected from E63K, G12V and G12R, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

Accordingly, there is provided an EGFR inhibitor in combination with a MEK inhibitor for use in the treatment of E63K and/or G12V NRAS mutated cancer, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

Accordingly there is provided an EGFR inhibitor in combination with a MEK inhibitor for use in the treatment of cancer in a patient whose tumour cells comprise a gain of copy number of *NRAS* gene, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

Accordingly, there is provided an EGFR inhibitor in combination with a MEK inhibitor for use in the treatment of E63K NRAS mutated cancer, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof. Accordingly, there is provided an EGFR inhibitor in combination with a MEK inhibitor for use in the treatment of G12V NRAS mutated cancer, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

In one embodiment there is provided the an EGFR inhibitor in combination with a MEK inhibitor for use in the treatment of G12R NRAS mutated cancer, wherein the EGFR inhibitor is selected from gefitinib, erlotinib, afatinib, AZD9291 and CO-1686; or a pharmaceutically acceptable salt thereof, and wherein the MEK inhibitor is selected from selumetinib, trametinib, MEK-162 and cobimetinib; or a pharmaceutically acceptable salt thereof.

In any embodiments described herein, the MEK inhibitor may be selumetinib or a pharmaceutically acceptable salt thereof.

As disclosed herein, there is provided the an EGFR inhibitor in combination with a MEK inhibitor for use in the treatment of NRAS mutated cancer, wherein the NRAS mutation is selected from E63K, G12V, G12R, G12A, G12D, G12S and G12C, and wherein the MEK inhibitor is selumetinib or a pharmaceutically acceptable salt thereof.

As disclosed herein, there is provided the an EGFR inhibitor in combination with a MEK inhibitor for use in the treatment of NRAS mutated cancer, wherein the NRAS mutation is selected from G12R, G12A, G12D, G12S and G12C, and wherein the MEK inhibitor is selumetinib or a pharmaceutically acceptable salt thereof.

In one embodiment there is provided an EGFR inhibitor in combination with a MEK inhibitor for use in the treatment of NRAS mutated cancer, wherein the NRAS mutation is selected from E63K, G12V and G12R, and wherein the MEK inhibitor is selumetinib or a pharmaceutically acceptable salt thereof.

Accordingly there is provided an EGFR inhibitor in combination with a MEK inhibitor for use in the treatment of E63K and/or G12V NRAS mutated cancer, wherein the MEK inhibitor is selumetinib or a pharmaceutically acceptable salt thereof.

In one embodiment there is provided an EGFR inhibitor in combination with a MEK inhibitor for use in the treatment of G12R NRAS mutated cancer, wherein the MEK inhibitor is selumetinib or a pharmaceutically acceptable salt thereof.

Accordingly, there is provided an EGFR inhibitor in combination with a MEK inhibitor for use in the treatment of cancer in a patient whose tumour cells comprise a gain of copy number of *NRAS* gene, wherein the MEK inhibitor is selumetinib; or a pharmaceutically acceptable salt thereof.

Accordingly, there is provided an EGFR inhibitor in combination with a MEK inhibitor for use in the treatment of E63K NRAS mutated cancer, wherein the MEK inhibitor is selumetinib; or a pharmaceutically acceptable salt thereof.

Accordingly, there is provided an EGFR inhibitor in combination with a MEK inhibitor for use in the treatment of G12V NRAS mutated cancer, wherein the MEK inhibitor is selumetinib or a pharmaceutically acceptable salt thereof.

In any embodiments described herein the EGFR inhibitor may be selected from gefitinib and AZD9291 and the MEK inhibitor is selumetinib (where any of the aforementioned may be in the form of a pharmaceutically acceptable salt).

Accordingly, there is provided an EGFR inhibitor in combination with a MEK inhibitor for use in the treatment of E63K and/or G12V NRAS mutated cancer, wherein the EGFR inhibitor is selected from gefitinib and AZD9291; or a pharmaceutically acceptable salt thereof, and wherein the MEK inhibitor is selumetinib or a pharmaceutically acceptable salt thereof.

In one embodiment there is provided the an EGFR inhibitor in combination with a MEK inhibitor for use in the treatment of G12R NRAS mutated cancer, wherein the EGFR inhibitor is selected from gefitinib and AZD9291; or a pharmaceutically acceptable salt thereof, and wherein the MEK inhibitor is selumetinib or a pharmaceutically acceptable salt thereof.

In one embodiment there is provided the an EGFR inhibitor in combination with a MEK inhibitor for use in the treatment of G12R NRAS mutated cancer, wherein the EGFR inhibitor is selected from AZD9291 and CO1686; or a pharmaceutically acceptable salt thereof, and wherein the MEK inhibitor is selumetinib or a pharmaceutically acceptable salt thereof.

In one embodiment there is provided the an EGFR inhibitor in combination with a MEK inhibitor for use in the treatment of G12R NRAS mutated cancer, wherein the EGFR inhibitor is AZD9291 or a pharmaceutically acceptable salt thereof, and wherein the MEK inhibitor is selumetinib or a pharmaceutically acceptable salt thereof.

Accordingly, there is provided an EGFR inhibitor in combination with a MEK inhibitor for use in the treatment of cancer in a patient whose tumour cells comprise a gain of copy number of *NRAS* gene, wherein the EGFR inhibitor is selected from gefitinib and AZD9291; or a pharmaceutically acceptable salt thereof, and wherein the MEK inhibitor is selumetinib or a pharmaceutically acceptable salt thereof.

Accordingly, there is provided an EGFR inhibitor in combination with a MEK inhibitor for use in the treatment of E63K NRAS mutated cancer, wherein the EGFR inhibitor is selected from gefitinib and AZD9291; or a pharmaceutically acceptable salt thereof, and wherein the MEK inhibitor is selumetinib or a pharmaceutically acceptable salt thereof. Accordingly, there is provided an EGFR inhibitor in combination with a MEK inhibitor for use in the treatment of G12V NRAS mutated cancer, wherein the EGFR inhibitor is selected from gefitinib and AZD9291; or a pharmaceutically acceptable salt thereof, and wherein the MEK inhibitor is selumetinib or a pharmaceutically acceptable salt thereof.

In any embodiments herein describing "E63K NRAS mutated cancer" the cancer may be "E63K NRAS mutated lung cancer" or "E63K NRAS mutated non-small cell lung cancer" or "E63K NRAS mutated cancer that has previously been treated with an EGFR TKI".

In any embodiments herein describing "G12V NRAS mutated cancer" the cancer may be "G12V NRAS mutated lung cancer" or "G12V NRAS mutated non-small cell lung cancer" or a "G12V NRAS mutated cancer that has previously been treated with an EGFR TKI". The same pattern may be applied to the other NRAS protein mutations to provide further embodiments, as described herein.

In any embodiments herein describing "NRAS mutated cancer" the cancer may be further defined as "NRAS mutated lung cancer" or "NRAS mutated non-small cell lung cancer" or "NRAS mutated cancer that has previously been treated with an EGFR TKI".

In any embodiments herein describing "cancer in a patient whose tumour cells comprise a gain of copy number of *NRAS* gene" the cancer may be "non-small cell lung cancer whose tumour cells comprise a gain of copy number of *NRAS* gene" or "cancer in a patient whose tumour cells comprise a gain of copy number of *NRAS* gene wherein the cancer has previously been treated with an EGFR TKI".

### Synergy of combination treatment

A combination treatment of the present invention is expected to produce a synergistic or beneficial effect in treating a cancer in a subject. Such an effect may be determined, for example, by one or more of the extent of anti-tumour effect, the response rate, the time to disease progression, or the survival rate.

In one aspect, a synergistic or beneficial effect is achieved if the effect is therapeutically superior, as measured by, for example, the extent of the response, the response/recovery rate, the time to disease progression, side-effects experienced, or the survival period, to that achievable on applying one of the components of the combination treatment, for example, at its conventional dose or concentration.

A synergistic or beneficial effect may be obtained if the combined effect is therapeutically superior to the sum of the individual effects achieved using one of the components of the combination, and/or if an effect is achieved in a group of patients that does not respond (or responds poorly) to one of the components individually.

In addition, a combination treatment may be defined as affording a synergistic or beneficial effect if one of the components is applied at its conventional dose or concentration, and the other component(s) is/are applied at a reduced dose or concentration and the therapeutic effect is equivalent to or better than, that achievable on applying conventional amounts of the components of the combination treatment.

In particular, synergy or benefit may be deemed to be present if a conventional dose of one of the components of the combination treatment may be reduced without detriment to one or more of: the extent of the response, the response rate, the time to disease progression and survival data, in particular without detriment to the duration of the response, but with fewer and/or less troublesome side-effects than those that occur when conventional doses or concentrations of each component are used.

### Formulations and delivery routes

A pharmaceutical composition or combination product for use in vivo typically comprises active agent or agents (e.g. EGFR inhibitor and/or MEK inhibitor as described herein), in admixture with one or more pharmaceutically acceptable excipients, carriers or diluents, adjuvants, fillers, buffers, stabilisers, preservatives, lubricants, or other materials well known to those skilled in the art.

Pharmaceutically acceptable excipients useful herein are conventional. Remington's Pharmaceutical Sciences, by E. W. Martin, Mack Publishing Co, Easton, PA, 15th Edition (1975), describes compositions and formulations suitable for pharmaceutical delivery of the compounds herein disclosed.

Such formulations may further routinely contain pharmaceutically acceptable concentrations of salt, buffering agents, preservatives, antioxidants and/or compatible carriers.

Formulations may also include antioxidants and/or preservatives. As antioxidants may be mentioned tocopherols, butylated hydroxyanisole, butylated hydroxytoluene, sulfurous acid salts (e.g. sodium sulfate, sodium bisulfite, acetone sodium bisulfite, sodium metabisulfite, sodium sulfite, sodium formaldehyde sulfoxylate, sodium thiosulfate) and nordihydroguaiareticacid. Suitable preservatives may for instance be phenol, chlorobutanol, benzylalcohol, methyl paraben, propyl paraben, benzalkonium chloride and cetylpyridinium chloride.

Formulations may be presented in unit dosage form.

Compositions and products described herein may be delivered to target cells (or to tissue, organ or subject comprising the target cells, by any suitable means.

Examples of administration routes and/or delivery means for delivery to a subject include: oral, parenteral, transdermal, intradermal, inter-arterial or intravenous or topical. In one example, administration may be by intravenous, inter-arterial or subcutaneous injection or infusion, or by oral administration.

A composition or product may be for oral administration. In one aspect, an oral composition may comprise an oral dosage form comprising an active agent in combination with an enhancer to improve bioavailability and/or absorption of the agent.

Formulations suitable for oral administration (e.g., by ingestion) may be presented as discrete units such as capsules, cachets or tablets, each containing a predetermined amount of the active agent; as a powder or granules; as a solution or suspension in an aqueous or non-aqueous liquid; or as an oil-in-water liquid emulsion or a water-in-oil liquid emulsion; as a bolus; as an electuary; or as a paste.

In one example, a composition or product may be for parenteral administration. Parenteral preparations can be administered by one or more routes, such as intravenous, subcutaneous, intradermal and infusion; a particular example is intravenous. A formulation disclosed herein may be administered using a syringe, injector, plunger for solid formulations, pump, or any other device recognized in the art for parenteral administration.

A composition or product may be for topical administration, for example to the skin.

### Amounts/doses/therapeutically effective

Actual amounts (e.g. dosage levels) or concentrations of active ingredient (e.g. EGFR inhibitor, or MEK inhibitor, or other active agent) in compositions, e.g. pharmaceutical compositions, may be varied so as to obtain an amount of active ingredient that is effective to achieve the desired therapeutic response, for a particular subject, composition, and mode of administration (referred to herein as a "therapeutically effective" amount or dose).

The selected dosage level may, for example, depend upon the activity of the particular active ingredient, the severity of the condition being treated and the condition and, if appropriate, prior medical history of the subject being treated. However, it is within the skill of the art to start doses at levels lower than required for to achieve the desired effect and to gradually increase the dosage until the desired effect is achieved.

The dosage of an inhibitor in a combination described herein for a given subject or patient may be determined by an attending physician or other skilled person, taking into consideration various factors known to modify the action of drugs including severity and type of disease or condition, body weight, sex, diet, time and route of administration, other medications and other relevant factors, e.g. clinical factors. Therapeutically effective dosages may be determined by either *in vitro* or *in vivo* methods.

The therapeutically effective amount to be used will depend, for example, upon the therapeutic objectives, the route of administration, and the condition of the subject. Accordingly, it is preferred for the therapist or other skilled person to titer the dosage and modify the route of administration as required to obtain the optimal therapeutic effect. A typical daily dosage might range from about 0.0001mg/kg to up to 250mg/kg or more, depending on the factors mentioned above. Typically, the clinician or other skilled person will administer the inhibitor or combination (e.g. combination product), as described herein, until a dosage is reached that achieves the desired effect. Where separate formulations of agents in a combination are administered, the sequence in which the agents in the combination may be administered (i.e. whether and at what point sequential, separate and/or simultaneous administration takes place) may be determined by the physician or skilled person.

Administration of a combination of agents may take place as hereinbefore described, for example separate formulations of agents may be administered sequentially, separately and/or simultaneously.

### Pharmaceutically acceptable salts

The invention also relates to pharmaceutically acceptable salts of specific EGFR inhibitors and MEK inhibitors referred to herein. Reference herein to "EGFR inhibitor" or "MEK inhibitor" may be interpreted accordingly to encompass both specific named inhibitors and their pharmaceutically acceptable salts.

Uses and applications described for a named inhibitor may be considered to apply mutatis mutandis to pharmaceutically acceptable salts thereof.

The term "pharmaceutically acceptable" as used herein pertains to compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio.

It may be convenient or desirable to prepare, purify, and/or handle a corresponding salt of an agent described herein (e.g. an EGFR inhibitor or an MEK inhibitor), for example, a pharmaceutically-acceptable salt.

A suitable pharmaceutically-acceptable salt may be, for example, an acid-addition salt which is sufficiently basic, for example an acid-addition salt with an inorganic or organic acid. Such acid-addition salts include but are not limited to, furmarate, methanesulfonate, hydrochloride, hydrobromide, citrate and maleate salts and salts formed with phosphoric and sulfuric acid. A suitable pharmaceutically-acceptable salt may be, for example, a salt which is sufficiently acidic, for example an alkali or alkaline earth metal salt. Such alkali or alkaline earth metal salts include but are not limited to, an alkali metal salt for example sodium or potassium, an alkaline earth metal salt for example calcium or magnesium, an ammonium salt, or organic amine salt for example triethylamine, ethanolamine, diethanolamine, triethanolamine, morpholine, *N-*methylpiperidine, *N-*ethylpiperidine, dibenzylamine or amino acids such as lysine. A salt may be for example, a mesylate salt or a HBr salt.

### EXAMPLES

The invention will now be described by way of specific Examples and with reference to the accompanying Figures, which are provided for illustrative purposes only and are not to be construed as limiting upon the teachings herein.

### Example 1. Generation of PC9 gefitinib-resistant cell population and PC9 AZD9291 resistant cell population

### Reagents

RPMI-1640 medium (Sigma R7509)
Dulbeccos Phosphate buffered saline (PBS) (Sigma D8537)
L-glutamine 200mM (100 x) (Gibco, Life Technologies 25030)
Foetal Calf Serum (Sigma F7524)
TrypLE Express (Gibco, Life Technologies 12605)
AZD9291 and gefitinib (in house)

### Growth media

RPMI-1640 medium
10% Foetal calf serum
2mM L-glutamine

### Cells

PC9 human NSCLC-derived cells.
All the reagents, compounds and cells are available from commercial sources.

### Generation of PC9 gefitinib, AZD9291 or afatinib-resistant cell populations using a dose escalation method

PC9 cells were seeded at 5 x 10⁵ cells in multiple fresh T75 flasks in growth media and incubated at 37°C, 5% CO₂. The following day the media in the flasks was removed and replaced with fresh growth media supplemented with either 20nM gefitinib, 10nM AZD9291 or 0.8nM afaitnib (these concentrations represent the concentration of either gefitinib, AZD9291 or afatinib required to inhibit growth of PC9 cells by 50% (EC₅₀) as previously determined). The cells were returned to the incubator and culturing continued in media supplemented with 20nM gefitinib, 10nM AZD9291 or 0.8nM afatinib with media changes every 2 - 3 days.

For each of the PC9 flasks initially most of the cells in the culture died and became detached from the flask, these cells were removed as the media was changed. A small number of the cells remained attached to the flask and started to grow as resistant colonies. Growth of these colonies was continued until the flask was about 80% confluent. At this stage the media was removed from the flask and 10mls of PBS added. The PBS was gently washed over the cells and removed. 2mls of TrypLE Express were added and the flask rocked to ensure coverage of all of the cells with the trypsin solution. The cells were incubated at 37°C, 5% CO₂ for 10 minutes. The flask was then gently tapped to dislodge all of the cells and the cells resuspended in a total of 10mls of growth media supplemented with gefitinib, AZD9291 or afatinib as above. The cells were counted and used to seed 5 x 10⁵ cells in fresh T75 flasks in growth media supplemented with either 40nM gefitinib, 20nM AZD9291 or 1.6nM afatinib (ie. a doubling of the gefitinib, AZD9291 or afatinib concentration).

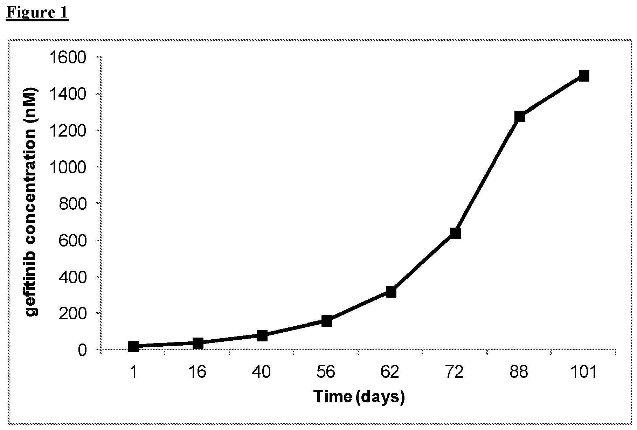

For each of the PC9 flasks culturing and passaging of the cells was continued as described above with the gefitinib, AZD9291 or afatinib concentrations doubled each time the resistant cells reached about 80% confluency until a maximum concentration of 1500nM gefitinib, 160nM AZD9291 or 1500nM afatinib was achieved. For each resistant population the time taken to reach each concentration doubling (i.e. time taken for PC9 cells to reach about 80% confluency) was recorded and plotted against concentration. **Figure 1** shows example data plotted for the time taken to generate a population of PC9 cells resistant to gefitinib. The data indicates that once the cells have become resistant to ∼320nM gefitinib (∼62 days) further increases in concentration of the inhibitor have less effect on the growth rate and survival of the cells. This suggests that these PC9 cells have acquired a mechanism of resistance over the first 62 days that can circumvent EGFR inhibition and allow the cells to survive even when the concentration of gefitinib is increased further to 1500nM.

These resistant cells were maintained as a population of cells resistant to gefitinib. The resistant populations generated were expanded and cell samples frozen for further molecular profiling to identify potential resistance mechanisms within the population.

### Generation of PC9 AZD9291 resistant cell populations using a single concentration of AZD9291.

PC9 cells were seeded at 5 x 10⁵ cells in a fresh T75 flask in growth media and incubated at 37°C, 5% CO₂. The following day the media in the flask was removed and replaced with fresh growth media supplemented with 160nM AZD9291 (previously determined as a clinically relevant concentration of AZD9291). The cells were returned to the incubator and culturing continued in media supplemented with 160nM AZD9291 with media changes every 2 - 3 days. Initially most of the cells in the culture died and became detached from the flask, these cells were removed as the media was changed. A very small number of the cells remained attached to the flask and started to grow as resistant colonies. Growth of these colonies was continued until the flask was about 80% confluent (∼70 days in culture).

This population of cells was expanded and cell samples frozen for further analysis to identify potential resistance mechanisms within the population.

### Example 2. Genetic profiling of gefitinib, AZD9291 and afatinib resistant PC9 cell populations and identification of NRAS alterations.

### Preparation of cell pellets from resistant cells

Samples of the PC9 gefitinib resistant, PC9 AZD9291 resistant and PC9 afatinib resistant cell populations were cultured in T75 flasks until they were about 80% confluent. The cells were trypsinised as described previously and resuspended in a total volume of 10mls of PBS. The cells were pelleted by centrifuging at 1000rpm for 5 minutes and washed in a further 10mL of PBS. The cells were repelleted and as much PBS removed as possible. The cell pellets were frozen at -20°C for a maximum of 1 week prior to further processing. Similar methods were used to obtain cell pellets from other cell populations, e.g. parental PC9 cells, as necessary.

### Preparation of DNA from cells

DNA samples were prepared using the Allprep DNA/RNA/miRNA Universal kit (Qiagen) according to the manufacturer's instructions, and including an RNase step to prevent RNA carry-over.

The DNA samples were used to determine the presence of DNA mutations and/or gene copy number changes using at least two platforms: next generation sequencing (NGS) and array comparative genomic hybridisation (aCGH).

### Identification of the NRAS E63K, G12V and G12R mutations

NGS was carried out for the gefitinb-resistant, AZD9291-resistant and afatinib-resistant PC9 cell populations. Analysis was carried out using the Qiagen GeneRead system (multiplexed PCR primers targeting all exons from 20 lung cancer related genes) to enrich purified DNA, followed by sequencing using Illumina technology. Results were validated by sequencing a subset of the samples using Sanger sequencing, Life Technologies Ion Torrent PGM sequencing, or Agilent Haloplex™ enrichment followed by Illumina sequencing. Sanger sequencing was interpreted by direct reading of chromatograms and Next Generation Sequencing based methods by standard methodologies - sequencing read QC, alignment to the human genome reference hg19, variant identification and calling of mutations after visual inspection

Overall, a number of PC9 gefitinib resistant cell lines (PC9 IR-GM, PC9 IRLR, PC9 IR-4, PCR IR-6) and AZD9291-resistant cell lines (PC9 9291-5, PC9 9291_6, PC9 9291-LOB_1, PC9 9291-3 and PC9 9291-2) were analysed, as well as the parental PC9 cell line. A new NRAS mutation, E63K, was found in one gefitinib-resistant cell line (PC9 IR-LR) and also in one AZD9291-resistant cell line (PC9 9291-LOB1).

Analysis using the Qiagen GeneRead system, and the Haloplex™ (Agilent Technologies) system showed that the corresponding DNA mutation (C to T) that encodes for the E63K mutation did not occur at a significant level (above noise) in the parental PC9 cell line or other gefitinib resistant or AZD9291-resistant cell lines tested (see Tables 1 and 2 below). Accordingly, the E63K mutation does not appear to be pre-existing, but is acquired in response to EGFR inhibition.

NGS analysis was carried out as described above for the E63K mutation.

**Table 1- GeneRead results**

| **Sample** | **Also known as** | **A** | **C (Ref)** | **G** | **T** |
|---|---|---|---|---|---|
| **PC9 IR-GM** | PC9 GR_1 | 2 (18.5) | 2028 (37.4) | | |
| **PC9 IRLR** | PC9 GR_2 | 3 (17.3) | 2501 (37.2) | 2 (14.5) | **693 (37.7)** |
| **PC9 9291R-5** | PC9 AZDR_3 | 2 (17.5) | 3044 (37.3) | 1 (19.0) | 1 (16.0) |
| **PC9** | | | 2840 (37.1) | 1 (19.0) | |
| **PC9 9291R_6** | PC9 AZDR_4 | 1 (33.0) | 2138 (37.0) | | |
| **PC9 9291R-L0B_1** | PC9 AZDR_5 | 1 (19.0) | 2677 (37.5) | 1 (19.0) | **699 (38.0)** |
| **PC9 9291R-3** | PC9 AZDR_2 | 4 (23.2) | 2657 (37.4) | 2 (17.0) | 1 (15.0) |
| **PC9 IR-4** | PC9 GR_6 | 8 (17.5) | 3353 (37.4) | 1 (17.0) | 3 (16.7) |
| **PC9 IR-6** | PC9 GR_8 | 2 (38.0) | 2165 (37.4) | | 2 (18.5) |
| **PC9 9291R-2** | PC9 AZDR_1 | 3 (16.7) | 3967 (37.3) | 1 (14.0) | 4 (21.5) |
| **PC9** | | 4 (17.0) | 2627 (37.0) | 2 (18.0) | 1 (17.0) |

The first number given indicates the number of reads, and the number in parenthesis indicates the mean phred score from sequencing (a score below 25 would be considered noise). The E63K (C to T) mutation is bolded.

**Table 2 - Haloplex results**

| **Sample** | **Also known as** | **A** | **C (Ref)** | **T** |
|---|---|---|---|---|
| **PC9 IR-GM** | PC9 GR_1 | | 686 (38.1) | |
| **PC9 IRLR** | PC9 GR_2 | | 342 (37.7) | **105 (38.0)** |
| **PC9 9291R-5** | PC9 AZDR_3 | 1 (37.0) | 378 (37.5) | |
| **PC9** | | | 459 (37.7) | |
| **PC9 9291R_6** | PC9 AZDR_4 | | 482 (37.5) | |
| **PC99291R-L0B_1** | PC9 AZDR_5 | | 295 (37.7) | **100 (37.9)** |
| **PC9 9291R-3** | PC9 AZDR_2 | | 1344 (37.7) | |
| **PC9 IR-4** | PC9 GR_6 | 1 (20.0) | 704 (38.0) | |
| **GDNA** | | | 1353 (36.7) | 1 (16.0) |
| **PC9 IR-6** | PC9 GR_8 | | 457 (37.7) | |
| **PC9 9291R-2** | PC9 AZDR_1 | | 929 (37.7) | |

The first number given indicates the number of reads, and the number in parenthesis indicates the mean phred score from sequencing. The E63K (C to T) mutation is bolded. When cell lines were tested for the EGFR T790M mutation, neither of the E63K resistant cell lines were found to have this mutation. The T709M mutation was detected in other gefitinib-resistant cell lines (PC9 IR-4, PC9 IR-GM and PC9 IR-6), but was not present in any of the AZD9291-resistant cell lines.

### Identification of gain of NRAS copy number

Gain of *NRAS* copy number was identified using aCGH with parental PC9 cells and PC9 afatinib-resistant cells (PC9_afatinib_5). The gain of *NRAS* gene copy number is detailed below, in both estimated copy number (Table 3) and log2 copy number ratio (Table 4):

**Table 3 - Estimated number of NRAS gene copies in afatinib _5 compared to the average of the PC9 parental cell lines**

| **GeneID** | **GeneSymbol** | **Sample** | **Estimated_Copy_Number** |
|---|---|---|---|
| 4893 | NRAS | PC9_afatinib-5 | 8.479034525 |
| 4893 | NRAS | Average of PC9 Parental | 3.296191359 |

**Table 4 - Log2 ratio of NRAS gene copy number in PC9_afatinib resistant_5 compared to the average of the PC9 parental cell lines**

| **GeneID** | **GeneSymbol** | **Sample** | **Log2_Ratio** |
|---|---|---|---|
| 4893 | NRAS | PC9_afatinib-5 | 2.0839 |
| 4893 | NRAS | Average of PC9 Parental | 0.7208 |

The above values were calculated using a Perl Script. The resulting log2 data was subsequently analysed, formatted, and visualised. A threshold of >1 and <-0.5 fold change was applied to the data in order to visualise the aCGH data from all samples. From the resulting visualisation the NRAS gene was identified as showing copy number gain, in comparison to the average of the parental PC9 cell linesUsing NGS as described above *NRAS* gene copy gain was also detected in 2 PC9 AZD9291 resistant populations **Table 5**

**Table 5 - Detection of NRAS gene copy number gain by sequencing analysis. Values represent gain as fold change relative to respective parental cells.**

| **Sample** | **Also known as** | ***NRAS* gain** |
|---|---|---|
| **PC9 IR4 9291R_2** | PC9 GR6 AZDR_2 | 2.4 |
| **PC9 IR4 9291R_3** | PC9 GR6 AZDR_3 | 3.68 |

### Example 4. Analysis of sensitivity of resistant cell lines to a combination of selumetinib (MEK inhibitor) and EGFR inhibitor

The effects of a panel of canonical pathway inhibitors on cell growth and survival was measured using a cell assay using Sytox Green staining as an end point.

### Reagents

RPMI-1640 medium (Sigma R7509)
Dulbecco's Phosphate buffered saline (PBS) (Sigma D8537)
L-glutamine 200mM (100 x) (Gibco, Life Technologies 25030)
Foetal Calf Serum (Sigma F7524)
TrypLE Express (Gibco, Life Technologies 12605)
AZD9291 and gefitinib (in house)

### Growth media

RPMI-1640 medium
10% Foetal calf serum
2mM L-glutamine
Sytox Green solution - Sytox Green stain, Invitrogen S7020 5mM stock diluted to 2µM in TBS containing 5mM EDTA pH 7.5
0.25% Saponin solution per well (Sigma-Aldrich Catalogue number 84510. (Saponin 2.5% stock solution prepared in TBS containing 5mM EDTA pH 7.5 and filter sterilised)

### Cell lines tested:

PC9 (NRAS WT)
PC9_IRGM (NRAS WT) (AKA. PC9 GR_1)
PC9_9291R LOB1 (NRAS E63K) (AKA PC9 AZDR_5)
PC9 IRLR (NRAS E63K) (AKA. PC9 GR_2)
PC9_9291R 3 (NRAS G12V) (AKA. PC9 AZDR_2)
PC9_9291R 5 (NRAS WT) (AKA. PC9 AZDR_3)
PC9_afatinib_5 (NRAS WT gain) (AKA. PC9 AR_5)
PC9_IR4_9291R _2 (NRAS WT gain) (AKA. PC9 GR6 AZDR 2)
PC9_IR4_9291R _3 (NRAS WT gain) (AKA. PC9 GR6 AZDR 3)

### Methods

Cell lines were plated in 384 well plates at 1000 cells per well in 70µL per well of RPMI media containing 10% foetal calf serum, 2mM L-glutamine and originating EGFR inhibitor.

The cells were allowed to attach overnight at 37°C, 5% CO₂. The following day titrations of test compound were added to the assay plates using an Echo Liquid Handler Labcyte (California, USA) and the treated cells incubated for a further 72 hours at 37°C, 5% CO₂. Each compound was tested as an 11 point dose response with a top concentration of 10µM and 1 in 3 dilutions. Following 72 hours incubation of the compound treated plates, 5µL of 2µM SYTOX Green Nucleic Acid Stain, Life Technologies (Paisley, UK) was added per well and the plates incubated at room temperature for one hour. The number of fluorescent cells per well was measured on the Acumen TTP LabTech Ltd. (Melbourn, UK) this number representing the dead cell count. 10µL of 0.25% Saponin Sigma (Dorset, UK) was added per well and the plates incubated over night at room temperature. The total number of fluorescent cells per well was acquired on the Acumen. The number of dead cells was subtracted from the total number of cells and the live cell number plotted to determine EC₅₀ values from dose response curves.

It was observed that the cell lines that had acquired an NRAS mutation were more sensitive to selumetinib when compared to the parental cells and other cell lines in which an NRAS mutation had not been detected.

The table below **(Table 6)** shows the EC₅₀ µM values across the NRAS WT and mutant cell lines tested. The data indicates that the cell lines which have acquired an NRAS mutation are more sensitive to selumetinib when treated in combination with the originating EGFR inhibitor and compared to the parental cell line. In contrast cell lines in which no NRAS mutation was detected are not sensitive to selumetinib when treated in combination with the originating EGFR inhibitor.

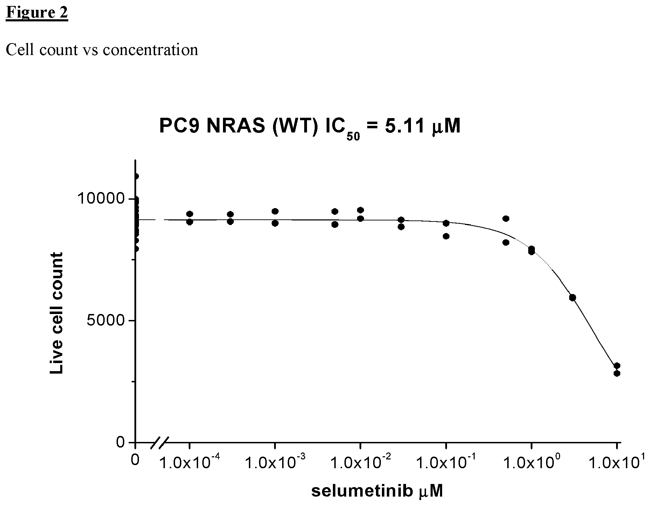

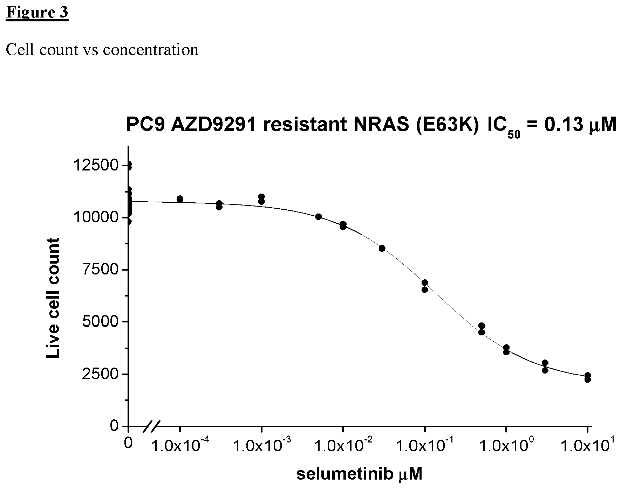

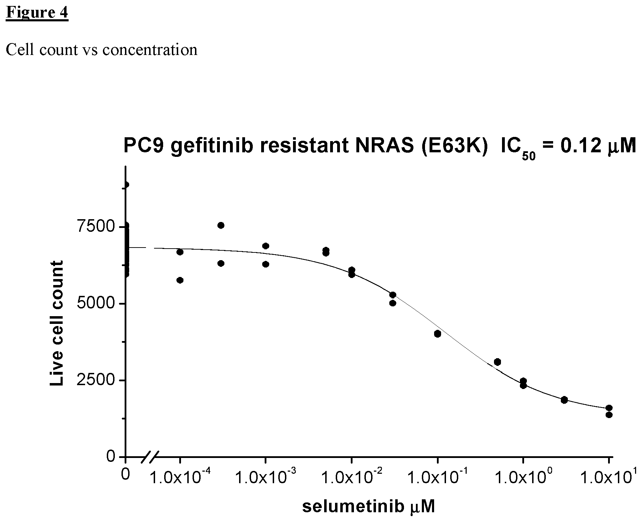

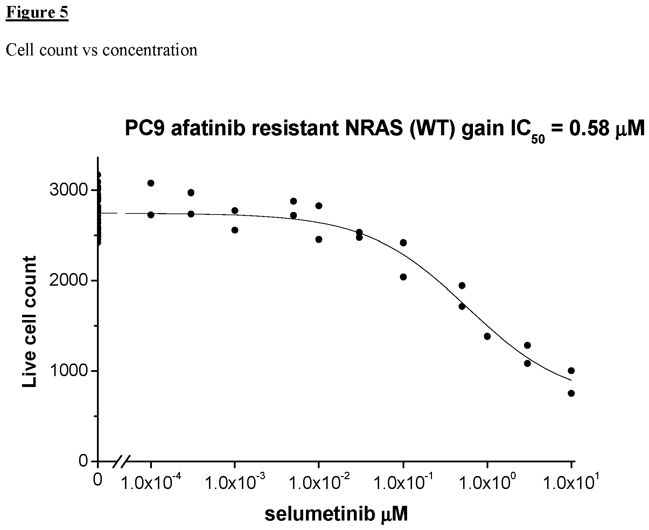

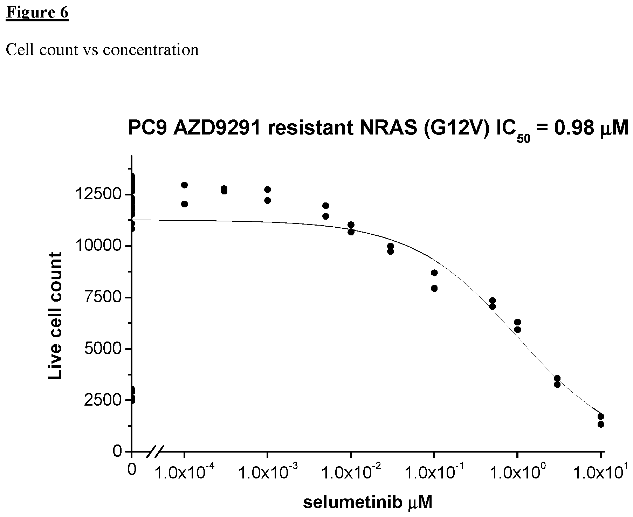

**Table 6 Cell lines were treated with dose titrations of selumetinib and EC₅₀ µM values were determined from dose response curves. Example dose response curves are shown in Figures 2 - 6.**

| **Cell line** | **Selumetinib µM** |
|---|---|
| PC9 (NRAS WT) | 6.95 |
| PC9_IRGM (NRAS WT) | **7.24** |
| PC9_IRLR (NRAS E63K) | **0.62** |
| PC9_afatinib_5 (NRAS gain) | **0.89** |
| PC9_9291R_3 (NRAS G12V) | **1.4** |
| PC9_9291R_LOB1 (NRAS E63K) | **0.167** |
| PC9_9291R_LOB3 (NRAS G12R) | **0.14** |
| PC9_IR4_9291R_2 (NRAS WT gain) | **0.54** |
| PC9_IR4_9291R_3 (NRAS WT gain) | **0.13** |

### Example 5: Analysis of the functional role of the NRAS E63K mutation in survival of gefitinib or AZD9291 resistant cell populations

In order to investigate whether the novel NRAS E63K mutation is an activating mutation that drives survival of the gefitinib or AZD9291 resistant cells, the inventors used a range of techniques:
(a) RAS activation assays to compare basal levels of active NRAS in parental PC9 cells (WT NRAS) with basal levels of active NRAS in resistant cells with mutant NRAS.
(b) siRNA knockdown of expression of WT and mutant *NRAS* to investigate the effects on downstream signaling and cell growth.
(c) Exogenous expression of NRAS WT and NRAS E63K mutant variants in PC9 cells to investigate the effects on cell growth inhibition by gefitinib or AZD9291.

### (a). RAS activation assays.

### Method

PC9 and PC9 AZD9291 resistant cells were cultured in media containing no AZD9291 for 4 days. Cells were then plated into 6 well plates and serum starved overnight. The following day the cells were treated for 2 hours with media supplemented with or without 160nM AZD9291. Lysates were prepared and active NRAS isolated using a GST-RAS binding domain pull down assay (Thermo Scientific). Pull down lysates were analysed by western blot using an NRAS specific antibody.

### Results

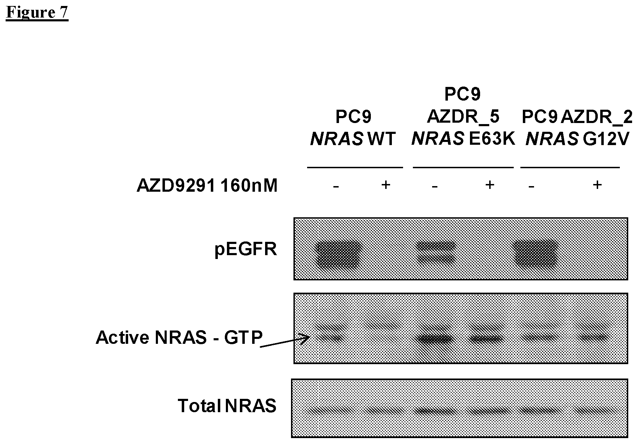

Basal active NRAS levels were lower in parental PC-9 cells compared to resistant PC-9 cell populations in which an E63K or a G12V NRAS mutation had been detected. In addition treatment of parental PC-9 cells with 160nM AZD9291 for 2 hours caused a decrease in phosphorylated EGFR and active NRAS. In contrast in the mutant NRAS cells a decrease in phosphorylated EGFR was not associated with corresponding decrease in active NRAS suggesting constitutive activation of NRAS independent of EGFR in these cells. Results are shown in **Figure 7****.**

### (b) Analysis of the effect of NRAS and KRAS siRNA treatment on PC9, PC9 gefitinib-resistant and PC9 AZD9291-resistant cell populations.

PC9, PC9 gefitinib-resistant and PC9 AZD9291-resistant cells were plated at 5 x 10⁵ cells per well in 6 well plates in 2mls per well of growth media supplemented with EGFR inhibitor where appropriate (resistant cells were grown in the presence of EGFR inhibitor). The cells were incubated over night at 37°C, 5% CO₂. The following day the cells were treated with a final concentration of 20nM of siRNA from Dharmacon as detailed in **Table 7.**

**Table 7 Sequences for NRAS and KRAS siRNA constructs.**

| *siRNA* | *Reference* | *Sequence* | *SEQ ID NO:* |
|---|---|---|---|
| Nontargeting control (NTC) | D-001810-10 | UGGUUUACAUGUCGACUAA | 9 |
| | | UGGUUUACAUGUUGUGUGA | 10 |
| | | UGGUUUACAUGUUUUCUGA | 11 |
| | | UGGUUUACAUGUUUUCCUA | 12 |
| NRAS_1 | J-003919-05 | GAGCAGAUUAAGCGAGUAA | 13 |
| NRAS_2 | J-003919-06 | GAAAUACGCCAGUACCGAA | 14 |
| NRAS_3 | J-003919-07 | GUGGUGAUGUAACAAGAUA | 15 |
| KRAS_1 | J-005069-10 | GAAGUUAUGGAAUUCCUUU | 16 |

Each siRNA construct was prepared in Optimem (Life Technologies) media using RNA iMAX (Invitrogen) as transfection reagent. For each well: 2.5µl of siRNA (20µM stock) was mixed with 250µL of Optimem; and 2.5µL of RNA iMAX was mixed with 250µL of Optimem. The 2 solutions were mixed together by pipetting and allowed to stand for 5 minutes at room temperature. The final 500µL solution was then pipetted into the appropriate 2mL of growth media in the 6 well plates. The plates were swirled gently and further incubated over night at 37°C, 5% CO₂. The following day each well of cells was harvested and the cell number counted.

A sample of cells from each transfection were then seeded in 96 well plates at 3000 cells per well in 100µL of growth media supplemented with EGFR inhibitor where appropriate across 5 replicate wells for each transfection condition. The remainder of the cells were re-plated in fresh 6 well plates. Both the 96 and 6 well plates were returned to the incubator for further growth.

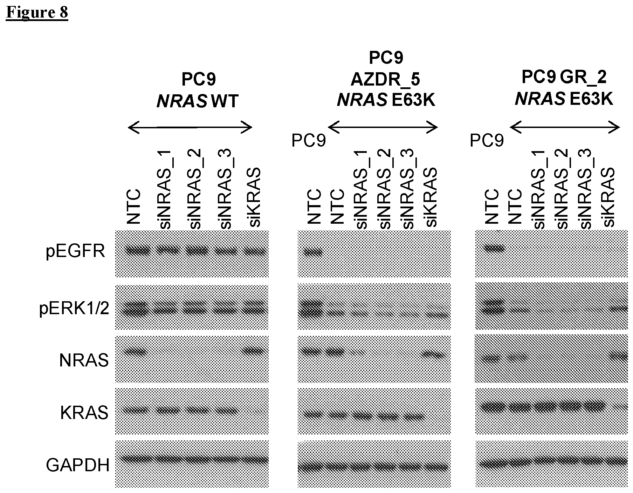

The following day, lysates were prepared from the 6 well plates so that knockdown of protein expression could be assessed following 48 hours treatment with siRNA. The lysates were analysed by western blot for levels of: phosphorylated EGFR, phosphorylated ERK, NRAS and KRAS. Levels of GAPDH were used as a loading control across all of the cell samples. **(****Figure 8****)**

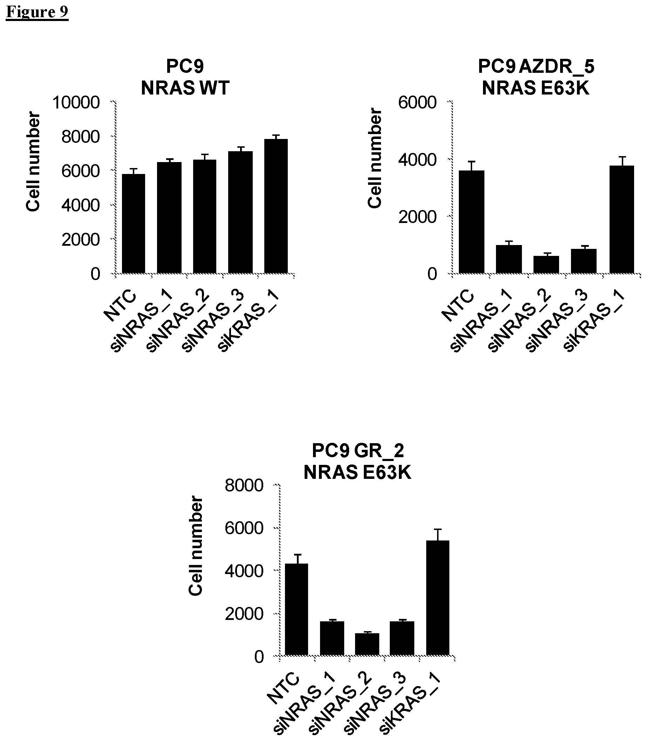

Corresponding cells in the 96 well plates were allowed to grow for a further 5 days after which they were fixed by addition of 100µL of 4% formaldehyde per well for 30 minutes at room temperature. The wells were washed with PBS and the nuclei stained by addition of Hoechst (1 in 5000 dilution) for 30 minute at room temperature. The cell number was counted using the Cellomics Arrayscan counting 9 fields per well at 10 x magnification. The average cell number was plotted **(****Figure 9****).**

The results of the western blots indicated that NRAS expression was knocked down in all of the cell lines by all 3 NRAS siRNA constructs used, but that no effect on NRAS expression was seen with the NTC or KRAS siRNA construct. On the other hand, only the KRAS_1 construct caused knockdown of KRAS expression whilst having no affect on expression of NRAS **(****Figure 8****).**

The effect on cell growth by the siRNA transfections indicated that in the gefitinib-resistant and the AZD9291-resistant cell lines in which the novel NRAS mutation has been identified, knockdown of NRAS expression significantly inhibited cell growth whereas knockdown of KRAS expression had no effect on cell growth. In contrast neither knockdown of NRAS or KRAS had an effect on the growth of the PC9 parental cells **(****Figure 9****).**

This data suggests that the NRAS E63K mutation is an activating mutation and that it is driving survival of the resistant cells.

### (c) Analysis of over expression of NRAS WT and NRAS E63K mutant DNA variants in PC9 cells to investigate the effects on cell growth inhibition by gefitinib or AZD9291.

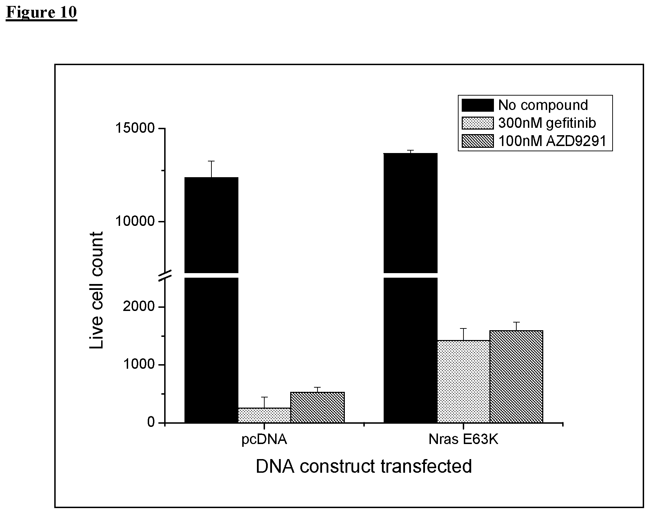

PC9 cells were transfected with control DNA construct, pcDNA 3.1+ control, or a construct designed to express E63K mutant *NRAS*, pcDNA 3.1+ / NRAS E63K, (Life Technologies Ltd.). For 96 hour expression MaxCyte transfection technology was used to electroporate the PC9 cells. PC9 cells were passaged the day before the transfection. On the day of the transfection cells were harvested and resuspended at 9 x 10⁷ cells per 600 µL MaxCyte buffer. 100 µL of cell suspension was transfered to a MaxCyte cuvette and the cells electroporated woth 20 µg DNA construct. Following electroporation the cells for each condition were transfered to a 6 well plate and incubated at 37°C for 30 minutes after which they were seeded in a 6 well plate at 4 x 10⁵ cells per well. Following over night incubation the cells were harvested and replated in 384 well plate at 1000 cells/well. The next day the cells were dosed with either 100nM AZD9291 or 300nM gefitinib and the plates incubated for a further 96 hours. A live cell number was determined using the sytox green method as described hereinbefore. Figure 10 shows that cell growth inhibition by AZD9291 and gefitinib was reduced when cells over expressed E63K mutant NRAS.

### Sensitivity to MEK inhibitor (selumetinib) of a cell population containing NRAS G12R mutation

An AZD9291-resistant PC9 cell line was prepared using standard methods as illustrated hereinbefore. A range of such resistant cell populations were cultured and analysed. One such cell population was found to contain the NRAS G12R mutation and this population also showed >5 fold increased sensitivity to the MEK inhibitor, selumetinib, as compared to the parental cell line.

## Claims

1. An EGFR inhibitor for use in the treatment of NRAS mutated non-small cell lung cancer, wherein the EGFR inhibitor is administered in combination with a MEK inhibitor and the NRAS mutation is selected from E63K, G12V, G12R, or a gain of copy number of the *NRAS* gene.

2. An EGFR inhibitor for use as claimed in claim 1, wherein the EGFR inhibitor is selected from gefitinib, erlotinib, afatinib, AZD9291 and CO1686; or a pharmaceutically acceptable salt thereof.

3. An EGFR inhibitor for use as claimed in claim 1 or claim 2, wherein the EGFR inhibitor is selected from AZD9291 and CO 1686; or a pharmaceutically acceptable salt thereof.

4. An EGFR inhibitor for use as claimed in any one of claims 1 to 3, wherein the MEK inhibitor is selected from selumetinib, trametinib, MEK-162 and cobimetinib; or a pharmaceutically acceptable salt thereof.

5. An EGFR inhibitor for use as claimed in any one of claims 1 to 4, wherein the EGFR inhibitor is AZD9291 or a pharmaceutically acceptable salt thereof.

6. An EGFR inhibitor for use as claimed in any one of claims 1 to 5, wherein the MEK inhibitor is selumetinib or a pharmaceutically acceptable salt thereof.

7. An EGFR inhibitor for use as claimed in claim 1, wherein the EGFR inhibitor is AZD9291 or a pharmaceutically acceptable salt thereof and the MEK inhibitor is selumetinib or a pharmaceutically acceptable salt thereof.

## Patentansprüche

1. EGFR-Inhibitor zur Verwendung bei der Behandlung von NRAS-mutiertem nicht-kleinzelligem Lungenkrebs, wobei der EGFR-Inhibitor in Kombination mit einem MEK-Inhibitor verabreicht wird und die NRAS-Mutation aus E63K, G12V, G12R oder einer Zunahme der Kopienzahl des NRAS-Gens ausgewählt ist.

2. EGFR-Inhibitor zur Verwendung nach Anspruch 1, wobei der EGFR-Inhibitor ausgewählt ist aus Gefitinib, Erlotinib, Afatinib, AZD9291 und CO1686; oder einem pharmazeutisch annehmbaren Salz davon.

3. EGFR-Inhibitor zur Verwendung nach Anspruch 1 oder Anspruch 2, wobei der EGFR-Inhibitor ausgewählt ist aus AZD9291 und CO1686; oder einem pharmazeutisch annehmbaren Salz davon.

4. EGFR-Inhibitor zur Verwendung nach einem der Ansprüche 1 bis 3, wobei der MEK-Inhibitor ausgewählt ist aus Selumetinib, Trametinib, MEK-162 und Cobimetinib; oder einem pharmazeutisch annehmbaren Salz davon.

5. EGFR-Inhibitor zur Verwendung nach einem der Ansprüche 1 bis 4, wobei es sich bei dem EGFR-Inhibitor um AZD9291 oder ein pharmazeutisch annehmbares Salz davon handelt.

6. EGFR-Inhibitor zur Verwendung nach einem der Ansprüche 1 bis 5, wobei es sich bei dem MEK-Inhibitor um Selumetinib oder ein pharmazeutisch annehmbares Salz davon handelt.

7. EGFR-Inhibitor zur Verwendung nach Anspruch 1, wobei es sich bei dem EGFR-Inhibitor um AZD9291 oder ein pharmazeutisch annehmbares Salz davon handelt, und es sich bei dem MEK-Inhibitor um Selumetinib oder ein pharmazeutisch annehmbares Salz davon handelt.

## Revendications

1. Inhibiteur d'EGFR pour une utilisation dans le traitement du cancer du poumon non à petites cellules à mutation NRAS, l'inhibiteur d'EGFR étant administré en combinaison avec un inhibiteur de MEK et la mutation NRAS étant choisie parmi E63K, G12V, G12R, ou un gain du nombre de copies du gène NRAS.

2. Inhibiteur d'EGFR pour une utilisation selon la revendication 1, l'inhibiteur d'EGFR étant choisi parmi le gefitinib, l'erlotinib, l'afatinib, l'AZD9291 et le CO1686 ; ou un sel pharmaceutiquement acceptable correspondant.

3. Inhibiteur d'EGFR pour une utilisation selon la revendication 1 ou la revendication 2, l'inhibiteur d'EGFR étant choisi parmi l'AZD9291 et le CO1686 ; ou un sel pharmaceutiquement acceptable correspondant.

4. Inhibiteur d'EGFR pour une utilisation selon l'une quelconque des revendications 1 à 3, l'inhibiteur de MEK étant choisi parmi le selumetinib, le tramétinib, le MEK-162 et le cobimetinib ; ou un sel pharmaceutiquement acceptable correspondant.

5. Inhibiteur d'EGFR pour une utilisation selon l'une quelconque des revendications 1 à 4, l'inhibiteur d'EGFR étant l'AZD9291 ou un sel pharmaceutiquement acceptable correspondant.

6. Inhibiteur d'EGFR pour une utilisation selon l'une quelconque des revendications 1 à 5, l'inhibiteur de MEK étant le selumetinib ou un sel pharmaceutiquement acceptable correspondant.

7. Inhibiteur d'EGFR pour une utilisation selon la revendication 1, l'inhibiteur d'EGFR étant l'AZD9291 ou un sel pharmaceutiquement acceptable correspondant et l'inhibiteur de MEK étant le selumetinib ou un sel pharmaceutiquement acceptable correspondant.
